(19) Europäisches Patentamt
European Patent Office
Office européen des brevets

(11) Numéro de publication: **0 191 662 B1**

(12) **FASCICULE DE BREVET EUROPEEN**

(45) Date de publication du fascicule du brevet: **01.06.88**

(51) Int. Cl.[4]: **C 07 K 5/00**, A 61 K 37/02

(21) Numéro de dépôt: **86400050.0**

(22) Date de dépôt: **10.01.86**

(54) **Nouveaux dérivés de la pristinamycine IIB, leur préparation et les compostions pharmaceutiques qui les contiennent.**

(30) Priorité: **11.01.85 FR 8500377**

(43) Date de publication de la demande: **20.08.86 Bulletin 86/34**

(45) Mention de la délivrance du brevet: **01.06.88 Bulletin 88/22**

(84) Etats contractants désignés: **AT BE CH DE FR GB IT LI LU NL SE**

(56) Documents cités:
**CHEMICAL ABSTRACTS, vol. 101, 1984, page 726, abrégé no. 191457r, Columbus, Ohio, US; E. FUJITA: "Organic synthesis utilizing thiazolidine and the related heterocycles"**
**BULLETIN DE LA SOCIETE CHIMIQUE DE FRANCE, no. 2, 1968, pages 585-591; J. PREUD'HOMME et al.: "Pristinamycine isolement, caractérisation et identification des constituants"**
**THE JOURNAL OF ANTIBIOTICS, vol. XXXVII, no. 10, octobre 1984, pages 1246-1252; P. LACROIX et al.: "Pristinamycin accumulation by Staphylococcus Aureus"**
**CHEMICAL ABSTRACTS, vol. 100, 1984, page 564, abrégé no. 209476n, Columbus, Ohio, US; Y. NAGAO et al.: "Synthetic studies on virginiamycin M2 utilizing functional five-membered heterocycles"**

(73) Titulaire: **RHONE-POULENC SANTE, Les Miroirs 18 Avenue d'Alsace, F-92400 Courbevoie Cédex (FR)**

(72) Inventeur: **Barriere, Jean-Claude, 23 rue Henri Gilbert, F-91300 Massy (FR)**
Inventeur: **Cotrel, Claude, 17 A avenue du Docteur Arnold Netter, F-75012 Paris (FR)**
Inventeur: **Paris, Jean-Marc, 8 rue des Acacias, F-77360 Vaires sur Marne (FR)**

(74) Mandataire: **Gaumont, Robert et al, RHONE-POULENC RECHERCHES Service Brevets Pharma 25, Quai Paul Doumer, F-92408 Courbevoie Cedex (FR)**

EP 0 191 662 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

Dans le Bulletin de la Société Chimique de France, 2, 585 (1968) est décrit l'isolement des composantes de la pristinamycine.

La publication de Fujita, Heterocycles 21 (1), 41 (1984) décrit la préparation de la pristinamycine $II_B$ par synthèse totale.

La présente invention concerne de nouveaux dérivés de la pristinamycine $II_B$ de formule générale:

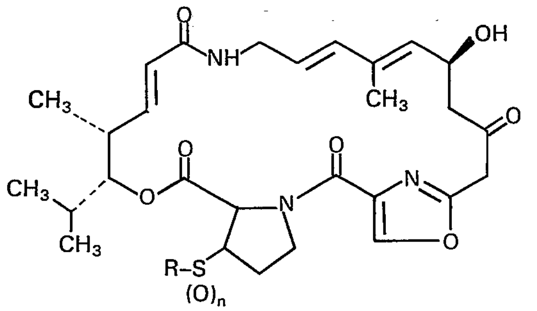

(I)

leurs sels, leur préparation et les compositions pharmaceutiques qui les contiennent.

Dans la formule générale (I), le symbole R représente:

— soit un radical hétérocyclyle azoté de 4 à 7 chaînons, contenant éventuellement 1 ou plusieurs autres hétéroatomes choisis parmi l'azote, l'oxygène ou le soufre à l'état de sulfoxyde ou de sulfone, et éventuellement substitué par un radical alcoyle,

— soit une chaîne alcoyle contenant 2 à 4 atomes de carbone substituée par 1 ou 2 radicaux choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces 2 derniers radicaux pouvant éventuellement former avec l'atome d'azote auquel elles sont rattachées un hétérocycle saturé ou insaturé de 4 à 7 chaînons contenant éventuellement un autre hétéroatome choisi parmi l'azote, l'oxygène ou le soufre à l'état de sulfoxyde ou de sulfone, et éventuellement substitué par un radical alcoyle), ou substituée par un ou plusieurs hétérocycles azotés de 4 à 7 chaînons contenant éventuellement 1 ou 2 autres hétéroatomes choisis parmi l'azote, l'oxygène ou le soufre à l'état de sulfoxyde ou de sulfone, et éventuellement substitués par un radical alcoyle, lesdits hétérocycles étant rattachés à la chaîne qui les porte par l'intermédiaire d'un atome de carbone, étant entendu que l'un au moins des substituants portés par la chaîne alcoyle ci-dessus est un substituant azoté capable de former des sels, et

le symbole n est égal à 1 ou 2.

Il est entendu que les radicaux et portions alcoyle cités ci-dessus sont droits ou ramifiés et contiennent sauf mention spéciale 1 à 10 atomes de carbone.

Il est également entendu que les produits de formule générale (I) présentent des formes isomères, et que les isomères et leurs mélanges entrent dans le cadre de la présente invention.

Lorsque R représente un radical hétérocyclyle, ce radical peut être choisi à titre d'exemple parmi: azétidinyle-3, pyrrolidinyle-3, pipéridyle-3 ou -4 ou azépinyle-3 ou -4.

Lorsque R représente un radical hétérocyclylalcoyle, le radical hétérocyclyle peut être choisi à titre d'exemple parmi les radicaux cités ci-dessus ou les radicaux azétidinyle-2, pyrrolidinyle-2, pipéridyle-2, azépinyle-2, pipérazinyle, alcoyl-4 pipérazinyle, quinolyle, isoquinolyle ou imidazolyle.

Lorsque R contient un radical dialcoylamino ou dialcoylcarbamoyloxy dont les parties alcoyle forment avec l'atome d'azote auquel elles sont rattachées un hétérocycle, ce dernier peut être choisi à titre d'exemple parmi: azétidinyle-1, pyrrolidinyle-1, pipéridino, azépinyle-1, morpholino, thiomorpholino à l'état de sulfoxyde ou de sulfone, pipérazinyle-1, alcoyl-4 pipérazinyle-1, N-alcoyl homopipérazinyle-1, imidazolyle-1.

A titre d'exemple on peut notamment citer les produits de formule générale (I) suivants:

(azétidinyl-3) sulfinyl-26 pristinamycine $II_B$
(méthyl-1 azétidinyl-3) sulfinyl-26 pristinamycine $II_B$
(éthyl-1 azétidinyl-3) sulfinyl-26 pristinamycine $II_B$
(isopropyl-1 azétidinyl-3) sulfinyl-26 pristinamycine $II_B$
(pyrrolidinyl-3) sulfinyl-26 pristinamycine $II_B$
(méthyl-1 pyrrolidinyl-3) sulfinyl-26 pristinamycine $II_B$
(éthyl-1 pyrrolidinyl-3) sulfinyl-26 pristinamycine $II_B$
(isopropyl-1 pyrrolidinyl-3) sulfinyl-26 pristinamycine $II_B$
(pipéridyl-3) sulfinyl-26 pristinamycine $II_B$
(méthyl-1 pipéridyl-3) sulfinyl-26 pristinamycine $II_B$
(éthyl-1 pipéridyl-3) sulfinyl-26 pristinamycine $II_B$
(pipéridyl-4) sulfinyl-26 pristinamycine $II_B$
(méthyl-1 pipéridyl-4) sulfinyl-26 pristinamycine $II_B$
(éthyl-1 pipéridyl-4) sulfinyl-26 pristinamycine $II_B$
(azépinyl-3) sulfinyl-26 pristinamycine $II_B$
(azépinyl-4) sulfinyl-26 pristinamycine $II_B$
(cyclopropylamino-2 éthyl) sulfinyl-26 pristinamycine $II_B$
(cyclobutylamino-2 éthyl) sulfinyl-26 pristinamycine $II_B$
(cyclopentylamino-2 éthyl) sulfinyl-26 pristinamycine $II_B$
(cyclohexylamino-2 éthyl) sulfinyl-26 pristinamycine $II_B$
(N-cyclohexyl N-méthyl-amino-2 éthyl) sulfinyl-26 pristinamycine $II_B$
(méthylamino-2 éthyl) sulfinyl-26 pristinamycine $II_B$
(éthylamino-2 éthyl) sulfinyl-26 pristinamycine $II_B$
(propylamino-2 éthyl) sulfinyl-26 pristinamycine $II_B$
(isopropylamino-2 éthyl) sulfinyl-26 pristinamycine $II_B$
(butylamino-2 éthyl) sulfinyl-26 pristinamycine $II_B$
(isobutylamino-2 éthyl) sulfinyl-26 pristinamycine $II_B$
(n.décylamino-2 éthyl) sulfinyl-26 pristinamycine $II_B$
(diméthylamino-2 éthyl) sulfinyl-26 pristinamycine $II_B$
(diéthylamino-2 éthyl) sulfinyl-26 pristinamycine $II_B$
(dipropylamino-2 éthyl) sulfinyl-26 pristinamycine $II_B$
(diisopropylamino-2 éthyl) sulfinyl-26 pristinamycine $II_B$
(dibutylamino-2 éthyl) sulfinyl-26 pristinamycine $II_B$
(diisobutylamino-2 éthyl) sulfinyl-26 pristinamycine $II_B$

(N-éthyl N-méthyl-amino-2 éthyl) sulfinyl-26 pristinamycine $II_B$
[(azétidinyl-1)-2 éthyl] sulfinyl-26 pristinamycine $II_B$
[(pyrrolidinyl-1)-2 éthyl] sulfinyl-26 pristinamycine $II_B$
(pipéridino-2 éthyl) sulfinyl-26 pristinamycine $II_B$
[(azépinyl-1)-2 éthyl] sulfinyl-26 pristinamycine $II_B$
(morpholino-2 éthyl) sulfinyl-26 pristinamycine $II_B$
[(pipérazinyl-1)-2 éthyl] sulfinyl-26 pristinamycine $II_B$
[(méthyl-4 pipérazinyl-1)-2 éthyl] sulfinyl-26 pristinamycine $II_B$
[(méthyl-4 homopipérazinyl-1)-2 éthyl] sulfinyl-26 pristinamycine $II_B$
[(imidazolyl-1)-2 éthyl] sulfinyl-26 pristinamycine $II_B$
(diméthylaminocarbamoyloxy-2 éthyl) sulfinyl-26 pristinamycine $II_B$
(diéthylaminocarbamoyloxy-2 éthyl) sulfinyl-26 pristinamycine $II_B$
(diisopropylaminocarbamoyloxy-2 éthyl) sulfinyl-26 pristinamycine $II_B$
[(méthyl-4 pipérazinyl-1)carbamoyloxy-2 éthyl] sulfinyl-26 pristinamycine $II_B$
[(azétidinyl-2)-2 éthyl] sulfinyl-26 pristinamycine $II_B$
[(azétidinyl-3)-2 éthyl] sulfinyl-26 pristinamycine $II_B$
[(pyrrolidinyl-2)-2 éthyl] sulfinyl-26 pristinamycine $II_B$
[(pyrrolidinyl-3)-2 éthyl] sulfinyl-26 pristinamycine $II_B$
[(pipéridyl-2)-2 éthyl] sulfinyl-26 pristinamycine $II_B$
[(pipéridyl-3)-2 éthyl] sulfinyl-26 pristinamycine $II_B$
[(pipéridyl-4)-2 éthyl] sulfinyl-26 pristinamycine $II_B$
[(azépinyl-2)-2 éthyl] sulfinyl-26 pristinamycine $II_B$
[(azépinyl-3)-2 éthyl] sulfinyl-26 pristinamycine $II_B$
[(azépinyl-4)-2 éthyl] sulfinyl-26 pristinamycine $II_B$
[(quinolyl-3)-2 éthyl] sulfinyl-26 pristinamycine $II_B$
[(quinolyl-4)-2 éthyl] sulfinyl-26 pristinamycine $II_B$
[(tétrahydro-1,2,3,4 isoquinolyl-2)-2 éthyl] sulfinyl-26 pristinamycine $II_B$
[(isoquinolyl-1)-2 éthyl] sulfinyl-26 pristinamycine $II_B$
(imidazolyl-2 éthyl) sulfinyl-26 pristinamycine $II_B$
(cyclopropylamino-2 méthyl-1 éthyl) sulfinyl-26 pristinamycine $II_B$
(cyclobutylamino-2 méthyl-1 éthyl) sulfinyl-26 pristinamycine $II_B$
(cyclopentylamino-2 méthyl-1 éthyl) sulfinyl-26 pristinamycine $II_B$
(cyclohexylamino-2 méthyl-1 éthyl) sulfinyl-26 pristinamycine $II_B$
[(N-cyclohexyl N-méthyl-amino)-2 méthyl-1 éthyl] sulfinyl-26 pristinamycine $II_B$
(méthylamino-2 méthyl-1 éthyl) sulfinyl-26 pristinamycine $II_B$
(éthylamino-2 méthyl-1 éthyl) sulfinyl-26 pristinamycine $II_B$
(méthyl-1 propylamino-2 éthyl) sulfinyl-26 pristinamycine $II_B$
(isopropylamino-2 méthyl-1 éthyl) sulfinyl-26 pristinamycine $II_B$
(butylamino-2 méthyl-1 éthyl) sulfinyl-26 pristinamycine $II_B$
(i.butylamino-2 méthyl-1 éthyl) sulfinyl-26 pristinamycine $II_B$
(méthyl-1 n.décylamino-2 éthyl) sulfinyl-26 pristinamycine $II_B$
(diméthylamino-2 méthyl-1 éthyl) sulfinyl-26 pristinamycine $II_B$
(diéthylamino-2 méthyl-1 éthyl) sulfinyl-26 pristinamycine $II_B$
(dipropylamino-2 méthyl-1 éthyl) sulfinyl-26 pristinamycine $II_B$
(diisopropylamino-2 méthyl-1 éthyl) sulfinyl-26 pristinamycine $II_B$
(dibutylamino-2 méthyl-1 éthyl) sulfinyl-26 pristinamycine $II_B$
(diisobutylamino-2 méthyl-1 éthyl) sulfinyl-26 pristinamycine $II_B$
[(N-éthyl N-méthyl-amino)-2 méthyl-1 éthyl] sulfinyl-26 pristinamycine $II_B$
[(azétidinyl-1)-2 méthyl-1 éthyl] sulfinyl-26 pristinamycine $II_B$
[méthyl-1 (pyrrolidinyl-1)-2 éthyl] sulfinyl-26 pristinamycine $II_B$
(méthyl-1 pipéridino-2 éthyl) sulfinyl-26 pristinamycine $II_B$
[(azépinyl-1)-2 méthyl-1 éthyl] sulfinyl-26 pristinamycine $II_B$
(méthyl-1 morpholino-2 éthyl) sulfinyl-26 pristinamycine $II_B$
[méthyl-1 (pipérazinyl-1)-2 éthyl] sulfinyl-26 pristinamycine $II_B$
[(méthyl-4 pipérazinyl-1)-2 méthyl-1 éthyl] sulfinyl-26 pristinamycine $II_B$
[(méthyl-4 homopipérazinyl-1)-2 méthyl-1 éthyl] sulfinyl-26 pristinamycine $II_B$
[(imidazolyl-1)-2 méthyl-1 éthyl] sulfinyl-26 pristinamycine $II_B$
(diméthylaminocarbamoyloxy-2 méthyl-1 éthyl) sulfinyl-26 pristinamycine $II_B$
(diéthylaminocarbamoyloxy-2 méthyl-1 éthyl) sulfinyl-26 pristinamycine $II_B$
(diisopropylaminocarbamoyloxy-2 méthyl-1 éthyl) sulfinyl-26 pristinamycine $II_B$
[(méthyl-4 pipérazinyl-1)carbamoyloxy-2 méthyl-1 éthyl] sulfinyl-26 pristinamycine $II_B$
[(azétidinyl-2)-2 méthyl-1 éthyl] sulfinyl-26 pristinamycine $II_B$
[(azétidinyl-3)-2 méthyl-1 éthyl] sulfinyl-26 pristinamycine $II_B$
[méthyl-1 (pyrrolidinyl-2)-2 éthyl] sulfinyl-26 pristinamycine $II_B$
[méthyl-1 (pyrrolidinyl-3)-2 éthyl] sulfinyl-26 pristinamycine $II_B$
[méthyl-1 (pipéridyl-2)-2 éthyl] sulfinyl-26 pristinamycine $II_B$
[méthyl-1 (pipéridyl-3)-2 éthyl] sulfinyl-26 pristinamycine $II_B$
[méthyl-1 (pipéridyl-4)-2 éthyl] sulfinyl-26 pristinamycine $II_B$
[(azépinyl-2)-2 méthyl-1 éthyl] sulfinyl-26 pristinamycine $II_B$
[(azépinyl-3)-2 méthyl-1 éthyl] sulfinyl-26 pristinamycine $II_B$
[(azépinyl-4)-2 méthyl-1 éthyl] sulfinyl-26 pristinamycine $II_B$
[méthyl-1 (quinolyl-3)-2 éthyl] sulfinyl-26 pristinamycine $II_B$

[méthyl-1 (quinolyl-4)-2 éthyl] sulfinyl-26 pristinamycine $II_B$
[méthyl-1 (tétrahydro-1,2,3,4 isoquinolyl-2)-2 éthyl] sulfinyl-26 pristinamycine $II_B$
[(isoquinolyl-1)-2 méthyl-1 éthyl] sulfinyl-26 pristinamycine $II_B$
(imidazolyl-2 méthyl-1 éthyl) sulfinyl-26 pristinamycine $II_B$
(cyclopropylamino-2 méthyl-2 éthyl) sulfinyl-26 pristinamycine $II_B$
(cyclobutylamino-2 méthyl-2 éthyl) sulfinyl-26 pristinamycine $II_B$
(cyclopentylamino-2 méthyl-2 éthyl) sulfinyl-26 pristinamycine $II_B$
(cyclohexylamino-2 méthyl-2 éthyl) sulfinyl-26 pristinamycine $II_B$
[(N-cyclohexyl N-méthyl-amino)-2 méthyl-2 éthyl] sulfinyl-26 pristinamycine $II_B$
(méthylamino-2 méthyl-2 éthyl) sulfinyl-26 pristinamycine $II_B$
(éthylamino-2 méthyl-2 éthyl) sulfinyl-26 pristinamycine $II_B$
(méthyl-2 propylamino-2 éthyl) sulfinyl-26 pristinamycine $II_B$
(isopropylamino-2 méthyl-2 éthyl) sulfinyl-26 pristinamycine $II_B$
(butylamino-2 méthyl-2 éthyl) sulfinyl-26 pristinamycine $II_B$
(isobutylamino-2 méthyl-2 éthyl) sulfinyl-26 pristinamycine $II_B$
(méthyl-2 n.décylamino-2 éthyl) sulfinyl-26 pristinamycine $II_B$
(diméthylamino-2 méthyl-2 éthyl) sulfinyl-26 pristinamycine $II_B$
(diéthylamino-2 méthyl-2 éthyl) sulfinyl-26 pristinamycine $II_B$
(dipropylamino-2 méthyl-2 éthyl) sulfinyl-26 pristinamycine $II_B$
(diisopropylamino-2 méthyl-2 éthyl) sulfinyl-26 pristinamycine $II_B$
(dibutylamino-2 méthyl-2 éthyl) sulfinyl-26 pristinamycine $II_B$
(diisobutylamino-2 méthyl-2 éthyl) sulfinyl-26 pristinamycine $II_B$
[(N-éthyl N-méthyl-amino)-2 méthyl-2 éthyl] sulfinyl-26 pristinamycine $II_B$
[(azétidinyl-1)-2 méthyl-2 éthyl] sulfinyl-26 pristinamycine $II_B$
[méthyl-2 (pyrrolidinyl-1)-2 éthyl] sulfinyl-26 pristinamycine $II_B$
(méthyl-2 pipéridino-2 éthyl) sulfinyl-26 pristinamycine $II_B$
[(azépinyl-1)-2 méthyl-2 éthyl] sulfinyl-26 pristinamycine $II_B$
(méthyl-2 morpholino-2 éthyl) sulfinyl-26 pristinamycine $II_B$
[méthyl-2 (pipérazinyl-1)-2 éthyl] sulfinyl-26 pristinamycine $II_B$
[(méthyl-4 pipérazinyl-1)-2 méthyl-2 éthyl] sulfinyl-26 pristinamycine $II_B$
[(méthyl-4 homopipérazinyl-1)-2 méthyl-2 éthyl] sulfinyl-26 pristinamycine $II_B$
[(imidazolyl-1)-2 méthyl-2 éthyl] sulfinyl-26 pristinamycine $II_B$
(diméthylaminocarbamoyloxy-2 méthyl-2 éthyl) sulfinyl-26 pristinamycine $II_B$
(diéthylaminocarbamoyloxy-2 méthyl-2 éthyl) sulfinyl-26 pristinamycine $II_B$
(diisopropylaminocarbamoyloxy-2 méthyl-2 éthyl) sulfinyl-26 pristinamycine $II_B$
[(méthyl-4 pipérazinyl-1)carbamoyloxy-2 méthyl-2 éthyl] sulfinyl-26 pristinamycine $II_B$
[(azétidinyl-2)-2 méthyl-2 éthyl] sulfinyl-26 pristinamycine $II_B$
[(azétidinyl-3)-2 méthyl-2 éthyl] sulfinyl-26 pristinamycine $II_B$
[méthyl-2 (pyrrolidinyl-2)-2 éthyl] sulfinyl-26 pristinamycine $II_B$
[méthyl-2 (pyrrolidinyl-3)-2 éthyl] sulfinyl-26 pristinamycine $II_B$
[méthyl-2 (pipéridyl-2)-2 éthyl] sulfinyl-26 pristinamycine $II_B$
[méthyl-2 (pipéridyl-3)-2 éthyl] sulfinyl-26 pristinamycine $II_B$
[méthyl-2 (pipéridyl-4)-2 éthyl] sulfinyl-26 pristinamycine $II_B$
[(azépinyl-2)-2 méthyl-2 éthyl] sulfinyl-26 pristinamycine $II_B$
[(azépinyl-3)-2 méthyl-2 éthyl] sulfinyl-26 pristinamycine $II_B$
[(azépinyl-4)-2 méthyl-2 éthyl] sulfinyl-26 pristinamycine $II_B$
[méthyl-2 (quinolyl-3)-2 éthyl] sulfinyl-26 pristinamycine $II_B$
[méthyl-2 (quinolyl-4)-2 éthyl] sulfinyl-26 pristinamycine $II_B$
[méthyl-2 (tétrahydro-1,2,3,4 isoquinolyl-2)-2 éthyl] sulfinyl-26 pristinamycine $II_B$
[(isoquinolyl-1)-2 méthyl-2 éthyl] sulfinyl-26 pristinamycine $II_B$
(imidazolyl-2 méthyl-2 éthyl) sulfinyl-26 pristinamycine $II_B$
(diméthylamino-2 phényl-3 propyl) sulfinyl-26 pristinamycine $II_B$
(diméthylamino-2 butyl) sulfinyl-26 pristinamycine $II_B$
(azétidinyl-3) sulfonyl-26 pristinamycine $II_B$
(méthyl-1 azétidinyl-3) sulfonyl-26 pristinamycine $II_B$
(éthyl-1 azétidinyl-3) sulfonyl-26 pristinamycine $II_B$
(isopropyl-1 azétidinyl-3) sulfonyl-26 pristinamycine $II_B$
(pyrrolidinyl-3) sulfonyl-26 pristinamycine $II_B$
(méthyl-1 pyrrolidinyl-3) sulfonyl-26 pristinamycine $II_B$
(éthyl-1 pyrrolidinyl-3) sulfonyl-26 pristinamycine $II_B$
(isopropyl-1 pyrrolidinyl-3) sulfonyl-26 pristinamycine $II_B$
(pipéridyl-3) sulfonyl-26 pristinamycine $II_B$
(méthyl-1 pipéridyl-3) sulfonyl-26 pristinamycine $II_B$
(éthyl-1 pipéridyl-3) sulfonyl-26 pristinamycine $II_B$
(pipéridyl-4) sulfonyl-26 pristinamycine $II_B$
(méthyl-1 pipéridyl-4) sulfonyl-26 pristinamycine $II_B$
(éthyl-1 pipéridyl-4) sulfonyl-26 pristinamycine $II_B$
(azépinyl-3) sulfonyl-26 pristinamycine $II_B$
(azépinyl-4) sulfonyl-26 pristinamycine $II_B$
(cyclopropylamino-2 éthyl) sulfonyl-26 pristinamycine $II_B$

(cyclobutylamino-2 éthyl) sulfonyl-26 pristinamycine $II_B$
(cyclopentylamino-2 éthyl) sulfonyl-26 pristinamycine $II_B$
(cyclohexylamino-2 éthyl) sulfonyl-26 pristinamycine $II_B$
(N-cyclohexyl N-méthyl-amino-2 éthyl) sulfonyl-26 pristinamycine $II_B$
(méthylamino-2 éthyl) sulfonyl-26 pristinamycine $II_B$
(éthylamino-2 éthyl) sulfonyl-26 pristinamycine $II_B$
(propylamino-2 éthyl) sulfonyl-26 pristinamycine $II_B$
(isopropylamino-2 éthyl) sulfonyl-26 pristinamycine $II_B$
(butylamino-2 éthyl) sulfonyl-26 pristinamycine $II_B$
(isobutylamino-2 éthyl) sulfonyl-26 pristinamycine $II_B$
(n.decylamino-2 éthyl) sulfonyl-26 pristinamycine $II_B$
(diméthylamino-2 éthyl) sulfonyl-26 pristinamycine $II_B$
(diéthylamino-2 éthyl) sulfonyl-26 pristinamycine $II_B$
(dipropylamino-2 éthyl) sulfonyl-26 pristinamycine $II_B$
(diisopropylamino-2 éthyl) sulfonyl-26 pristinamycine $II_B$
(dibutylamino-2 éthyl) sulfonyl-26 pristinamycine $II_B$
(diisobutylamino-2 éthyl) sulfonyl-26 pristinamycine $II_B$
(N-éthyl N-méthyl-amino-2 éthyl) sulfonyl-26 pristinamycine $II_B$
[(azétidinyl-1)-2 éthyl] sulfonyl-26 pristinamycine $II_B$
[(pyrrolidinyl-1)-2 éthyl] sulfonyl-26 pristinamycine $II_B$
(pipéridino-2 éthyl) sulfonyl-26 pristinamycine $II_B$
[(azépinyl-1)-2 éthyl] sulfonyl-26 pristinamycine $II_B$
(morpholino-2 éthyl) sulfonyl-26 pristinamycine $II_B$
[(pipérazinyl-1)-2 éthyl] sulfonyl-26 pristinamycine $II_B$
[(méthyl-4 pipérazinyl-1)-2 éthyl] sulfonyl-26 pristinamycine $II_B$
[(méthyl-4 homopipérazinyl-1)-2 éthyl] sulfonyl-26 pristinamycine $II_B$
[(imidazolyl-1)-2 éthyl] sulfonyl-26 pristinamycine $II_B$
(diméthylaminocarbamoyloxy-2 éthyl) sulfonyl-26 pristinamycine $II_B$
(diéthylaminocarbamoyloxy-2 éthyl) sulfonyl-26 pristinamycine $II_B$
(diisopropylaminocarbamoyloxy-2 éthyl) sulfonyl-26 pristinamycine $II_B$
[(méthyl-4 pipérazinyl-1)carbamoyloxy-2 éthyl] sulfonyl-26 pristinamycine $II_B$
[(azétidinyl-2)-2 éthyl] sulfonyl-26 pristinamycine $II_B$
[(azétidinyl-3)-2 éthyl] sulfonyl-26 pristinamycine $II_B$
[(pyrrolidinyl-2)-2 éthyl] sulfonyl-26 pristinamycine $II_B$
[(pyrrolidinyl-3)-2 éthyl] sulfonyl-26 pristinamycine $II_B$
[(pipéridyl-2)-2 éthyl] sulfonyl-26 pristinamycine $II_B$
[(pipéridyl-3)-2 éthyl] sulfonyl-26 pristinamycine $II_B$
[(pipéridyl-4)-2 éthyl] sulfonyl-26 pristinamycine $II_B$
[(azépinyl-2)-2 éthyl] sulfonyl-26 pristinamycine $II_B$
[(azépinyl-3)-2 éthyl] sulfonyl-26 pristinamycine $II_B$
[(azépinyl-4)-2 éthyl] sulfonyl-26 pristinamycine $II_B$
[(quinolyl-3)-2 éthyl] sulfonyl-26 pristinamycine $II_B$
[(quinolyl-4)-2 éthyl] sulfonyl-26 pristinamycine $II_B$
[(tétrahydro-1,2,3,4 isoquinolyl-2)-2 éthyl] sulfonyl-26 pristinamycine $II_B$
[(isoquinolyl-1)-2 éthyl] sulfonyl-26 pristinamycine $II_B$
(imidazolyl-2 éthyl) sulfonyl-26 pristinamycine $II_B$
(cyclopropylamino-2 méthyl-1 éthyl) sulfonyl-26 pristinamycine $II_B$
(cyclobutylamino-2 méthyl-1 éthyl) sulfonyl-26 pristinamycine $II_B$
(cyclopentylamino-2 méthyl-1 éthyl) sulfonyl-26 pristinamycine $II_B$
(cyclohexylamino-2 méthyl-1 éthyl) sulfonyl-26 pristinamycine $II_B$
[(N-cyclohexyl N-méthyl-amino)-2 méthyl-1 éthyl] sulfonyl-26 pristinamycine $II_B$
(méthylamino-2 méthyl-1 éthyl) sulfonyl-26 pristinamycine $II_B$
(éthylamino-2 méthyl-1 éthyl) sulfonyl-26 pristinamycine $II_B$
(méthyl-1 propylamino-2 éthyl) sulfonyl-26 pristinamycine $II_B$
(isopropylamino-2 méthyl-1 éthyl) sulfonyl-26 pristinamycine $II_B$
(butylamino-2 méthyl-1 éthyl) sulfonyl-26 pristinamycine $II_B$
(isobutylamino-2 méthyl-1 éthyl) sulfonyl-26 pristinamycine $II_B$
(méthyl-1 n.décylamino-2 éthyl) sulfonyl-26 pristinamycine $II_B$
(diméthylamino-2 méthyl-1 éthyl) sulfonyl-26 pristinamycine $II_B$
(diéthylamino-2 méthyl-1 éthyl) sulfonyl-26 pristinamycine $II_B$
(dipropylamino-2 méthyl-1 éthyl) sulfonyl-26 pristinamycine $II_B$
(diisopropylamino-2 méthyl-1 éthyl) sulfonyl-26 pristinamycine $II_B$
(dibutylamino-2 méthyl-1 éthyl) sulfonyl-26 pristinamycine $II_B$
(diisobutylamino-2 méthyl-1 éthyl) sulfonyl-26 pristinamycine $II_B$
[(N-éthyl N-méthyl-amino)-2 méthyl-1 éthyl] sulfonyl-26 pristinamycine $II_B$
[(azétidinyl-1)-2 méthyl-1 éthyl] sulfonyl-26 pristinamycine $II_B$
[méthyl-1 (pyrrolidinyl-1)-2 éthyl] sulfonyl-26 pristinamycine $II_B$
(méthyl-1 pipéridino-2 éthyl) sulfonyl-26 pristinamycine $II_B$
[(azépinyl-1)-2 méthyl-1 éthyl] sulfonyl-26 pristinamycine $II_B$
(méthyl-1 morpholino-2 éthyl) sulfonyl-26 pristinamycine $II_B$
[méthyl-1 (pipérazinyl-1)-2 éthyl] sulfonyl-26 pristinamycine $II_B$
[(méthyl-4 pipérazinyl-1)-2 méthyl-1 éthyl] sulfonyl-26 pristinamycine $II_B$
[(méthyl-4 homopipérazinyl-1)-2 méthyl-1 éthyl] sulfonyl-26 pristinamycine $II_B$

[(imidazolyl-1)-2-méthyl-1 éthyl] sulfonyl-26 pristinamycine $II_B$
(diméthylaminocarbamoyloxy-2 méthyl-1 éthyl) sulfonyl-26 pristinamycine $II_B$
(diéthylaminocarbamoyloxy-2 méthyl-1 éthyl) sulfonyl-26 pristinamycine $II_B$
(diisopropylaminocarbamoyloxy-2 méthyl-1 éthyl] sulfonyl-26 pristinamycine $II_B$
[(méthyl-4 pipérazinyl-1) carbamoyloxy-2 méthyl-1 éthyl] sulfonyl-26 pristinamycine $II_B$
[(azétidinyl-2)-2 méthyl-1 éthyl] sulfonyl-26 pristinamycine $II_B$
[(azétidinyl-3)-2 méthyl-1 éthyl] sulfonyl-26 pristinamycine $II_B$
[méthyl-1 (pyrrolidinyl-2)-2 éthyl] sulfonyl-26 pristinamycine $II_B$
[méthyl-1 (pyrolidinyl-3)-2 éthyl] sulfonyl-26 pristinamycine $II_B$
[méthyl-1 (pipéridyl-2)-2 éthyl] sulfonyl-26 pristinamycine $II_B$
[méthyl-1 (pipéridyl-3)-2 éthyl] sulfonyl-26 pristinamycine $II_B$
[méthyl-1 (pipéridyl-4)-2 éthyl] sulfonyl-26 pristinamycine $II_B$
[(azépinyl-2)-2 méthyl-1 éthyl] sulfonyl-26 pristinamycine $II_B$
[(azépinyl-3)-2 méthyl-1 éthyl] sulfonyl-26 pristinamycine $II_B$
[(azépinyl-4)-2 méthyl-1 éthyl] sulfonyl-26 pristinamycine $II_B$
[méthyl-1 (quinolyl-3)-2 éthyl] sulfonyl-26 pristinamycine $II_B$
[méthyl-1 (quinolyl-4)-2 éthyl] sulfonyl-26 pristinamycine $II_B$
[méthyl-1 (tétrahydro-1,2,3,4 isoquinolyl-2)-2 éthyl] sulfonyl-26 pristinamycine $II_B$
[(isoquinolyl-1)-2 méthyl-1 éthyl] sulfonyl-26 pristinamycine $II_B$
(imidazolyl-2 méthyl-1 éthyl) sulfonyl-26 pristinamycine $II_B$
(cyclopropylamino-2 méthyl-2 éthyl) sulfonyl-26 pristinamycine $II_B$
(cyclobutylamino-2 méthyl-2 éthyl) sulfonyl-26 pristinamycine $II_B$
(cyclopentylamino-2 méthyl-2 éthyl) sulfonyl-26 pristinamycine $II_B$
(cyclohexylamino-2 méthyl-2 éthyl) sulfonyl-26 pristinamycine $II_B$
[(N-cyclohexyl N-méthyl-amino)-2 méthyl-2 éthyl] sulfonyl-26 pristinamycine $II_B$
(méthylamino-2 méthyl-2 éthyl) sulfonyl-26 pristinamycine $II_B$
(éthylamino-2 méthyl-2 éthyl) sulfonyl-26 pristinamycine $II_B$
(méthyl-2 propylamino-2 éthyl) sulfonyl-26 pristinamycine $II_B$
(isopropylamino-2 méthyl-2 éthyl) sulfonyl-26 pristinamycine $II_B$
(butylamino-2 méthyl-2 éthyl) sulfonyl-26 pristinamycine $II_B$
(isobutylamino-2 méthyl-2 éthyl) sulfonyl-26 pristinamycine $II_B$
(méthyl-2 n.décylamino-2 éthyl) sulfonyl-26 pristinamycine $II_B$
(diméthylamino-2 méthyl-2 éthyl) sulfonyl-26 pristinamycine $II_B$
(diéthylamino-2 méthyl-2 éthyl) sulfonyl-26 pristinamycine $II_B$
(dipropylamino-2 méthyl-2 éthyl) sulfonyl-26 pristinamycine $II_B$
(diisopropylamino-2 méthyl-2 éthyl) sulfonyl-26 pristinamycine $II_B$
(dibutylamino-2 méthyl-2 éthyl) sulfonyl-26 pristinamycine $II_B$
(diisobutylamino-2 méthyl-2 éthyl) sulfonyl-26 pristinamycine $II_B$
[(N-éthyl N-méthyl-amino)-2 méthyl-2 éthyl] sulfonyl-26 pristinamycine $II_B$
[(azétidinyl-1)-2 méthyl-2 éthyl] sulfonyl-26 pristinamycine $II_B$
[méthyl-2 (pyrrolidinyl-1)-2 éthyl] sulfonyl-26 pristinamycine $II_B$
(méthyl-2 pipéridino-2 éthyl) sulfonyl-26 pristinamycine $II_B$
[(azépinyl-1)-2 méthyl-2 éthyl] sulfonyl-26 pristinamycine $II_B$
(méthyl-2 morpholino-2 éthyl) sulfonyl-26 pristinamycine $II_B$
[méthyl-2 (pipérazinyl-1)-2 éthyl] sulfonyl-26 pristinamycine $II_B$
[(méthyl-4 pipérazinyl-1)-2 méthyl-2 éthyl] sulfonyl-26 pristinamycine $II_B$
[(méthyl-4 homopipérazinyl-1)-2 méthyl-2 éthyl] sulfonyl-26 pristinamycine $II_B$
[(imidazolyl-1)-2 méthyl-2 éthyl] sulfonyl-26 pristinamycine $II_B$
(diméthylaminocarbamoyloxy-2 méthyl-2 éthyl) sulfonyl-26 pristinamycine $II_B$
(diéthylaminocarbamoyloxy-2 méthyl-2 éthyl) sulfonyl-26 pristinamycine $II_B$
(diisopropylaminocarbamoyloxy-2 méthyl-2 éthyl) sulfonyl-26 pristinamycine $II_B$
[(méthyl-4 pipérazinyl-1)carbamoyloxy-2 méthyl-2 éthyl] sulfonyl-26 pristinamycine $II_B$
[(azétidinyl-2)-2 méthyl-2 éthyl] sulfonyl-26 pristinamycine $II_B$
[(azétidinyl-3)-2 méthyl-2 éthyl] sulfonyl-26 pristinamycine $II_B$
[méthyl-2 (pyrrolidinyl-2)-2 éthyl] sulfonyl-26 pristinamycine $II_B$
[méthyl-2 (pyrrolidinyl-3)-2 éthyl] sulfonyl-26 pristinamycine $II_B$
[méthyl-2 (pipéridyl-2)-2 éthyl] sulfonyl-26 pristinamycine $II_B$
[méthyl-2 (pipéridyl-3)-2 éthyl] sulfonyl-26 pristinamycine $II_B$
[méthyl-2 (pipéridyl-4)-2 éthyl] sulfonyl-26 pristinamycine $II_B$
[(azépinyl-2)-2 méthyl-2 éthyl] sulfonyl-26 pristinamycine $II_B$
[(azépinyl-3)-2 méthyl-2 éthyl] sulfonyl-26 pristinamycine $II_B$
[(azépinyl-4)-2 méthyl-2 éthyl] sulfonyl-26 pristinamycine $II_B$
[méthyl-2 (quinolyl-3)-2 éthyl] sulfonyl-26 pristinamycine $II_B$
[méthyl-2 (quinolyl-4)-2 éthyl] sulfonyl-26 pristinamycine $II_B$

[méthyl-2 (tétrahydro-1,2,3,4 isoquinolyl-2)-2 éthyl] sulfonyl-26 pristinamycine $II_B$

[(isoquinolyl-1)-2 méthyl-2 éthyl] sulfonyl-26 pristinamycine $II_B$

(imidazolyl-2 méthyl-2 éthyl) sulfonyl-26 pristinamycine $II_B$

(diméthylamino-2 phényl-3 propyl) sulfonyl-26 pristinamycine $II_B$

(diméthylamino-2 butyl) sulfonyl-26 pristinamycine $II_B$

Selon l'invention, les produits de formule générale (I) peuvent être préparés par oxydation d'un dérivé de pristinamycine $II_B$, de son sel ou d'un dérivé protégé, de formule générale:

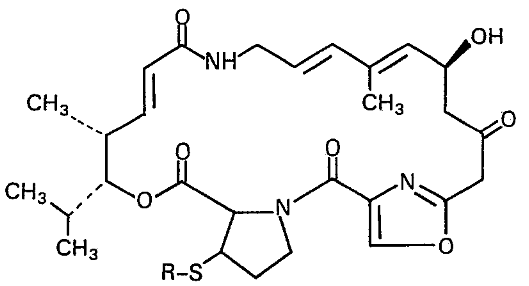

(II)

dans laquelle R est défini comme précedemment, étant entendu que dans les cas où R contient un hétérocycle soufré, l'atome de soufre peut se trouver à l'état de sulfure, de sulfoxyde ou de sulfone.

La réaction s'effectue généralement au moyen d'un agent d'oxydation éventuellement préparé in situ, en milieu aqueux ou dans un solvant organique, de préférence un solvant chloré (chlorure de méthylène, dichloro-1,2 éthane, chloroforme par exemple) ou un alcool (méthanol, t.butanol par exemple) ou un mélange de ces solvants. Eventuellement in opère sous azote.

Parmi les agents d'oxydation convenables pour obtenir un produit de formule générale (I) dans laquelle n = 1, peuvent être cités les peracides organiques: acides percarboxyliques ou persulfoniques (par exemple les acides peracétique, pertrifluoracétique, performique, perbenzoïque, m.chloroperbenzoïque, p.nitroperbenzoïque, permaléique, monoperphtalique, percamphorique ou p.toluènepersulfonique) ou les peracides minéraux (par exemple acide periodique ou persulfurique).

Lorsque l'on veut obtenir un produit de formule générale (I) dans laquelle n = 2, on opère avantageusement en présence de dioxyde de sélénium et d'eau oxygénée sur le sel du produit de formule générale (II), ou en présence d'un peracide tel que ceux cités ci-dessus notamment l'acide pertrifluoracétique, ou l'acide m.chloroperbenzoïque.

Lorsque l'on met en œuvre le dérivé de pristinamycine $II_B$ de formule générale (II) sous forme de sel, on utilise les sels formés avec les acides organiques ou minéraux, de préférence avec les acides trifluoracétique, tartrique, acétique, benzoïque ou chlorhydrique.

Lorsque le produit de formule générale (II) est utilisé sous forme de sel ou de dérivé protégé, la réaction s'effectue avatangeusement à une température comprise entre −40 et 50°C.

Lorsque l'on veut obtenir un produit de formule générale (I) dans laquelle n = 1, il est également intéressant d'opérer à partir du dérivé de pristinamycine $II_B$ de formule générale (II) en présence d'un bicarbonate alcalin (bicarbonate de sodium par exemple) à une température comprise entre −60 et −40°C.

Lorsque R contient un substituant alcoylamino ou cycloalcoylamino, il est également possible de mettre en œuvre un dérivé protégé du produit de formule générale (II), ce dernier peut être protégé par tout groupement protecteur d'amine dont la mise en place et l'élimination n'affectent pas le reste de la molécule; on utilise avantageusement le groupement trifluoracétyle qui peut être éliminé après la réaction par traitement par un bicarbonate alcalin (bicarbonate de sodium ou de potassium) en solution aqueuse.

Les produits de formule générale (II) peuvent être préparés par action d'un produit de formule générale:

R-H (III)

dans laquelle R est défini comme précédemment sur le produit de formule:

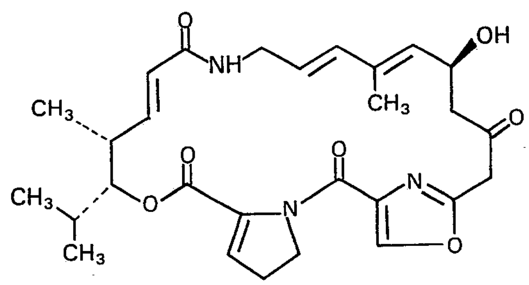

(IV)

c'est-à-dire la pristinamycine $II_A$.

La réaction s'effectue généralement dans un solvant organique tel qu'un alcool comme le méthanol ou l'éthanol ou un solvant chloré comme le chlorure de méthylène, le dichloro-1,2 éthane ou le chloroforme, ou dans un mélange de ces solvants (par exemple chlorure de méthylène-méthanol) à une température comprise entre −30 et 50°C.

Il peut être parfois avantageux d'opérer en présence d'une amine tertiaire, par exemple la triéthylamine, ou d'une éthanolamine (diméthyléthanolamine par exemple).

Il est entendu pour l'homme du métier que, lorsque R représente un radical qui contient une fonction amine secondaire pouvant interférer avec la réaction, cette dernière fonction devra être préalablement protégée avant de faire réagir le produit de formule générale (III) sur le produit de formule (IV). On peut utiliser à cet effet tout moyen habituel permettant de bloquer une fonction amine secondaire sous forme d'un radical labile. Il est particulièrement avantageux d'utiliser comme radical bloquant le radical trifluoracétyle qui peut être éliminé comme décrit précédemment. Cependant, dans un tel cas il n'est pas absolument nécessaire d'éliminer le radical protecteur, le dérivé protégé peut être directement mis en œuvre dans la réaction d'oxydation.

Selon l'invention, les produits de formule générale (I) dans laquelle n est égal à 2, peuvent également être préparés par oxydation d'un produit de formule générale (I) dans laquelle n est égal à 1.

La réaction s'effectue dans des conditions analogues aux conditions décrites précédemment pour obtenir un produit de formule générale (I) dans laquelle n = 2 à partir d'un dérivé de pristinamycine $II_B$ de formule générale (II).

Les nouveaux produits de formule générale (I) peuvent être purifiés par les méthodes connues, par exemple par cristallisation, chromatographie ou extractions successives en milieu acide et basique. Pour l'homme du métier connaissant la sensibilité des synergistines en milieu alcalin, on entend par «milieu basique» un milieu juste suffisamment alcalin pour libérer la substance-mère de son sel d'addition avec un acide, c'est-à-dire un milieu dont le pH n'excède pas 8.

Il est bien connu que les synergistines obtenues par fermentation constituent des produits très recherchés par les médecins pour le traitement de beaucoup d'affections dues à des bactéries Gram-positives (du genre Staphylocoques, Streptocoques, pneumocoques, entérocoques) et Gram-négatives (du genre Haemophilus, gonocoques, méningocoques). Toutefois ces produits présentent l'inconvénient d'être insolubles en milieu aqueux et ne peuvent donc être administrés que par voie orale, généralement sous forme de gellules, de dragées ou de comprimés. Compte tenu de cette insolubilité, il est impossible d'utiliser les synergistines connues jusqu'ici lorsque le malade n'est pas en l'état de déglutir; c'est notamment le cas en pédiatrie et en réanimation, alors que le spectre d'activité de ces produits en ferait une indication précieuse dans un grand nombre de circonstances, par exemple dans les cas de septicémies comateuses.

Les nouveaux produits selon l'invention présentent l'avantage considérable de pouvoir être solubilisés dans l'eau, généralement à l'état de sels, aux doses thérapeutiques utilisables et d'exalter par un phénomène de synergie l'action abtibactérienne de la pristinamycine $I_A$, de la virginiamycine S ou de dérivés de synergistines solubles de formule générale:

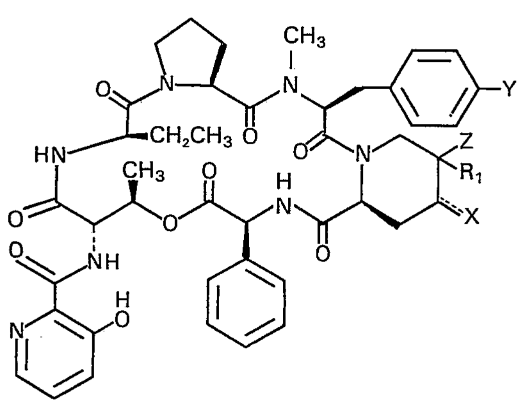

(V)

dans laquelle Y représente un atome d'hydrogène ou un radical diméthylamino et

1) ou bien ------- représente une liaison simple, Z et $R_1$ représentent un atome d'hydrogène et X représente un radical de formule générale:

$$-N\begin{matrix} R_2 \\ R_3 \end{matrix} \qquad (VI)$$

dans laquelle

– soit $R_2$ représente un atome d'hydrogène et $R_3$ représente un radical hydroxy ou alcoyle éventuellement substitué par un radical carboxy, alcoyloxycarbonyle, hydroxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou bien $R_3$ représente un radical cycloalcoyle contenant 3 à 7 atomes de carbone ou un hétérocycle saturé à 4 à 7 chaînons choisi parmi les cycles azétidine, pyrrolidine, pipéridine et azépine, ces hétérocycles pouvant être éventuellement substitués sur l'atome d'azote par un radical alcoyle,

– soit $R_2$ représente un radical formyle ou alcoylcarbonyle et $R_3$ représente un radical alcoyle substitué par un radical carboxy, alcoylamino ou dialcoylamino dont les radicaux alcoyle peuvent former avec l'atome d'azote auquel ils sont rattachés un hétérocycle à 4 à 7 chaînon choisi parmi azétidinyle, pyrrolidinyle, pipéridinyle, pipérazinyle, N-alcoylpipérazinyle ou azépinyle, ou bien $R_3$ représente un hétérocycle à 4 à 7 chaînons choisi parmi les cycles azétidine, pyrrolidine, pipéridine et azépine, ces hetérocycles pouvant être substitués sur l'atome d'azote par un radical alcoyle,

– soit $R_2$ et $R_3$, identiques ou différents, représentent un radical alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle

– soit $R_2$ et $R_3$ forment ensemble avec l'atome d'azote auquel ils sont rattachés un hétérocycle à 4 à 7 chaînons choisi parmi les cycles azétidine, pyrrolidine, pipéridine, morpholine et pipérazine éventuellement substitué par un radical alcoyle,

2) ou bien ------- représente une double liaison, X représente un atome d'oxygène et Z représente un radical de formule générale:

$$-CH\begin{matrix} R_4 \\ R_5 \end{matrix} \qquad (VII)$$

défini de la manière suivante:

a) soit $R_1$ et $R_5$ représentent chacun un atome d'hydrogène et $R_4$ représente un radical pyrrolidinyl-3 thio ou pipéridyl-3 ou 4 thio (ces radicaux étant éventuellement substitués par un radical alcoyle) ou bien $R_4$ représente un radical alcoylthio substitué par un ou deux radicaux hydroxysulfonyle, alcoylamino, dialcoylamino (éventuellement substitué par un radical mercapto ou dialcoylamino) ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle),

b) soit $R_1$ et $R_5$ forment ensemble une liaison de valence et $R_4$ représente un radical pyrrolidinyl-3 amino, pipéridinyl-3 ou 4 amino, pyrrolidinyl-3 oxy, pipéridyl-3 ou 4 oxy, pyrrolidinyl-3 thio, pipéridyl-3 ou 4 thio (ces radicaux étant éventuellement substitués sur l'atome d'azote du cycle par un radical alcoyle), ou bien $R_4$ représente un radical alcoylamino, alcoyloxy ou alcoylthio substitués par un ou deux radicaux hydroxysulfonyle, alcoylamino), dialcoylamino (éventuellement substitué par un radical dialcoylamino), trialcoylammonio ou imidazolyle-4 ou 5 ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle), étant entendu que les radicaux alcoyle et portions alcoyle se rapportant aux symboles de la formule générale (V) contiennent 1 à 5 atomes de carbone et sont en chaîne droite ou ramifiée.

Certains des dérivés de synergistines de formule générale (V) peuvent présenter des formes isomères. Il est entendu que ces formes isomères aussi bien que leurs mélanges peuvent être avantageusement associés aux produits de formule générale (I).

Les produits de formule générale (V) définis comme précédemment en 1) à l'exception de ceux pour lesquels $R_2$ représente un radical formyle ou alcoylcarbonyle, peuvent être préparés par action d'une amine de formule générale:

$$HN\Big\langle{}^{R_2}_{R_3} \qquad (VIII)$$

dans laquelle $R_2$ et $R_3$ sont définis comme ci-dessus, sur une synergistine de formule générale:

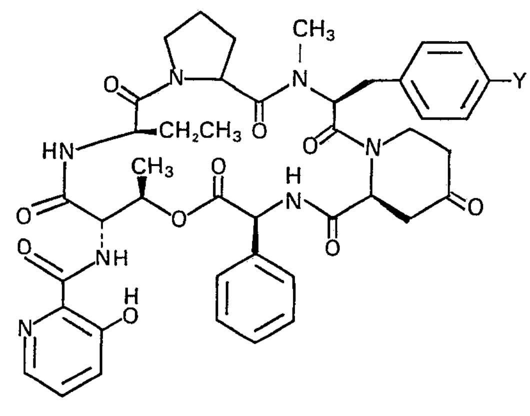

(IX)

dans laquelle Y représente un atome d'hydrogène (virginiamycine S) ou le radical diméthylamino (pristinamycine $I_A$), en présence d'un cyanoborohydrure alcalin.

On opère généralement avec un excès d'amine de formule générale (VIII) en présence d'un cyanoborohydrure alcalin comme le cyanoborohydrure de sodium, dans un solvant organique tel qu'un alcool dans lequel on a dissous de l'acide chlorhydrique gazeux (méthanol chlorhydrique ou éthanol chlorhydrique), à une température comprise entre 0°C et la température de reflux du mélange réactionnel, de préférence à une température voisine de 20°C.

La réaction peut être avantageusement effectuée en présence d'un agent de dessication tel que des tamis moléculaires.

Les produits de formule générale (V) définis comme précédemment en 1) dans laquelle $R_2$ représente un radical formyle ou alcoylcarbonyle et $R_3$ représente un radical alcoyle substitué par un radical carboxy, alcoylamino ou dialcoylamino dont les radicaux alcoyles forment éventuellement avec l'atome d'azote auquel ils sont rattachés, un hétérocycle à 4 à 7 chaînons choisis parmi azétidinyle, pyrrolidinyle, pipéridinyle, pipérazinyle, alcoylpipérazinyle ou azépinyle, ou représente un hétérocycle saturé à 4 à 7 chaînons choisi parmi les cycles azétidine, pyrrolidine, pipéridine et azépine, ces hétérocycles pouvant être substitués sur l'atome d'azote par un radical alcoyle, et Y est défini comme précédemment, peuvent être préparés par action d'un produit de formule générale:

$$R_6 - CO - Q \qquad (X)$$

dans laquelle $R_6$ représente un atome d'hydrogène ou un radical alcoyle et Q représente un atome d'halogène ou un radical alcoylcarbonyloxy, sur un produit de formule générale:

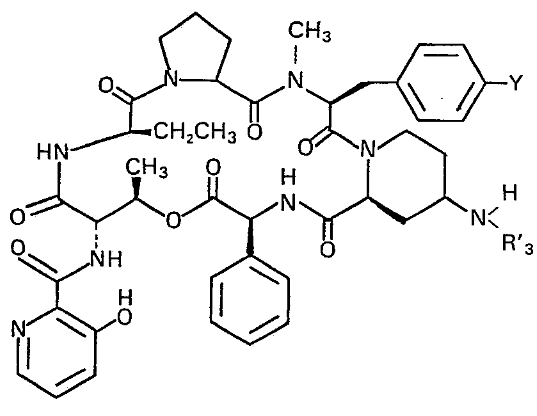

(XI)

dans laquelle Y est défini comme précédemment et $R'_3$ a la définition de $R_3$ correspondante donnée ci-dessus.

La réaction s'effectue généralement dans un solvant organique tel que la pyridine, dans un solvant chloré (chlorure de méthylène) ou un éther (tétrahydrofuranne) en présence d'un accepteur d'acide tel qu'une base organique comme la triéthylamine ou une base minérale telle qu'un carbonate ou un bicarbonate alcalin comme le bicarbonate de sodium, en opérant à une température comprise entre 0 et 80°C.

Il est entendu que, lorsque $R'_3$ représente un radical contenant une fonction amine secondaire, ladite fonction doit être protégée avant de faire réagir le produit de formule générale (X) sur le produit de formule générale (XI). La protection s'effectue dans les conditions décrites précédemment pour la préparation du produit de formule générale (II).

Il est également entendu que, lorsque $R_2$ et/ou $R_3$, dans la formule générale (VIII), représentent un radical contenant une fonction amine secondaire, cette dernière doit être préalablement protégée avant de faire réagir le produit de formule générale (VIII) sur le produit de formule générale (IX). Le blocage et le déblocage s'effectuent comme décrit précédemment.

Les produits de formule générale (V) définis comme précédemment en 2), pour lesquels Y est défini comme précédemment et les autres symboles sont définis comme précédemment en 2) a) peuvent être préparés par action d'un produit de formule générale:

$$R'_4 - H \qquad (XII)$$

dans laquelle $R'_4$ a la définition de $R_4$ donnée précédemment en 2) a) sur le produit de formule générale:

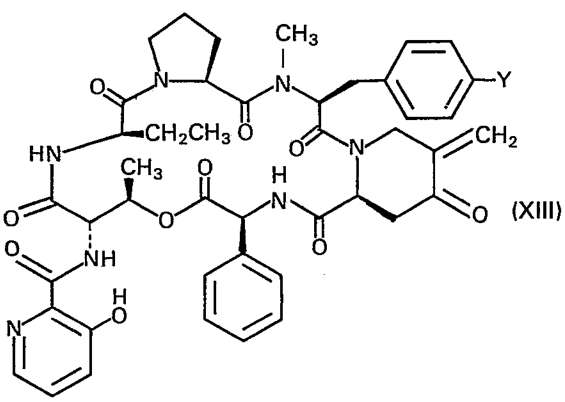

(XIII)

dans laquelle Y est défini comme précédemment.

On opère généralement dans un solvant organique tel qu'un alcool comme le méthanol ou un solvant chloré comme le chloroforme ou un mélange de ces solvants, à une température comprise entre 0°C et la température de reflux du mélange réactionnel, de préférence à une température voisine de 20°C.

Les produits de formule générale (XIII) peuvent être préparés par action d'un borohydrure alcalin tel que le cyanoborohydrure de sodium sur un produit de formule générale:

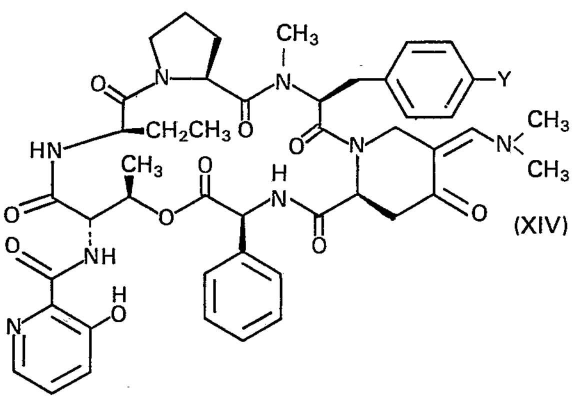

(XIV)

dans laquelle Y est défini comme précédemment.

On opère généralement dans un solvant organique tel qu'un éther comme le tétrahydrofuranne ou un alcool par exemple l'isopropanol en présence d'un acide tel que l'acide trifluoroacétique, à une température comprise entre 0°C et la température de reflux du mélange réactionnel, de préférence à une température voisine de 20°C.

Les produits de formule générale (XIV) peuvent être obtenus par action d'un produit de formule:

$$\begin{matrix} CH_3 \\ CH_3 \end{matrix} > N - CH < \begin{matrix} X_1 \\ X_2 \end{matrix} \qquad (XV)$$

dans laquelle ou bien $X_1$ représente un radical alcoyloxy et $X_2$ représente un radical alcoyloxy ou diméthylamino, ou bien $X_1$ et $X_2$ représentent tous les deux un radical diméthylamino, sur un produit de formule générale (IX).

Dans la pratique, il est avantageux de faire réagir le tert-butoxy bis(diméthylamino) méthane sur le produit de formule générale (IX), en opérant dans un solvant organique tel qu'un solvant chloré comme le dichloro-1,2 éthane ou un amide (diméthylformamide par exemple) à une température comprise entre 0 et 80°C, de préférence à une température voisine de 20°C.

Les produits de formule générale (XV) peuvent être préparés selon les méthodes décrites par H. BREDERECK et coll., Chem. Ber., 101, 41 et 3058 (1968) et Chem. Ber., 106, 3725 (1973).

Les produits de formule générale (V) dans laquelle Y est défini comme précédemment et les autres symboles sont définis comme précédemment en 2) b) à l'exception pour $R_4$ de représenter un radical pyrrolidinyl-3 oxy, pipéridyl-3 ou 4 oxy ou alcoyloxy éventuellement substitués comme défini en 2) b), peuvent être préparés par action d'un produit de formule générale:

$$R''_4 - H \qquad (XVI)$$

dans laquelle $R''_4$ a la définition de $R_4$ donnée ci-dessus, sur un produit de formule générale (XIV) dans laquelle Y est défini comme précédemment.

La réaction s'effectue en milieu organique, en présence d'un acide (par exemple l'acide acétique ou un mélange d'acide acétique et de quantités catalytiques d'acide trifluoracétique), en présence ou non d'un solvant, à une température comprise entre 0 et 50°C; de préférence à une température voisine de 20°C.

Le cas échéant, le solvant peut être choisi parmi les solvants organiques comme les éthers (tétrahydrofuranne), les alcools (éthanol) et les solvants chlorés (chlorure de méthylène ou chloroforme par exemple).

Les produits de formule générale (V) dans laquelle Y est défini comme précédemment et les autres symboles sont définis comme précédemment en 2) b) peuvent être préparés par action d'un produit de formule générale:

$$R'''_4 - H \qquad (XVII)$$

dans laquelle R'''$_4$ est défini comme $R_4$ en 2) b), sur un produit de formule générale:

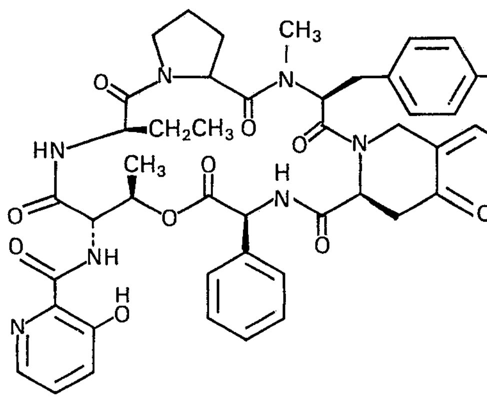

(XVIII)

dans laquelle Y est défini comme précédemment et $Z_1$ représente un radical tosyloxy, acétyloxy, triméthylsilyloxy ou dialcoyloxyphosphoryloxy dont les parties alcoyle contiennent 1 à 4 atomes de carbone en chaîne droite ou ramifiée, ou bien $Z_1$ représente un atome de chlore.

On opère généralement dans un solvant organique tel qu'un éther comme le tétrahydrofuranne, un alcool comme l'éthanol ou un solvant chloré (chlorure de méthylène ou chloroforme par exemple) à une température voisine de 20°C. La réaction s'effectue en milieu basique, par exemple en présence d'un hydrure alcalin ou d'un alcoolate alcalin comme l'éthylate de sodium ou le tert-butylate de potassium.

Lorsque R'''$_4$ est autre qu'alcoyloxy substitué ou hétérocyclyloxy, il est également possible d'opérer soit en milieu neutre à une température comprise entre 0 et 50°C, dans l'un des solvants cités ci-dessus en milieu acide dans les conditions identiques à celles décrites précédemment pour l'action d'un produit de formule générale (XVI) sur un produit de formule générale (XIV).

Les produits de formule générale (XVIII) peuvent être préparés par hydrolyse acide d'un produit de formule générale (XIV) pour obtenir un produit de formule générale:

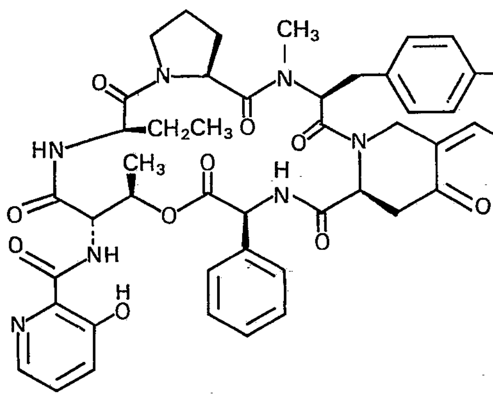

(XIX)

suivie

α) ou bien de l'action d'un produit de formule générale:

$$Z'_1 - X \qquad (XX)$$

dans laquelle X représente un atome d'halogène et $Z'_1$ a la définition donnée précédemment pour $Z_1$ à l'exception de représenter un atome de chlore

β) ou bien de l'action d'un produit de formule:

$$(C_6H_5)_3\,P\,Cl_2 \qquad (XXI)$$

pour obtenir un produit de formule générale (XVIII) dans laquelle $Z_1$ représente un atome de chlore.

L'hydrolyse du produit de formule générale (XIV) en produit de formule générale (XVIII) s'effectue au moyen d'une solution aqueuse d'un acide minéral tel qu'une solution aqueuse 0,1 N d'acide chlorhydrique en opérant à une température voisine de 20°C.

La réaction du produit de formule générale (XX) sur le produit de formule générale (XIX) s'effectue généralement dans un solvant organique tel que le chlorure de méthylène en présence d'un accepteur d'acide tel qu'une base organique comme la triéthylamine ou une base minérale comme un carbonate ou un bicarbonate alcalin, par exemple le bicarbonate de sodium ou de potassium. On opère généralement à une température comprise entre −20 et +20°C.

La réaction du produit de formule générale (XXI) sur le produit de formule générale (XIX) s'effectue généralement dans un solvant chloré tel que le chlorure de méthylène à une température comprise entre −20 et +20°C.

Les produits de formules générales (III), (VIII), (XII), (XVI) et (XVII) peuvent être préparés selon ou par analogie avec les méthodes décrites ci-après dans les exemples et notamment selon:

– G.G. Urquart et coll., Org. Synth., 21, 36 (1941)
– A.I. Vogel, J. Chem. Soc., 1922 (1948)
– J.H. Chapman et L.N. Owen, J. chem. Soc., 589 (1950)
– H.R. Snyder et coll., J. Am. Soc., 69, 2672 (1947)
– D.D. Reynolds et coll., J. Org. Chem., 26, 5125 (1961)
– J.W. Haeffele et coll., Proc. Sci. Toilet Goods Assoc., 32, 52 (1959)
– H. Barrer et coll., J. Org. Chem., 27, 641 (1962)
– J.H. Biel et coll., J. Amer. Chem. Soc., 77, 2250 (1955)

lorsqu'il s'agit d'un produit de formule générale (III), (XII), (XVI) ou (XVII) pour lequel R, R'$_4$, R''$_4$ ou R'''$_4$ représente un radical alcoylthio substitué ou hétérocyclylthio, ou selon:

– A.J.W. Headlee et coll., J. Amer. Chem. Soc., 55, 1066 (1933)
– B.K. Campbell et K.N. Campbell, J. Amer. Chem. Soc., 60, 1372 (1938)
– R.C. Elderfeld et coll., J. Amer. Chem. Soc., 68, 1579 (1946)

lorsqu'il s'agit d'un produit de formule générale (XVI) ou (XVII) pour lequel R''$_4$ ou R'''$_4$ représente un radical alcoyloxy substitué ou hétérocyclyloxy, ou selon:

– J. Amer. Chem. Soc., 54, 1499 (1932) et
– J. Amer. Chem. Soc., 54, 3441 (1932),

lorsqu'il s'agit d'un produit de formule générale

(VIII) ou de formule générale (III), (XVI) ou (XVII) pour lequel R, $R''_4$ ou $R'''_4$ sont des radicaux alcoylamino substitués, ou selon:

– E.F. Elslanger et coll., J. Med. Chem., 17, 99 (1974)

– L.M. Werbel et coll., J. Het. Chem., 10, 363 (1973),

lorsqu'il s'agit d'un produit de formule générale (III), (XVI) ou (XVII) pour lequel R, $R''_4$ ou $R'''_4$ sont des radicaux hétérocyclylamino.

Il est entendu que dans les méthodes ci-dessus, lorsque R, $R_2$, $R_3$, $R'_4$, $R''_4$ ou $R'''_4$ contiennent une fonction amine secondaire pouvant interférer avec la réaction, celle-ci doit être préalablement protégée par toute méthode connue qui n'altère pas le reste de la molécule. Le radical protecteur est éliminé après réaction dans les conditions décrites précédemment.

Le cas échéant, les isomères des produits de formule générale (I) et/ou des produits de formule générale (V) peuvent être séparés par chromatographie liquide hautes performances.

Les produits de formule générale (V) peuvent être purifiés comme mentionné précédemment pour les produits de formule générale (I).

Les nouveaux dérivés de la pristinamycine $II_B$ selon l'invention et leurs sels pharmaceutiquement acceptables manifestent des propriétés antibactériennes particulièrement intéressantes in vitro et in vivo.

In vitro, les produits de formule générale (I) se sont montrés actifs sur Staphylococcus aureus Smith à des doses comprises entre 4 et 100 µg/cm$^3$; de plus, ils synergisent l'action antibactérienne de la pristinamycine $I_A$ à des doses comprises entre 0,1 et 10 µg/cm$^3$.

In vivo, les produits selon l'invention se sont montrés actifs sur les infections expérimentales de la souris à Staphylococcus aureus Smith à des doses comprises entre 40 mg/kg et des doses supérieures à 300 mg/kg par voie sous-cutanée. Ils synergisent l'action antimicrobienne à des doses comprises entre 8 et 200 mg/kg par voie sous-cutanée lorsqu'ils sont associés à la pristinamycine $I_A$ dans des proportions variant entre 10-90% et 90-10%.

La toxicité aiguë des produits de formule générale (I), exprimée par leur $DL_{50}$, est généralement comprise entre 300 mg/kg par voie sous-cutanée chez la souris et des doses supérieures à 1 g/kg.

D'un intérêt particulier sont les produits de formule générale (I) dans laquelle le symbole R représente

– soit un radical hétérocyclyle azoté à 5 ou 6 chaînons éventuellement substitué par un radical alcoyle,

– soit une chaîne alcoyle contenant 2 à 4 atomes de carbone substituée par 1 ou 2 radicaux choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces deux derniers radicaux pouvant éventuellement former avec l'atome d'azote auquel elles sont rattachées, un hétérocycle saturé ou insaturé à 5 ou 6 chaînons contenant éventuellement un autre hétéroatome choisi parmi l'azote, l'oxygène ou le soufre à l'état de sulfoxyde ou de sulfone, et éventuellement substitué par un radical alcoyle), ou substituée par un hétérocycle azoté à 5 ou 6 chaînons contenant éventuellement un autre hétéroatome choisi parmi l'azote, l'oxygène ou le soufre à l'état de sulfoxyde ou de sulfone et éventuellement substitué par un radical alcoyle, ledit hétérocycle étant rattaché à la chaîne alcoyle qui le porte par l'intermédiaire d'un atome de carbone, étant entendu que l'un au moins des substituants portés par la chaîne alcoyle ci-dessus est un substituant azoté capable de former des sels, et le symbole n est égal à 1 ou 2; et parmi ces produits, plus particulièrement actifs sont les produits de formule générale (I) dans laquelle le symbole R représente une chaîne alcoyle contenant 2 à 4 atomes de carbone substituée par 1 ou 2 radicaux choisis parmi phényle, cycloalcoylamino ou N-alcoyl N-cycloalcoyl-amino contenant 5 ou 6 chaînons, alcoylamino contenant 1 à 4 atomes de carbone, dialcoylamino (dont les parties alcoyle contiennent 1 à 3 atomes de carbone ou forment avec l'atome d'azote auquel elles sont rattachées un hétérocycle saturé à 5 ou 6 chaînons), ou représente un hétérocycle azoté à ou 6 chaînons éventuellement substitué par un radical alcoyle contenant 1 à 4 atomes de carbone, étant entendu que l'un au moins des substituants portés par la chaîne alcoyle ci-dessus est un substituant azoté capable de former des sels et que l'un au moins des radicaux portés par cette chaîne est placé en position −1 ou en position −2, et le symbole n est égale à 1 ou 2.

Les dérivés de pristinamycine $II_B$ de formule générale (I) cités ci-après ont tout particulièrement retenu notre intérêt:

– la (diéthylamino-2 méthyl-1 éthyl) sulfinyl-26 pristinamycine $II_B$

– la [diméthylamino-2 butyl(2R)] sulfinyl-26 pristinamycine $II_B$

– la (diéthylamino-1 propyl) sulfinyl-26 pristinamycine $II_B$

– la (diisopropylamino-2 éthyl) sulfonyl-26 pristinamycine $II_B$.

Pour l'emploi thérapeutique, il peut être fait usage des nouveaux produits selon l'invention tels quels c'est-à-dire à l'état de base en association avec des synergistines déjà connues, mais comme le principal avantage des produits selon l'invention est leur solubilisation possible dans l'eau, il est particulièrement avantageux de les utiliser sous forme de sels pharmaceutiquement acceptables, en association avec des synergistines connues ou les synergistines de formule générale (V) elles-mêmes solubilisées soit à l'état de sels pharmaceutiquement acceptables soit, le cas échéant à l'état de base lorsque la solubilité est suffisante pour que la solution obtenue contienne une quantité de produit au moins égale à la dose thérapeutiquement active.

Comme sels pharmaceutiquement acceptables, aussi bien pour les produits de formule générale (I) que pour les produits de formule générale (V), on peut citer les sels d'addition avec les acides minéraux tels que chlorhydrates, bromhydrates, sulfates, nitrates, phosphates, ou organiques tels que acétates, propionates, succinates, maléates, fumarates, méthanesulfonates, p-toluènesulfonates, iséthionates ou des dérivés de substitution de ces composés. Comme sels pharmaceutiquement acceptables, on

peut encore citer les sels avec les métaux alcalins (tels que les sels de sodium et de potassium), avec les métaux alcalino-terreux (tels que le sel de magnésium), le sel d'ammonium et les sels d'addition avec les bases organiques azotées (éthanolamine, diéthanolamine, triméthylamine, triéthylamine, méthylamine, propylamine, diisopropylamine, NN-diméthyléthanolamine, benzylamine, dibenzylamine, dicyclohexylamine, N-benzyl-β-phénéthylamine, NN'-dibenzyléthylènediamine, benzhydrylamine, arginine, leucine, lysine ou N-méthylglucamine).

Comme sels pharmaceutiquement acceptables pour les produits de formule générale (V) dans laquelle Z représente un radical de formule générale (VII) dans laquelle $R_4$ représente un radical trialcoylammonio, on peut citer les sels d'ammonium quaternaire correspondant aux anions énumérés ci-dessus.

Les exemples suivants, donnés à titre non limitatif, montrent comment l'invention peut être mise en pratique. Les spectres de RMN des produits illustrés dans ce exemples et dans les exemples de référence qui sont à la suite, présentent des caractéristiques générales qui sont communes à tous les produits de formule générale (I) ou de formule générale (V) et des caractéristiques particulières qui sont propres à chacun des produits en fonction des substituants. Dans les exemples ou exemples de référence suivants ne sont mentionnées que les caractéristiques particulières dues aux radicaux variables. Pour les produits de formule générale (I) tous les protons sont désignés selon la numérotation indiquée dans la formule suivante:

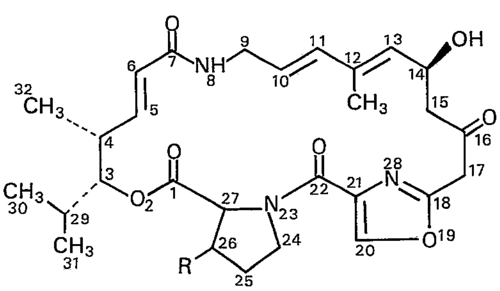
(XXII)

Pour les synergistines de formule générale (V) tous les protons sont désignés selon la numérotation indiquée dans la formule générale (XXIII); cette numérotation est celle recommandée par J.O. ANTEUNIS et coll., [Eur. J. Biochem., 58, 259 (1975)].

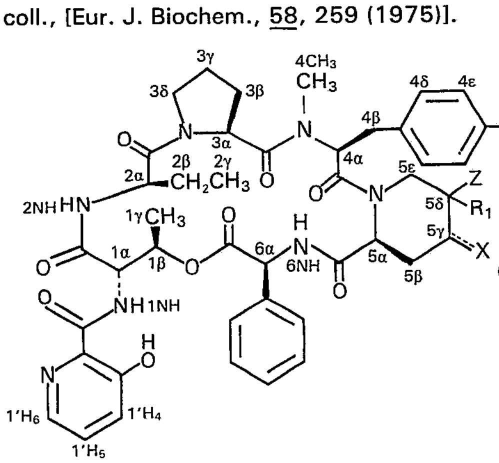
(XXIII)

Sauf mention spéciale, tous les spectres ont été faits à 250 MHz dans le deutérochloroforme; les déplacements chimiques sont exprimés en ppm par rapport au signal du tétraméthylsilane. Les abréviations utilisées dans la suite sont les suivantes:

s = singulett
d = doublet
t = triplet
mt = multiplet
m = massif
dd = doublet de doublet
dt = doublet de triplet
ddd = doublet de doublet de doublet
dddd = doublet de doublet de doublet de doublet

Il est entendu que les différents isomères ont été classés arbitrairement selon les déplacements chimiques observés en RMN.

On appelle isomère $A_1$ et isomère $A_2$ des produits de formule générale (I) dans laquelle n = 1, les isomères qui présentent les caractéristiques:
environ 1,7 (s, $-CH_3$ en 33); environ 3,8 (s, $>CH_2$ en 17); $<5$ (d, $-H_{27}$) isomères $A_2$ ou $>5$ (d, $-H_{27}$) isomère $A_1$; environ 5,50 (d large, $-H_{13}$); environ 6,20 (d, $-H_{11}$); environ 6,6 ($>NH$ en 8); $\geqslant 8$ (s, $-H_{20}$).

On appelle isomère $B_1$ et isomère $B_2$ des produits de formule générale (I) dans laquelle n = 1, les isomères qui présentent les caractéristiques:
environ 1,5 (s, $-CH_3$ en 33); environ 3,7 et 3,9 (2d, $>CH_2$ en 17); environ 4,8 (mt, $-H_{13}$); $<5$ (d, $-H_{27}$) isomère $B_2$ ou $>5$ (d, $-H_{27}$) isomère $B_1$; environ 5,70 (AB limite, $-H_{11}$ et $-H_{10}$); environ 7,7 ($>NH$ en 8); environ 7,8 (s, $-H_{20}$).

On appelle isomère A du produit de formule générale (II) l'isomère qui présente des caractéristiques RMN identiques à celles énoncées ci-dessus pour les isomères $A_1$ et $A_2$ des produits de formule générale (I), étant entendu que l'H en 27 est caractérisé par: 4,7 (d, J $\leqslant$ 1 Hz).

On appelle isomère B du produit de formule générale (II) l'isomère qui présente des caractéristiques RMN identiques à celles énoncées ci-dessus pour les isomères $B_1$ et $B_2$ des produits de formule générale (I), étant entendu que l'H en 27 est caractérisé par: 4,6 (d, J $\geqslant$ 2,5 Hz).

Dans les exemples qui suivent, on appelle chromatographie «flash» une technique de purification caractérisée en ce que l'on utilise une colonne de chromatographie courte et opère sous une pression moyenne (50 kPa) en utilisant une silice de granulométrie 40-53 µm, selon W.C. STILL, M. KAHN et A. MITRA [J. Org. Chem., 43, 2923 (1978)].

Dans les exemples décrits ci-après, sauf mention spéciale, tous les produits peuvent être mis en solution à 2% au moins, à l'état de chlorhydrate.

*Exemple 1*

A 3,59 g de (diisopropylamino-2 éthyl) thio-26 pristinamycine $II_B$ (isomère A) en solution dans 40 $cm^3$ de dichlorométhane, on ajoute à 0°C sous atmosphère d'azote, 0,4 $cm^3$ d'acide trifluoracétique puis 1,06 g d'acide métachloroperbenzoïque à 85% en maintenant la température à 0°C. Après 20 heures d'agitation à 25°C, le mélange réactionnel est additionné à une solution aqueuse saturée de bicarbonate de sodium. La phase organique est décantée puis la phase aqueuse lavée avec 3 fois 100 $cm^3$ de chlorure de méthylène. Les phases organiques sont réunies, séchées sur du sulfate de magnésium, filtrées puis concentrées à sec sous pression réduite (2,7 kPa) à 30°C pour donner 4,2 g d'un solide jaune qui est purifié par chromatographie «flash» [éluant: chloroforme-méthanol 90-10 en volumes)] en recueillant des fractions de 20 $cm^3$. Les fractions 22 à 28 sont réunies et concentrées à sec sous pression réduite (2,7 kPa) à 30°C pour donner un solide obtenu est séparé par filtration pour donner 0,62 g de (diisopropylamino-2 éthyl) sulfinyl-26 pristinamycine $II_B$ (isomère $A_2$) sous forme d'une poudre jaune fondant vers 155°C.

Spectre RMN:

0,90 à 1,15 [mt, $-CH_3$ en 32, 31, 30,
C$\underline{H}_3$
$>N(CH<$ $)_2]$
C$\underline{H}_3$

1,76 (s, $-CH_3$ en 33)

2,75 à 3,15 (mt, $>CH_2$ en 15, $-H_4$ et
C$\underline{H}$-
$-S-C\underline{H}_2-C\underline{H}_2N<$ )
↓ C$\underline{H}$-
O

3,81 (s, $>CH_2$ en 17)

4,76 (d, $-H_{27}$)

5,51 (d, $-H_{13}$)

6,20 (d, $-H_{11}$)

6,48 (m, $>NH$ en 8)

8,13 (s, $-H_{20}$)

Les fractions 35 à 45 sont réunies et concentrées à sec sous pression réduite (2,7 kPa) à 30°C pour donner un solide jaune clair qui est agité dans 15 $cm^3$ d'éther éthylique. Le solide obtenu est separé par filtration pour donner 1,07 g de (diisopropylamino-2 éthyl) sulfinyl-26 pristinamycine $II_B$ (isomère $A_1$ 80%, isomère $A_2$ 20%) sous forme d'une poudre jaune clair fondant vers 145°C.

Spectre RMN (isomère $A_1$):

1,72 (s, $-CH_3$ en 33)

2,70 à 3,15 (mt, $>CH_2$ en 15, $-H_4$,
$-S-CH_2-CH_2-N-CH\langle)$
↓ |
O CH

3,81 (s, $>CH_2$ en 17)

5,26 (d, $-H_{27}$)

5,46 (d, $-H_{13}$)

6,15 (d, $-H_{11}$)

8,11 (s, $-H_{20}$)

La (diisopropylamino-2 éthyl) thio-26 pristinamycine $II_B$ peut être préparée de la manière suivante:

A 52 g de pristinamycine $II_A$ en solution dans un mélange de 260 $cm^3$ de dichlorométhane et 520 $cm^3$ de méthanol on ajoute goutte à goutte sous atmosphère d'azote à −30°C, 16 g de diisopropylamino-2 éthanethiol en solution dans 30 $cm^3$ de dichlorométhane. La solution est agitée pendant 20 heures à −20°C puis concentrée sous pression réduite (2,7 kPa) à 30°C. Le solide obtenu est agité avec 2 fois 1000 $cm^3$ d'éther éthylique, séparé par filtration puis cristallisé dans 100 $cm^3$ d'acétonitrile. Les cristaux sont séparés par filtration puis séchés sous pression réduite (90 Pa) à 40°C. On obtient ainsi 33,6 g de (diisopropylamino-2 éthyl) thio-26 pristinamycine $II_B$ (isomère A) sous forme de cristaux blancs fondant vers 122°C.

Spectre RMN:

1 à 1,15 (mt, $-CH_3$ isopropyl)

1,72 (s, $-CH_3$ en 33)

1,80 à 2,20 (mt, $-H_{25}$, $-H_{29}$)

2,50 à 3 (mt, $-SCH_2CH_2-N<$ CH< / CH< )

3,40 (d, large, $-H_{26}$)

4,74 (s, large, $-H_{27}$)

6,32 (m, $-NH_8$)

8,15 (s, $-H_{20}$)

Le diisopropylamino-2 éthanethiol peut être préparé selon la méthode décrite par D.D. REYNOLDS, D.L. FIELDS et D.L. JOHNSON. J. Org. Chem. $\underline{26}$, 5125 (1961).

*Exemple 2*

A 10 g de (diisopropylamino-2 éthyl) thio-26 pristinamycine $II_B$ (isomère A) en solution dans 300 $cm^3$ de chloroforme on ajoute 1,22 g de bicarbonate de sodium. On refroidit à −50°C et on ajoute goutte à goutte 2,98 g d'acide métachloroperbenzoïque à 98% en solution dans 100 $cm^3$ de chloroforme. Le mélange est agité 2 heures 15 minutes à −50°C puis additionné d'une solution aqueuse saturée de bicarbonate de sodium. Après 15 minutes d'agitation à 25°C, le mélange est décanté puis la phase aqueuse est lavée avec 3 fois 200 $cm^3$ de dichlorométhane. Les phases organiques sont rassemblées, séchées sur sulfate de magnésium, filtrées puis concentrées à sec sous pression réduite (2,7 kPa) à 30°C pour donner 10,62 g d'une meringue blanchâtre. Celle-ci est dissoute dans 400 $cm^3$ d'acétate d'éthyle puis traitée par 140 $cm^3$ d'une solution aqueuse d'acide chlorhydrique 0,1N. Le pH de la solution aqueuse est alors ajusté à 4,2 par addition de 400 $cm^3$ de tampon pH 4,2. La phase aqueuse est décantée puis la phase organique est lavée avec 400 $cm^3$ de tampon pH 4,2. Les phases aqueuses sont rassemblées et lavées avec 2 fois 130 $cm^3$ d'acétate d'éthyle. Après décantation la phase aqueuse est ajustée à pH 7-8 par addition de bicarbonate de sodium puis lavée avec 3 fois 300 $cm^3$ de dichlorométhane. Les phases organiques sont rassemblées puis lavées avec 2 fois 200 $cm^3$ de tampon pH 7,5. La phase aqueuse est lavée avec 50 $cm^3$ de dichlorométhane puis les phases organiques sont rassemblées, séchées sur sulfate de magnésium, filtrées et concentrées à sec sous pression réduite (2,7 kPa) à 30°C pour donner 8,04 g d'un solide jaune clair qui est agité dans 100 $cm^3$ d'éther éthylique, séparé par filtration puis séché sous pression réduite éthylique, separé par filtration puis séché sous pression réduite (90 Pa) à 40°C. On obtient ainsi 7,5 g de (diisopropylamino-2 éthyl) sulfinyl-26 pristinamycine $II_B$ (isomère $A_2$) sous forme d'une poudre jaune fondant vers 158°C, dont les caractéristiques RMN sont identiques à celles de l'exemple 1.

*Exemple 3*

On opère d'une manière analogue à celle décrite à l'exemple 1, mais à partir de 53,2 g de (diéthylamino-2 éthyl) thio-26 pristinamycine $II_B$, de 6,25 $cm^3$

d'acide trifluoroacétique, de 16,4 g d'acide métachloroperbenzoïque. On effectue 3 purifications successives par chromatographie «flash» [éluant: chloroformeméthanol (90-10 en volumes)] en recueillant des fractions de 40 $cm^3$, selon le schéma suivant:

*Schéma de purification*

LOT A (68 g)

CHROMATOGRAPHIE «FLASH»

LOT B 55 g (fractions 21 à 60)

CHROMATOGRAPHIE «FLASH»

LOT $C_1$ (fractions 25 à 35) 10 g

CHROMATOGRAPHIE «FLASH»

LOT D (5,58 g) (fractions 18 à 30)

LOT $C_2$ (fractions 36 à 50) 18,8 g

CHROMATOGRAPHIE «FLASH»

LOT E (11,5 g) (fractions 18 à 45)

Dans tous les cas, les fractions récupérées sont concentrées à sec sous pression réduite (2,7 kPa) à 30°C.

Le lot D est agité dans 60 $cm^3$ d'éther éthylique. Le solide obtenu est séparé par filtration. On obtient 5 g de (diéthylamino-2 éthyl) sulfinyl-26 pristinamycine $II_B$ (isomère $A_2$) sous forme d'une poudre jaune fondant vers 172°C.

Spectre RMN:

1,00 à 1,14 [mt, $-CH_3$ en 32 + $CH_3$ de la chaîne)

1,75 (s, $-CH_3$ en 33)

2,55 à 3,20 (mt, $>CH_2$ en 15, $-H_4$,

$-SCH_2CH_2N<$ $CH_2-$ / $CH_2-$ )
↓
O

3,82 (s, $>CH_2$ en 17)

4,81 (d, $-H_{27}$)

5,51 (d, $-H_{13}$)

6,19 (d, $-H_{11}$)

6,46 (dd, >NH en 8)

8,13 (s, $-H_{20}$)

Le lot E est agité dans 10 $cm^3$ d'éther éthylique. Le solide obtenu est séparé par filtration. On obtient 10,9 g de (diéthylamino-2 éthyl) sulfinyl-26 pristinamycine $II_B$ (isomère $A_2$ 60%, isomère $A_1$ 15%, isomère $B_1$ 12%. isomère $B_2$ 13%).

Spectre RMN:

1,00 à 1,14 (mt, $-CH_3$ en 32 et $-N(CH_2C\underline{H}_3)_2$ de $A_1$ et $A_2$)

1,54 (s, $-CH_3$ en 33 de $B_1$ et $B_2$)

1,68 (s, $-CH_3$ en 33 de $A_1$)

1,75 (s, $-CH_3$ en 33 de $A_2$)

2,65 à 2,95 (mt, $-S(O)CH_2CH_2N<$ et $-H_4$ de $A_1$)

2,55 à 3,20 (mt, $>CH_2$ en 15, $-H_4$ et $-S(O)C\underline{H}_2C\underline{H}_2N<$ de $A_2$)

3,77 (AB limite, $>CH_2$ en 17 de $A_1$)

3,82 (s, $>CH_2$ en 17 de $A_2$)

4,81 (d, $-H_{27}$ de $A_2$)

5,24 et 5,25 (2d, $-H_{27}$ de $A_1$ et de $B_1$)

5,41 (d, $-H_{13}$ de $A_1$)

5,51 (d, $-H_{13}$ de $A_2$)

5,99 et 6 (2d, $-H_6$ de $B_1$ et $-H_6$ de $B_2$)

6,11 (d, $-H_{11}$ de $A_1$)

6,19 (d, $-H_{11}$ de $A_2$)

6,46 (dd, >NH en 8 de $A_2$)

6,79 (dd, >NH en 8 de $A_1$)

7,82 (s, $-H_20$ de $B_1$ et $B_2$)

8,12 (s, $-H_{20}$ de $A_1$)

8,13 (s, $-H_{20}$ de $A_2$)

La (diéthylamino-2 éthyl) thio-26 pristinamycine $II_B$ peut être préparée de la manière suivante:

A une suspension de 13,1 g de pristinamycine $II_A$ dans 150 $cm^3$ de méthanol, on ajoute une solution de 3,7 g de diéthylamino éthanethiol dans 15 $cm^3$ de chlorure de méthylène. La solution obtenue est agitée pendant 18 heures à une température voisine de 20°C, puis versée dans 1500 $cm^3$ d'eau distillée; le mélange obtenu est extraits 3 fois par 1000 $cm^3$ au total de chlorure de méthylène. Les phases organiques sont réunies, séchées sur du sulfate de magnésium, filtrées puis concentrées à sec sous pression réduite (2,7 kPa) à 30°C. Le résidu obtenu est purifié par chromatographie «flash» [éluant: chloroforme-méthanol (90-10 en volumes)]; après concentration à sec des fractions 5 à 23 sous pression réduite (2,7 kPa) à 30°C, on obtient 12,4 g de (diéthylamino-2 éthyl) thio-26 pristinamycine $II_B$ sous forme d'une poudre jaune fondant vers 105°C.

Spectre RMN:

1,05 (m, $-N(CH_2C\underline{H}_3)_2$ + $-H_{32}$)

1,70 (s, $-H_{33}$)

1,85 à 2,15 (m, $-H_{25}$, $-H_{29}$)
2,60 (q, $-N(CH_2CH_3)_2$)
2,75 (s, $-S-CH_2CH_2-$)
2,9 (dd, système ABX, $-H_{15}$)
3,10 (dd, système ABX, $-H_{15}$)
3,40 (ddd, $-H_{26}$)
3,80 (s, $-H_{17}$)
4,75 (d, $-H_{27}$)
5,50 (d, $-H_{13}$)
6,15 (d, $-H_{11}$)
6,60 (s, large, >NH en 8)
8,10 (s, $-H_{20}$)

*Exemple 4*

En opérant d'une manière analogue à celle décrite à l'exemple 1, mais à partir de 5,5 g de (diméthylamino-2 éthyl) thio-26 pristinamycine $II_B$, de 0,67 $cm^3$ d'acide trifluoroacétique, de 1,8 g d'acide métachloroperbenzoïque et après purification par chromatographie «flash» [éluant: chloroforme-méthanol (90-10 en volumes)] en recueillant des fractions de 30 $cm^3$ et concentration à sec des fractions 23 à 40 sous pression réduite (2,7 kPa) à 30°C, on obtient 0,4 g de (diméthylamino-2 éthyl) sulfinyl-26 pristinamycine $II_B$ (isomère $A_2$ 70%, isomère $A_1$ 15%, isomère $B_1$ 7%, isomère $B_2$ 8%)sous forme d'une poudre jaune fondant vers 150°C.

Spectre RMN (isomère $A_2$):
1,77 (s, $-CH_3$ en 33)
2,41 (s, $-N(CH_3)_2$)
2,70 à 3,20 (mt, $-SCH_2CH_2N<$, $>CH_2$ en 15 et $-H_4$)
↓
O
3,82 (s, $>CH_2$ en 17)
4,84 (mt, $-H_3$ et $-H_{27}$)
5,52 (d, $-H_{13}$)
6,19 (d, $-H_{11}$)
6,42 (m, >NH en 8)
8,14 (s, $-H_{20}$)

La (diméthylamino-2 éthyl) thio-26 pristinamycine $II_B$ peut être préparée de la manière suivante:

En opérant d'une manière analogue à celle décrite à l'exemple 3, mais à partir de 2,7 g de pristinamycine $II_A$ et 0,58 g de diméthylamino-2 éthanthiol et après purification par chromatographie «flash» [éluant: chloroforme-méthanol (90-10 en volumes)] et concentration à sec des fractions 11 à 17 sous pression réduite (2,7 kPa) à 30°C, on obtient 1,1 g de (diméthylamino-2 éthyl) thio-26 pristinamycine $II_B$ sous forme d'une poudre jaune fondant vers 100°C.

Spectre RMN:
2,35 (s, 6H : $-N(CH_3)_2$)
2,80 (m, 4H : $-S-CH_2CH_2-N<$)
3,40 (ddd, 1H : $-H_{26}$)
4,75 (d, 1H : $-H_{27}$)
8,10 (s, 1H : $-H_{20}$)

*Exemple 5*

En opérant d'une manière analogue à celle décrite à l'exemple 2, mais à partir de 4,7 g de (N-méthyl N-éthyl-amino-2 éthyl) thio-26 pristinamycine $II_B$(isomères A 90%, B 10%), de 1,22 g de bicarbonate de sodium, de 1,41 g d'acide métachloroperbenzoïque à 98% après purification par chromatographie «flash» [éluant: dichlorométhane-méthanol (90-10 en volumes)] en recueillant des fractions de 20 $cm^3$ et concentration à sec des fractions 44 à 52 sous pression réduite (2,7 kPa) à 30°C, on obtient 2,47 g d'un solide jaune qui est agité dans 50 $cm^3$ d'éther éthylique, séparé par filtration puis séché sous pression réduite (90 kPa) à 40°C. On obtient ainsi 2,3 g de (N-méthyl N-éthyl-amino-2 éthyl) sulfinyl-26 pristinamycine $II_B$ (isomère $A_2$) sous forme d'une poudre jaune fondant vers 145°C.

Spectre RMN:
1,09 (t, $>N-CH_2-CH_3$)
1,76 (s, $-CH_3$ en 33)
2,31 (s, $->N-CH_3$)
2,54 (mt, $>N-CH_2CH_3$)
2,80 (mt, $-H_4$)
2,70 à 3,10 (mt, $-S-CH_2-CH_2N<$)
↓
O
2,92 à 3,12 (2dd, $>CH_2$ en 15)
3,24 (mt, $-H_{26}$)
3,82 (s, $>CH_2$ en 17)
4,82 (s, $-H_{27}$)
5,51 (d, $-H_{13}$)
6,40 (dd, >NH en 8)
8,13 (s, $-H_{20}$)

La (N-méthyl N-éthyl-amino-2 éthyl) thio-26 pristinamycine $II_B$ (isomère A 90%, B 10%) peut être préparée en opérant d'une manière analogue à celle décrite à l'exemple 1, mais à partir de 14,11 g de pristinamycine $II_A$ et 3,2 g de N-méthyl N-éthyl-amino-2 éthanethiol. Après 4 jours d'agitation à −20°C et purification par chromatographie «flash» [éluant: chloroforme-méthanol (90-10 en volumes)] en recueillant des fractions de 80 $cm^3$ puis concentration à sec des fractions 25 à 48 sous pression réduite (2,7 kPa) à 30°C, on obtient 4,75 g d'un solide jaune qui est séchées sous pression réduite (90 kPa) à 40°C. On obtient ainsi 4,7 g de (N-méthyl N-éthyl-amino-2 éthyl) thio-26 pristinamycine $II_B$ (isomères A 90%, B 10%) sous forme d'une poudre jaune fondant vers 140°C.

Spectre RMN:
1,1 (mt, $CH_2CH_3$)
1,73 (s, $-CH_3$ en 33)
2,30 (s, $>N-CH_3$)
2,45 à 2,6 (mt, $>N-CH_2CH_3$)
2,68 à 2,78 (2mt, $-S-CH_2-CH_2N<$)
2,78 (mt, $-H_4$)
2,90 et 3,12 (2dd, $-CH_2-$ en 15)
3,40 (d, $-H_{26}$)
3,83 (s, $-CH_2-$ en 17)
4,76 (s, $-H_{27}$)
5,48 (d, $-H_{13}$)
6,14 (d, $-H_{11}$)
6,34 (mf, >NH en 8)
8,11 (s, $-H_{20}$)

La N-méthyl N-éthyl-amino-2 éthanethiol peut être obtenu par analogie avec la méthode décrite par D.D. REYNOLDS et coll., J. Org. Chem. 26, 5125 (1961), à partir de 25 g de N-méthyl N-éthylamine et de 43,7 g de thiocarbonate d'éthylène. Après distillation, 1,3 g de N-méthyl N-éthyl-amino-2 éthanethiol est obtenu sous forme d'un liquide incolore.

[Eb (6,7 kPa) = 52°C].

*Exemple 6*

En opérant d'une manière analogue à celle décrite à l'exemple 1, mais à partir de 9,8 g de (diméthylamino-3 propyl) thio-26 pristinamycine $II_B$(isomères A/B 50 : 50), de 1,18 $cm^3$ d'acide trifluoroacétique, de 3,1 g d'acide métachloroperbenzoïque et après purification par chromatographie «flash» [éluant: dichlorométhanol (80-20 en volumes)] en recueillant des fractions de 15 $cm^3$ et concentration à sec des fractions 53 à 75 sous pression réduite (2,7 kPa) à 30°C, on obtient 1,6 g de (diméthylamino-3 propyl) sulfinyl-26 pristinamycine $II_B$ (mélange d'isomères) sous forme d'une poudre jaune fondant vers 165°C.

Spectre RMN (mélange d'isomères de type $A_2 \simeq 45\%$, $B_2 \simeq 35\%$ et $B_1 \simeq 15\%$):

1,53 (s, $-CH_3$ en 33 de $B_2$ et $B_1$)
1,75 (s, $-CH_3$ en 33 de $A_2$)
2,26, 2,28 et 2,32 (3s, $>NCH_3$ des 3 isomères)
3,82 (s, $>CH_2$ en 17 de $A_2$)
3,70 et 3,88 (2d, $>CH_2$ en 17 de $B_1$)
3,69 et 3,91 (2d, $>CH_2$ en 17 de $B_2$)
4,76 (d, $-H_{27}$ de $B_2$)
5,25 (d, $-H_{27}$ de $B_1$)
5,50 (d, $-H_{13}$ de $A_2$)
7,63 (mt, $>NH$ en 8 de $B_2$)
7,74 (mt, $>NH$ en 8 de $B_1$)
7,82 (s, $-H_{20}$ de $B_2$ et $B_1$)
8,14 (s, $-H_{20}$ de $A_2$)

La (diméthylamino-3 propyl) thio-26 pristinamycine $II_B$ peut être obtenue de la manière suivante:

En opérant d'une manière analogue à celle décrite à l'exemple 3, mais à partir de 5,25 g de pristinamycine $II_A$ et 1,3 g de diméthylamino-3 propanethiol et après purification par chromatographie «flash» [éluant : chloroforme-méthanol (90-10 en volume)] et concentration à sec des fractions 6 à 29 sous pression réduite (2,7 kPa) à 30°C, on obtient 3,3 g de (diméthylamino-3 propyl) thio-26 pristinamycine $II_B$ sous forme d'une poudre jaune fondant vers 100°C.

Spectre RMN:

1,50 (s, 3H × 0,5 : $-H_{33}$ 1er isomère)
1,70 (s, 3H × 0,5 : $-H_{33}$ 2ème isomère)
1,80 (m, 2H : $-SCH_2-\underline{CH_2}N<$)
2,20 (s, 6H × 0,5 : $-N(CH_3)_2$ 1er isomère)
2,25 (s, 6H × 0,5 : $-N(CH_3)_2$ 2ème isomère)
2,40 (m, 2H : $-S\underline{CH_2}-CH_2-CH_2N<$)
2,70 (m, 2H : $-SCH_2CH_2-\underline{CH_2}N<$)
3,35 / 3,45 } (2m, 1H : $-H_{26}$ de chaque isomère)
4,60 / 4,70 } (2H, 1H : $-H_{27}$ de chaque isomère)
7,80 / 8,10 } (2s, 1H : $-H_{20}$ de chaque isomère)

*Exemple 7*

En opérant d'une manière analogue à celle décrite à l'exemple 1, mais à partir de 6,3 g de (diéthylamino-2 propyl) thio-26 pristinamycine $II_B$, de 0,72 $cm^3$ d'acide trifluoroacétique, de 1,91 g d'acide métachloroperbenzoïque et après purification par chromatographie «flash» [éluant : chloroforme-méthanol (90-10 en volumes)] en recueillant des fractions de 60 $cm^3$ et concentration à sec des fractions 7 à 9 sous pression réduite (2,7 kPa) à 30°C, on obtient 0,99 g de (diéthylamino-2 propyl) sulfinyl-26 pristinamycine $II_B$ (isomères $A_2$) sous forme d'une poudre jaune fondant vers 150°C.

Spectre RMN:

1,03 à 1,20 (mt, $-CH_2-CH(\underline{CH_3})N(CH_2\underline{CH_3})_2$ et $CH_3$ en 32)
1,76 (s, $-CH_3$ en 33)
3,82 (s, $>CH_2$ en 17)
4,79 (m, $-H_{27}$)
5,53 (d, $-H_{12}$)
6,20 (d, $-H_{11}$)
6,42 (m, $>NH$ en 8)
8,13 (s, $-H_{20}$)

Après concentration à sec des fractions 23 à 35 sous pression réduite (2,7 kPa) à 30°C, on obtient 0,64 g de (diéthylamino-2 propyl) sulfinyl-26 pristinamycine $II_B$ (isomères $A_1$) sous forme d'une poudre jaune beige fondant vers 160-170°C.

Spectre RMN:

1,14 (mt, $-N(CH_2\underline{CH_3})_2$)
1,24 (d large, $\underline{CH_3}-CH-N<$
                              |
1,73 (s, $-CH_3$ en 33)
3,81 (AB limite, $>CH_2$ en 17)
5,28 (d, $-H_{27}$)
5,43 (d, $-H_{13}$)
6,15 (d, $-H_{11}$)
6,88 (m, $>NH$ en 8)
8,10 (s, $-H_{20}$)

La (diéthylamino-2 propyl) thio-26 pristinamycine $II_B$ peut être préparée de la manière suivante:

En opérant d'une manière analogue à celle décrite à l'exemple 3, mais à partir de 3,15 g de pristinamycine $II_A$ et 1,8 g de diéthylamino-2 propanethiol et après purification par chromatographie «flash» [éluant: chlorure de méthylène-méthanol (90-10 en volumes)] en recueillant des fractions de 20 $cm^3$ et concentration à sec des fractions 3 à 5 sous pression réduite (2,7 kPa) à 30°C, on obtient 1,4 g de (diéthylamino-2 propyl) thio-26 pristinamycine $II_B$ sous forme d'une poudre jaune fondant vers 160°C.

Spectre RMN:

1 (m, 9H : $-H_{32}$ + $-N(CH_2\underline{CH_3})_2$)
2,50 (m, 6H : $-S-\underline{CH_2}-CH-N(\underline{CH_2}CH_3)_2$)
                                   |
3,30 (m, 1H : $-H_{26}$)
4,70 (d, 1H : $-H_{27}$)
8,12 (s, 1H : $-H_{20}$)

Le diéthylamino-2 propanethiol peut être préparé de la manière suivante:

A une solution de 29,5 g de dichlorhydrate d'S-isothiouréido-3 diéthylamino-2 propane dans 150 $cm^3$ d'eau distillée, on ajoute 25 $cm^3$ d'une solution aqueuse de soude 10N. Le mélange est porté à 100°C pendant 1 heure, refroidi à 20°C, ajusté à pH 9 par addition de 8 $cm^3$ d'une solution aqueuse d'acide chlorhydrique 12N, puis extrait par 3 fois 100 $cm^3$ d'éther éthylique. Les phases éthérés sont rassemblées, séchées sur carbonate de potassium, filtrées puis concentrées à sec sous pression réduite (2,7 kPa) à 30°C. Le mélange est purifié par distillation. On obtient 5,8 g de diéthylamino-2 propanethiol-1 sous forme d'un liquide incolore. [Eb (2,7 kPa) = 78°C].

Le dichlorhydrate d'S-isothiouréido-1 diéthylamino-2 propane peut être préparé de la manière suivante:

A une solution de 41 g de chlorhydrate de chloro-1 diéthylamino-2 propane dans 200 $cm^3$ de diméthylformamide, on ajoute 16,7 g de thiourée. Le mélange est porté à 100°C pendant 30 minutes, puis refroidi à 20°C. Le précipité blanc formé est recueilli par filtration, lavé avec 3 fois 20 $cm^3$ de diméthylformamide puis 3 fois 20 $cm^3$ d'éther éthylique. On obtient 29,6 g de dichlorhydrate d'S-isothiouréido-1 diéthylamino-2 propane sous forme de cristaux blanc fondant à 247-249°C.

Le chlorhydrate de chloro-1 diéthylamino-2 propane peut être obtenu de la manière suivante:

A 100 $cm^3$ de chlorure de thionyle on ajoute en 15 minutes 45,2 g de chlorhydrate de diéthylamino-2 propanol puis on chauffe à 80°C. Après 2 heures d'agitation, le chlorure de thionyle en excès est distillé et le résidu repris par 200 $cm^3$ d'éther éthylique. Le chlorhydrate de chloro-1 diéthylamino-2 propane cristallise. On obtient après filtration 48,2 g de cristaux blancs fondant à 112°C.

Le chlorhydrate de diéthylamino-2 propanol peut être obtenu de la manière suivante:

A une suspension maintenue sous azote de 10,6 g d'hydrure de lithium et d'aluminium dans 1 litre d'éther éthylique on ajoute lentement à 20°C une solution de 66 g de diéthylamino-2 propionate d'éthyle dans 330 $cm^3$ d'éther éthylique. La réaction est maintenue à une température de 35°C pendant 5 heures, puis abaissée à 0°C. On ajoute alors goutte à goutte à 0°C, 12,4 $cm^3$ d'eau, 9,1 $cm^3$ d'une solution aqueuse de soude 5N puis 41,3 $cm^3$ d'eau, on agite 30 minutes puis le mélange est filtré sur verre fritté puis lavé à l'éther éthylique. La phase éthérée est séchée sur carbonate de potassium, filtrée puis concentrée à sec sous pression réduite (2,7 kPa) à 30°C. On obtient 43,8 g d'un liquide jaune qui est dissous dans 200 $cm^3$ d'acétone puis on ajoute 78 $cm^3$ d'une solution 4,5N d'acide chlorhydrique gazeux dans l'éther éthylique. Le chlorhydrate de diéthylamino-2 propanol cristallise. On obtient après filtration, 45,2 g de cristaux blancs fondant à 97-100°C.

Le diéthylamino-2 propionate d'éthyle peut être obtenu selon BRAUN et coll., Beilstein, 61, 1425 (1928).

*Exemple 8*

On opère d'une manière analogue à celle décrite à l'exemple 2 mais à partir de 4 g de (diéthylamino-2 propyl) thio-26 pristinamycine $II_B$ (isomères A), de 1,16 g d'acide métachloroperbenzoïque à 98% et de 1 g de bicarbonate de sodium solide. Après purification par chromatographie «flash» [éluant: chloroformeméthanol (93-7) en volumes] et concentration à sec des fractions 21 à 48 sous pression réduite (2,7 kPa) à 30°C, en recueillant des fractions de 25 $cm^3$, on obtient 2,69 g de (diéthylamino-2 propyl) sulfinyl-26 pristinamycine $II_B$ (isomères $A_2$) sous forme d'une poudre jaune ayant des caractéristiques identiques à celles du produit obtenu à l'exemple 7.

La (diéthylamino-2 propyl) thio-26 pristinamycine $II_B$ (isomère A) peut être obtenue en opérant d'une manière analogue à celle décrite à l'exemple 1 mais à partir de 15 g de pristinamycine $II_A$ et de 4,62 g de diéthylamino-2 propanethiol. Après purification par chromatographie «flash» [éluant: chloroforme-méthanol (90-10 en volumes)] et concentration à sec des fractions 27 à 52 sous pression réduite (2,7 kPa) à 30°C, en recueillant des fractions de 40 cm3, on obtient 12 g d'un solide jaune qui est agité dans 60 $cm^3$ d'éther éthylique, filtré puis séché. On obtient 8,2 g de (diéthylamino-2 propyl) thio-26 pristinamycine $II_B$ (isomère A) sous forme d'une poudre jaune clair fondant vers 122°C.

Spectre RMN:

1 à 1,15 (mt, $-CH_3$ éthyl + $C\underline{H}_3$-CH-$N(C_2H_5)_2$)
                                                   |

1,70 (s, $-CH_3$ en 33)

                                   $C\underline{H}_2-CH_3$
2,35 à 2,60 (mt, -N<                )
                                   $C\underline{H}_2-CH_3$

2,50 à 3,10 (mt, $-SCH_2CH-$)
                                         |

2,75 (mt, $-H_4$)

2,89 et 3,05 (2dd }
2,92 et 3,08 (2dd } $>CH_2$ en 15)

3,30 (mt }
3,37 (mt } $-H_{26}$)

3,80 (s, $>CH_2$ en 17)

4,69 (d }
4,71 (d } $-H_{27}$

5,45 (d, $-H_{13}$)

6,13 (d }
6,14 (d } $-H_{11}$)

6,4 à 6,60 (mt, >NH en 8)

6,51 (dd }
6,53 (dd } $-H_5$)

8,09 (s, $-H_{20}$)

Le diéthylamino-2 propanethiol peut être obtenu comme décrit précédemment à l'exemple 7.

*Exemple 9*

On opère d'une manière analogue à celle décrite à l'exemple 2 mais à partir de 4,58 g de (diéthylamino-1 propyl-2) thio-26 pristinamycine $II_B$ (isomères A), de 1,29 g d'acide métachloroperbenzoïque à 98% et de 1,14 g de bicarbonate de sodium solide. Après purification par chromatographie «flash» [éluant: chloroformeméthanol (97-3) en volumes] en recueillant des fractions de 20 $cm^3$ et concentration à sec sous pression réduite (2,7 kPa) à 30°C respectivement des fractions 59 à 77 et des fractions 79 à 97, on obtient: à partir des fractions 79 à 97, 1,47 g de (diéthylamino-1 propyl-2) sulfinyl-26 pristinamycine $II_B$ (premier isomère) sous forme d'un solide jaune clair fondant vers 132°C.

Spectre RMN:

1,02 (t, $-CH_3$ éthyl)

1,34 (d, $C\underline{H}_3$-CH-$CH_2N(C_2H_5)_2$)
                          |

1,72 (s, $-CH_3$ en 33)

                                          $CH_2$-
2,5 à 2,7 (mt, $-CH_2$-N<          )
                                          $CH_2$-

2,77 (mt, $-H_4$)

2,87 et 3,09 (2dd, $>CH_2$ en 15)

2,97 (mt, -S-CH<)
                  ↓
                  O

3,72 (mt, $-H_{26}$ )

3,80 (s, $>CH$ en 17)
4,92 (mt, $-H_{27}$)
5,43 (d, $-H_{13}$)
6,15 (d, $-H_{11}$)
6,72 (dd, $>NH$ en 8)
8,06 (s, $-H_{20}$)

et à partir des fractions 59 à 77, 1,07 g de (diéthylamino-1 propyl-2) sulfinyl-26 pristinamycine $II_B$ (deuxième isomère) sous forme d'un solide jaune clair fondant vers 128°C.

Spectre RMN:
1,72 (s, $CH_3$ en 33)
3,4 (mt, $-H_{26}$)
3,79 (s, $CH_2$ en 17)
4,74 (mt, $-H_{27}$)
5,48 (d, $-H_{13}$)
6,18 (d, $-H_{11}$)
6,80 (mt, $>NH$ en 8)
8,09 (s, $-H_{20}$)

La (diéthylamino-1 propyl-2) thio-26 pristinamycine $II_B$ (isomères A) peut être obtenue en opérant d'une manière analogue à celle décrite à l'exemple 1 mais à partir de 13 g de pristinamycine $II_A$ et de 4 g de diéthylamino-1 propane-2 thiol. Après purification par chormatographie «flash» [éluant: chloroforme-méthanol (90-10 en volumes)] et concentration à sec des fractions 46 à 55 sous pression réduite (2,7 kPa) à 30°C en recueillant des fractions de 50 $cm^3$, on obtient 8 g d'un solide jaune pâle qui est cristallisé dans 30 $cm^3$ d'acétonitrile. Après filtration et séchage, on obtient 5,91 g de (diéthylamino-1 propyl-2) thio-26 pristinamycine $II_B$ (isomères A) sous forme de cristaux blancs fondant à 136°C.

Spectre RMN:
0,9 à 1,10 (mt, $-N(CH_2C\underline{H}_3)_2$)
1,33 à 1,37 (2d, $C\underline{H}_3-CH-CH_2N<$)
|
1,7 (s, $-CH_3$ en 33)
2,4 à 2,65 (mt, $-CH_2N<\begin{matrix}CH_2-\\CH_2-\end{matrix}$)
2,76 (mt, $-H_4$)
3 (mt, $-S-CH<$)
2,9 et 3,1 (2dd, $>CH_2$ en 15)
3,52 (mt, $-H_{26}$)
3,81 (s, $>CH_2$ en 17)
4,78 (mt, $-H_{27}$)
5,46 (d, $-H_{13}$)
6,14 (d, $-H_{11}$)
6,40 (mf, $>NH$ en 8)
8,09 et 8,10 (2s, $-H_{20}$)

Le diéthylamino-1-propane-2 thiol peut être obtenu suivant la méthode décrite par R.T. WRAGG, J. Chem. Soc. (C), 2087 (1969).

*Exemple 10*

On opère d'une manière analogue à celle décrite à l'exemple 2 mais à partir de 1,7 g de [diméthylamino-2 butyl 2(R)] thio-26 pristinamycine $II_B$ (isomère A), de 0,50 g de bicarbonate de sodium, de 0,45 d'acide métachloroperbenzoïque à 98%. Après purification par chromatographie «flash» [éluant: acétate d'éthyle-méthanol (85-15 en volumes)] et concentration à sec des fractions 35 à 58 sous pression réduite (2,7 kPa) à 30°C, on obtient 1,1 g d'un solide blanc qui est agité dans 30 $cm^3$ d'éther éthylique. Après filtration et séchage, on obtient 0,95 g de [diméthylamino-2 butyl 6(R)] sulfinyl-26 pristinamycine $II_B$ (isomère $A_2$) sous forme d'un solide blanc fondant vers 126°C.

Spectre RMN:
1 (mt, $>N-CH-CH_2CH_3$)
|
1,45 à 1,75 (mt, $>N-CH-C\underline{H}_2CH_3$)
|
1,78 (s, $-CH_3$ en 33)
2,50 à 3,05 (mt, $-S-CH_2-CH<$ et $-H_4$)
↓
O
2,93 et 3,14, (2dd, $>CH_2$ en 15)
3,31 (mt, $-H_{26}$)
3,84 (s, $>CH_2$ en 17)
4,84 (d, $-H_{27}$)
5,51 (d, $-H_{13}$)
6,19 (d, $-H_{11}$)
6,30 (dd, $>NH$ en 8)
8,15 (s, $-H_{20}$)

La [diméthylamino-2 butyl 2(R)] thio-26 pristinamycine $II_B$ (isomère A) peut être obtenue en opérant d'une manière analogue à celle décrite à l'exemple 1 mais à partir de 8 g de pristinamycine $II_A$ et 2,3 g de diméthylamino-2 butanethiol 2(R). Après purification par chromatographie «flash» [éluant: dichlorométhane-méthanol (90-10 en volumes)] et concentration à sec des fractions 36 à 55 sous pression réduite (2,7 kPa) à 30°C, on obtient 3 g de [diméthylamino-2 butyl 2(R)] thio-26 pristinamycine $II_B$ (isomère A) sous forme d'un solide jaune clair fondant vers 120°C.

La cristallisation de 0,9 g de ce produit dans 5 $cm^3$ d'acétonitrile conduit après séparation par filtration à 0,2 g de [diméthylamino-2 butyl 2(R)] thio-26 pristinamycine $II_B$ (isomère A) sous forme de cristaux blancs fondant à 122°C.

Spectre RMN:
1 (mt, $>N-CH-CH_2C\underline{H}_3$)
|
1,4 à 1,7 (mt, $>N-CH-C\underline{H}_2CH_3$)
|
1,72 (s, $-CH_3$ en 33)
2,30 (s, $-N(CH_3)_2$)
2,5 à 2,85 (mt, $-S-CH_2-CH<$ et $-H_4$
2,93 et 3,10 (2dd, $>CH_2$ en 15)
3,34 (d large, $-H_{26}$)
3,83 (s, $>CH_2$ en 17)
4,76 (s large, $-H_{27}$)
5,48 (d, $-H_{13}$)
6,14 (d, $-H_{11}$)
6,26 (dd, $>NH$ en 8)
8,13 (s, $-H_{20}$)

Le diméthylamino-2 butanethiol (R) peut être obtenu d'une manière analogue à celle décrite ci-après à l'exemple 11, à partir de 52,4 g de triphénylphosphine, de 40 $cm^3$ de diisopropylazodicarboxylate, de 12 g de diméthylamino-2 butenol (R) et de 15,2 $cm^3$ d'acide thiolacétique (dans ce cas le thioester intermédiaire est hydrolysé directement lors de la chromatographie sur gel de silice).

Après purification par chromatographie «flash» [éluant: dichlorométhane: 1000 $cm^3$, puis dichloro-

méthane-méthanol (85-15 en volumes): 2000 $cm^3$, puis dichlorométhane-méthanol (80-20 en volumes): 4000 $cm^3$] en recueillant des fractions de 100 $cm^3$ et concentration à sec sous pression réduite des fractions 42 à 60, on obtient 14 g d'une huile jaune qui est purifiée par distillation. On obtient ainsi 2,4 g de diméthylamino-2 butanethiol (R) sous forme d'un liquide incolore. [Eb (a kPa) = 70-75°C].

Le diméthylamino-2 butanol-1 (R) peut être obtenu d'une manière identique à celle décrite par M. WENGHOEFER et coll., J. Heterocycl. Chem., 7(6), 1407 (1970).

*Exemple 11*

En opérant d'une manière analogue à celle décrite à l'exemple 2 mais à partir de 2,67 g de [diméthylamino-2 phényl-3 propyl (2S)] thio-26 pristinamycine $II_B$ (isomère A), de 0,7 g de bicarbonate de sodium et de 0,7 g d'acide métachloroperbenzoïque à 98%, après purification par chromatographie «flash» [éluant: chloroforme-méthanol (90-10 en volumes)] en recueillant des fractions de 20 $cm^3$ et concentration à sec des fractions 19 à 23 sous pression réduite (2,7 kPa) à 30°C, on obtient 1,3 g d'un solide jaune clair qui est agité dans 50 $cm^3$ d'éther éthylique, séparé par filtration pour donner 1,18 g de [diméthylamino-2 phényl-3 propyl (2S)] sulfinyl-26 pristinamycine $II_B$ (isomère $A_2$) sous forme d'un solide jaune clair fondant vers 150°C.

Spectre RMN (400 MHz, $CDCl_3$)
1,73 (s, $-CH_3$ en 33)

2,4 à 2,6 (mt, H
-S-CH₂-C< )
2,8 à 3,15 (mt, ↓ | CH₂-
O

2,44 (s, $-N(CH_3)_2$)
2,77 (mt, $-H_4$)
2,89 et 3,1 (2dd, $>CH_2$ en 15)
3,18 (mt, $-H_{26}$)
3,82 (s, $>CH_2$ en 17)
4,68 (d, $-H_{27}$)
5,51 (d, $-H_{13}$)
6,19 (d, $-H_{11}$)
6,50 (dd, >NH en 8)
7,18 (d, -H en ortho du phényle)
7,23 (t, -H en para du phényle)
7,31 (t, -H en méta du phényle)
8,13 (s, $-H_{20}$)

On obtient une solution aqueuse à 1% de [diméthylamino-2 phényl-3 propyl(2S)] sulfinyl-26 pristinamycine $II_B$ (isomère $A_2$) avec:

| | | |
|---|---|---|
| — produit | | 30 mg |
| — acide chlorhydrique 0,1N | | 0,45 $cm^3$ |
| — eau distillée | qsp | 3 $cm^3$ |

La [diméthylamino-2 phényl-3 propyl (2S)] thio-26 pristinamycine $II_B$ (isomère A) peut être préparée en opérant d'une manière analogue à celle décrite à l'exemple 1 pour la préparation de la matière de départ mais à partir de 7,13 g de prtistinamycine $II_A$ et 2,65 g de diméthylamino-2 phényl-3 propanethiol (S) et après purification par chromatographie «flash» [éluant: acétate d'éthyle-méthanol (80-20 en volumes)] en recueillant des fractions de 60 $cm^3$ et concentration à sec des fractions 33 à 43 sous pression réduite (2,7 kPa) à 30°C, on obtient 4,6 g d'un solide jaune clair qui est agité dans 50 $cm^3$ d'éther éthylique, filtré puis séché sous pression réduite (90 Pa). On obtient ainsi 3,6 g de [diméthylamino-2 phényl-3 propane (2S)] thio-26 pristinamycine $II_B$ (isomère A) sous forme d'une poudre jaune pâle fondant vers 110°C.

Spectre RMN:
1,69 (s, $-CH_3$ en 33)
2,38 (s, $-N(CH_3)_2$)

H
2,35 à 3,05 (mt, -SCH₂-C< )
| CH₂-
N
/ \

2,73 (mt, $-H_4$)
2,89 et 3,10 (2dd, $>CH_2$ en 15)
3,26 (d large, $-H_{26}$)
3,81 (s, $>CH_2$ en 17)
4,68 (s large, $-H_{27}$)
5,47 (d, $-H_{13}$)
6,12 (d, $-H_{11}$)
6,27 (mf, >NH en 8)
7,18 (d, -H en ortho du phényle)
7,21 (t, -H en para du phényle)
7,30 (t, -H en méta du phényle)
8,11 (s, $-H_{20}$)

Le diméthylamino-2 phényle-3 propanethiol (S) peut être préparé de la manière suivante:

A 20 g de diméthylamino-2 phényl-3 propanethiolacétate (S) (brut) en solution dans 50 $cm^3$ de méthanol on ajoute sous atmosphère d'azote, 0,2 g de méthylate de sodium et on chauffe à reflux pendant 2 heures. Le mélange est ensuite concentré à sec sous pression réduite (2,7 kPa) à 30°C pour donner un liquide qui est purifié par distillation. On obtient 2,4 g de diméthylamino-2 phényl-3 propanethiol (S) sous forme d'un liquide incolore [Eb (14 Pa) = 95°C] qui est utilisé tel quel dans la réaction suivante.

Le diméthylamino-2 phényl-3 propanethiolacétate (S) peut être préparé de la manière suivante:

On ajoute à 0°C, sous atmosphère d'azote, 41,97 g de triphénylphosphine et 310 $cm^3$ de tétrahydrofuranne puis on additionne goutte à goutte 31,5 $cm^3$ de diisopropylazodicarboxylate et laisse agiter une demi heure à 0°C. A la suspension blanche obtenue on ajoute goutte à goutte un mélange de 15 g de diméthylamino-2 phényl-3 propanol (S) et de 11,44 $cm^3$ d'acide thiolacétique en solution dans 160 $cm^3$ de tétrahydrofuranne. Après 1 heure d'agitation à 0°C puis 1 heure 30 minutes à 25°C le mélange est concentré à sec sous pression réduite (2,7 kPa) à 30°C. L'huile obtenue est additionnée de 190 $cm^3$ de méthanol, le solide blanc qui précipite est éliminé par filtration et le filtrat est concentré à sec sous pression réduite (2,7 kPa) à 30°C. Le résidu est alors agité avec 200 $cm^3$ d'oxyde d'isopropyle, le solide blanc précipité est à nouveau éliminé par filtration et le filtrat est concentré pour donner 45 g d'une huile jaune qui est purifiée par chromatographie «flash» [éluant: dichlorométhane-méthanol (90-10 en volumes)] en recueillant des fractions de 110 $cm^3$. Après concentration à sec des fractions 37 à 55 sous

pression réduite (2,7 kPa) à 30°C, on obtient 10,4 g de diméthylamino-2 phényl-3 propanethiolacétate (S) sous forme d'une huile jaune orangée (contenant de l'oxyde de triphénylphosphine).

Le diméthylamino-3 phényl-3 propanol (S) peut être préparé par analogie avec la méthode décrite par T. HAYASHI et coll., J. Org. Chem., 48, 2195 (1983).

*Exemple 12*

En opérant d'une maniére analogue à celle décrite à l'exemple 1, mais à partir de 12,5 g de [(pyrrolidinyl-1)-2 éthyl] thio-26 pristinamycine $II_B$ (isomère A 90%), de 1,47 $cm^3$ d'acide trifluoroacétique, de 3,86 g d'acide métachloroperbenzoïque et après purification par chromatographie «flash» [éluant: chloroformeméthanol (85-15 en volumes)] en recueillant des fractions de 30 $cm^3$ et concentration à sec des fractions 18 à 25 sous pression réduite (2,7 kPa) à 30°C, on obtient 3,9 g de [(pyrrolidinyl-1)-2 éthyl] sulfinyl-26 pristinamycine $II_B$ (isomères $A_1$ 60%, $A_2$ 25%, $B_1$ 15%) sous forme d'une poudre jaune fondant vers 175°C.

Spectre de RMN (isomère $A_1$):

1,74 (s, $-CH_3$ en 33)

$CH_2$-
2,62 (mt, -N< )
$CH_2$-

2,70 à 3,20 (mt, $>CH_2$ en 15,
$-S-CH_2-CH_2N<$, $-H_4$)
↓
O

3,81 (s, $>CH_2$ en 17)
5,28 (s large, $-H_{27}$)
5,45 (d, $-H_{13}$)
6,14 (d, $-H_{11}$)
6,58 (mt, >NH en 8)
8,12 (s, $-H_{20}$)

Après concentration à sec des fractions 26 à 43 sous pression réduite (2,7 kPa) à 30°C, on obtient 4,36 g de [(pyrrolidinyl-1)-2 éthyl] sulfinyl-26 pristinamycine $II_B$ (isomère $A_2$ 75%, isomère $A_1$ 5%, isomère $B_1$ 10%, isomère $B_2$ 10%) sous forme d'une poudre jaune fondant vers 145°C.

Spectre RMN (isomère $A_2$):

1,76 (s, $-CH_3$ en 33)
1,82 (m, $>CH_2$ en -3 et -4 du pyrrolidinyle)
2,63 (mt, $-N-CH_2-$)
|
$CH_2$-
2,85 à 3,20 (mt $-S-CH_2-CH_2<$ et $>CH_2$ en 15)
3,82 (s, $>CH_2$ en 17)
4,84 (dd, $-H_3$ + d, $-H_{27}$)
5,51 (d, $-H_{13}$)
6,18 (d, $-H_{11}$)
6,47 (mt, > NH en 8)
8,13 (s, $-H_{20}$)

La [(pyrrolidinyl-1)-2 éthyl] thio-26 pristinamycine $II_B$ peut être préparée de la manière suivante:

En opérant d'une manière analogue à celle décrite à l'exemple 3, mais à partir de 5,25 g de pristinamycine $II_A$ et 1,7 g de (pyrrolidinyl-1)-2 éthanethiol et après purification par chromatographie «flash» [éluant: chloroforme-méthanol (95-5 en volumes)] de (pyrrolidinyl-1)-2 éthanethiol et après purification par chromatographie «flash» [éluant: chloroforme-méthanol (95-5 en volumes)] et concentration à sec des fractions 19 à 60 sous pression réduite (2,7 kPa) à 30°C, on obtient 3,9 g de [(pyrrolidinyl-1)-2 éthyl] thio-26 pristinamycine $II_B$ sous forme d'une poudre jaune fondant vers 115°C.

Spectre RMN:

1,90 (mt, 4H : 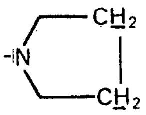 )

2,50 à 2,80 (m, 6H : $-S-CH_2CH_2N$ 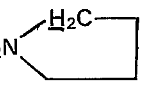 )

3,49 (d, 1H : $-H_{26}$)

4,75 (d, 1H : $-H_{27}$)
8,10 (s, 1H, $-H_{20}$)

Le pyrrolidinyl-1)-2 éthanethiol peut être préparé selon la méthode décrite par J.W. HAEFFELE et R.W. BROGE, Proc. Sci. Toilet Goods Assoc. 32, 52 (1959) [Chem. Abstr. 54, 17234e (1960)].

*Exemple 13*

En opérant d'une manière analogue à celle décrite à l'exemple 1 mais à partir de 6 g de (pipéridino-2 éthyl) thio-26 pristinamycine $II_B$ (isomère A), de 0,69 $cm^3$ d'acide trifluoroacétique et de 1,82 g d'acide métachloroperbenzoïque à 85%, après purification par chromatographie «flash» [éluant: chloroforme-méthanol (85-15 en volumes)] en recueillant des fractions de 20 $cm^3$ et concentration à sec des fractions 52 à 105 sous pression réduite (2,7 kPa) à 30°C, on obtient 4,7 g d'un solide jaune qui est à nouveau purifié par chromatographie «flash» [éluant: chloroforme-méthanol (85-15 en volumes)] en recueillant des fractions de 5 $cm^3$. Après concentration des fractions 92 à 99 sous pression réduite (2,7 kPa) à 30°C, on obtient 1,83 g d'un solide jaune qui est agité dans 20 $cm^3$ d'éther éthylique, séparé par filtration puis séché sous pression réduite (90 Pa) à 30°C. On obtient ainsi 1,51 g de (pipéridino-2 éthyl) thio-26 pristinamycine $II_B$ (isomères $A_2$ 90%, $A_1$ 10%) sous forme d'une poudre jaune fondant vers 162°C.

Spectre RMN (400 MHz, $CDCl_3$)

1,52 (mf, 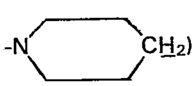 )

1,70 (mf, 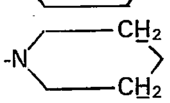 )

1,78 (s, $-CH_3$ en 33)

2,64 (mf, 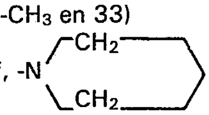 )

2,80 (mt, $-H_4$)
2,85 à 3,25 (mt, $-S-CH_2-CH_2-N<$)
↓
O
2,94 et 3,15 (2dd, $>CH_2$ en 15)
3,20 (mt, $-H_{26}$)
3,83 (s, $>CH_2$ en 17)
4,92 (d, $-H_{27}$)

5,54 (d, $-H_{13}$)
6,24 (d, $-H_{11}$)
6,70 (mf, >NH en 8)
8,14 (s, $-H_{20}$)

Après concentration à sec des fractions 100 à 140 sous pression réduite (2,7 kPa) à 30°C, on obtient 2,11 g d'un solide jaune qui est agité dans 20 $cm^3$ d'éther éthylique, séparé par filtration puis séché sous pression réduite (90 Pa) à 30°C. On obtient 1,75 g de (pipéridino-2 éthyl) thio-26 pristinamycine $II_B$ (isomères $A_1$ 50%, $A_2$ 50%) sous forme d'une poudre jaune fondant vers 152°C.

Spectre RMN (400 MHz, $CDCl_3$)
1,74 (s, $-CH_3$ en 33 isomère $A_1$)
1,78 (s, $-CH_3$ en 33 isomère $A_2$)
3,2 (mt, $-H_{26}$ isomère $A_2$)
3,46 (mt, $-H_{26}$ isomère $A_1$)
3,82 (AB limite, $>CH_2$ en 17 isomère $A_1$)
3,83 (s, $>CH_2$ en 17 isomère $A_2$)
4,90 (d, $-H_{27}$ isomère $A_2$)
5,30 (s, $-H_{27}$ isomère $A_1$)
5,52 (d, $-H_{13}$ isomère $A_1$)
5,54 (d, $-H_{13}$ isomère $A_2$)
6,60 (dd, $-H_5$ isomère $A_2$)
6,70 (dd, $-H_5$ isomère $A_1$)
8,14 (s, $-H_{20}$ des isomères $A_2$ et $A_1$)

La (pipéridino-2 éthyl) thio-26 pristinamycine $II_B$ (isomère A) peut être obtenue de la manière suivante:

En opérant d'une manière analogue à celle décrite à l'exemple 1 mais à partir de 11,8 g de pristinamycine $II_A$ et 3,58 g de piperidino-2 éthanethiol et après purification par chromatographie «flash» [éluant: chloroforme-méthanol (85-15 en volumes)] en recueillant des fractions de 60 $cm^3$ et concentration à sec des fractions 24 à 31 sous pression réduite (2,7 kPa) à 30°C, on obtient 8,3 g de (pipéridino-2 éthyl) thio-26 pristinamycine $II_B$ (isomère A) sous forme d'une poudre jaune clair fondant vers 120°C.

Spectre RMN:
1,08 (d, $-CH_3$ en 32)
1,40 à 1,60 (mt, -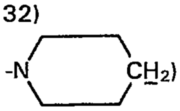)
1,60 à 1,80 (mt, -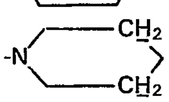 )
1,73 (s, $-CH_3$ en 33)
2,45 à 2,90 (mt, $-S-CH_2-CH_2-N<$ $CH_2-$ $CH_2-$ )
3,43 (mt, $-H_{26}$)
3,82 (s, $>CH_2$ en 17)
4,71 (s large, $-H_{27}$)
5,50 (d, $-H_{13}$)
8,13 (s, $-H_{20}$)

Le pipéridino-2 éthanethiol peut être obtenu d'une manière identique à celle décrite par D.D. REYNOLDS, D.L. FIELDS et D.J. JOHNSON, J. Org. Chem., 26, 5125 (1961).

*Exemple 14*

En opérant d'une manière analogue à celle décrite à l'exemple 2 mais à partir de 3,2 g d'[(imidazolyl-1)-2 éthyl] thio-26 pristinamycine $II_B$ (isomères A 85%, B 15%), de 1 g de bicarbonate de sodium et de 0,93 g d'acide métachloroperbenzoïque à 98%, après purification par chromatographie «flash» [éluant: chloroforme-méthanol (90-10 en volumes)] en recueillant des fractions de 25 $cm^3$ et concentration à sec des fractions 29 à 49 sous pression réduite (2,7 kPa) à 30°C, on obtient 1,4 g de solide jaune. Le solide obtenu est à nouveau purifié par chromatographie «flash» [éluant: chloroforme-méthanol 690-10 en volumes)] en recueillant des fractions de 10 $cm^3$. Après concentration à sec des fractions 47 à 55 sous pression réduite (2,7 kPa) à 30°C, on obtient 0,62 g d'un solide jaune clair qui est agité dans 20 $cm^3$ d'éther éthylique, séparé par filtration puis séché sous pression réduite (90 Pa) à 40°C. On obtient ainsi 0,6 g d'](imidazolyl-1)-2 éthyl sulfinyl-26 pristinamycine $II_B$ (isomère $A_2$) sous forme d'un solide jaune fondant vers 170°C.

Spectre RMN (400 MHz, $CDCl_3$)
1,80 (s, $-CH_3$ en 33)
2,72 (mt, $-H_4$)
2,97 à 3,09 (2dd, $>CH_2$ en 15)
3,0 (mt, $-H_{26}$ et un H de $-S-CH_2-$)
↓
O
3,48 (mt, l'autre H de $-S-CH_2-$)
↓
O
3,82 /AB, limite, $>CH_2$ en 17)
4,53 (dd, $>N-CH_2-$)
4,77 (d, $-H_{27}$)
5,52 (d, $-H_{13}$)
6,16 (d, $-H_{11}$)
6,46 (dd, >NH en 8)
7,12 (s, -N-CH=CH-N=)
|
7,69 (s, >N-CH=N-)
8,16 (s, $-H_{20}$)

L'[(imidazolyl-1)-2 éthyl] thio-26 pristinamycine $II_B$ peut être préparée en opérant d'une manière analoque à celle décrite à l'exemple 3 mais à partir de 14,35 g de pristinamycine $II_A$ et 3,5 g d'(imidazolyl-1)-2 éthanethiol, après 18 heures d'agitation à 20°C puis purification par chromatographie «flash» [éluant: acétate d'éthyle-méthanol (80-20 en volumes)] et concentration à sec des fractions 34 à 59 sous pression réduite (2,7 kPa) à 30°C, on obtient un solide jaune, qui est agité dans 60 $cm^3$ d'éther éthylique, puis séparé par filtration pour donner 10,9 g d'[(imidazolyl-1)-2 éthyl] thio-26 pristinamycine $II_B$ (isomère A 85%, B 15%) sous forme d'un solide jaune fondant vers 160°C.

Spectre RMN:
1,53 (s, $-CH_3$ en 33 de B)
1,73 (s, $-CH_3$ en 33 de A)
2,74 (mt, $-H_4$ de A)
2,86 et 3,14 (2dd, $>Ch_2$ en 15 de A)
2,85 à 3,05 (mt, $-SCH_2-$)
3,11 (mt, $-H_{26}$ de A)
3,32 (mt, $-H_{26}$ de B)
3,82 (AB limite, $>CH_2$ en 17 de A)
4,15 à 4,30 (mt, $-CH_2N<$)
4,58 (d, $-H_{27}$ de B)
4,68 (d fin, $-H_{27}$ de A)
5,44 (d, $-H_{13}$ de A)

6,16 (d, $-H_{11}$ de A)
6,83 (dd, >NH en 8 de A)
6,97 et 7,08 (2s, >N-CH = CHN< de B)
7,01 et 7,10 (2s, >N-CH = CHN< de A)
7,54 (s, >N-CH = N- de B)
7,61 (s, >N-CH = N- de A)
7,64 (mt, >NH en 8 de B)
7,82 (s, $-H_{20}$ de B)
8,09 (s, $-H_{20}$ de A)

L'(imidazolyl-1)-2 éthanethiol peut être préparé d'une manière analogue à celle décrite à l'exemple 11 pour la préparation de la matière de départ mais à partir de 21 g d'(imidazolyl-1)-2 éthanethiolacétate et de 0,5 g de méthylate de sodium. Après purification par distillation on obtient 2,3 g d'(imidazolyl-1)-2 éthanethiol sous forme d'une huile [Eb (20 Pa) = 99,5°C].

L'(imidazolyl-1)-2 éthanethiolacétate peut être préparé d'une manière analogue à celle décrite à l'exemple 11 pour la préparation de la matière première mais à partir de 15 g d'imidazolyl-1(-2 éthanol, de 70,2 g de triphénylphosphine, de 55,8 $cm^3$ de diisopropylazodicarboxylate et de 21 $cm^3$ d'acide thiolacétique. Après purification par chromatographie «flash» [éluant: chlorure de méthylène 1500 $cm^3$, puis acétate d'éthyle-méthanol (80-20 en volumes)] en recueillant des fractions de 100 $cm^3$ et concentration à sec sous pression réduite (2,7 kPa) à 30°C des fractions 21 à 35, on obtient 21,14 g d'(imidazolyl-1)-2 éthylthiolacétate sous forme d'une huile jaune orangée utilisée sans purification supplémentaire.

L'(imidazolyl-1)-2 éthanol peut être préparé d'une façon analogue à celle décrite par J. GEIBEL et coll., J. Am. Chem. Soc., <u>100</u>, 3575 (1978).

*Exemple 15*

En opérant d'une manière analogue à celle décrite à l'exemple 2 mais à partir de 5,5 g de (morpholino-2 éthyl) thio-26 pristinamycine $II_B$ (isomère A), de 1,3 g de bicarbonate de sodium, de 1,4 g d'acide métachloroperbenzoïque à 98%, on obtient après extraction du mélange réactionnel, séchage de la phase organique sur sulfate de magnésium, filtration et concentration à sec sous pression réduite (2,7 kPa) à 30°C, un solide jaune clair qui est agité dans 100 $cm^3$ d'oxyde d'isopropyle, séparé par filtration, puis séché sous pression réduite (90 Pa) à 35°C. On obtient ainsi 4,8 g de (morpholino-2 éthyl) sulfinyl-26 pristinamycine $II_B$ (isomère $A_2$) sous forme d'un solide jaune clair fondant vers 126°C.

Spectre RMN:
1,77 (s, $-CH_3$ en 33)
$CH_2$-
2,6 à 3,1 (mt, $-SCH_2-CH_2N$< et $-H_4$)
↓ $CH_2$-
O
2,95 et 3,13 (2dd, >$CH_2$ en 15)
3,20 (mt, $-H_{26}$)
3,78 (mt, $-CH_2-O-CH_2-$)
3,81 (s, >$CH_2$ en 17)
4,85 (mt, $-H_{27}$)
5,53 (d, $-H_{13}$)
6,20 (d, $-H_{11}$)
6,53 (mf, >NH en 8)
8,14 (s, $-H_{20}$)

La (morpholino-2 éthyl) thio-26 pristinamycine $II_B$ (isomère A) peut être obtenue d'une manière analogue à celle décrite à l'exemple 1 mais à partir de 15 g de pristinamycine $II_A$ et de 6,3 g de morpholino-2 éthanethiol. Après purification par chromatographie «flash» [éluant: acétate d'éthyle-méthanol (75,25 en volumes)] en recueillant des fractions de 30 $cm^3$ et concentration à sec des fractions 35 à 49 sous pression réduite (2,7 kPa) à 30°C, on obtient 11 g d'un solide beige qui est cristallisé dans 120 $cm^3$ d'acétonitrile. On obtient ainsi 5,7 g de (morpholino-2 éthyle) thio-26 pristinamycine $II_B$ (isomère A) sous forme de cristaux blancs fondant à 132°C.

Spectre RMN:
1,73 (s, $-CH_3$ en 33)
$CH_2$-
2,50 (mf, -N< )
$CH_2$-
2,6 à 2,9 (mt, $-H_4$)
2,64 (mt, >$N-CH_2$-)
2,79 (mt, $-SCH_2$-)
2,91 et 3,11 (2dd, <$Ch_2$ en 15)
3,37 (d large, $-H_{26}$)
$CH_2$-
3,74 (mf, O< )
$CH_2$-
3,83 (s, >$CH_2$ en 17)
4,74 (s large, $-H_{27}$)
5,45 (d, $-H_{13}$)
6,13 (d, $-H_{11}$)
6,28 (mf, <NH en 8)
8,13 (s, $-H_{20}$)

Le morpholino-2 éthanthiol peut être préparé d'une manière analogue à celle décrite par D.D. REYNOLDS et coll., J. Org. Chem., <u>26</u>, 5125 (1961).

*Exemple 16*

En opérant d'une manière analogue à celle décrite à l'exemple 1, mais à partir de 5,8 g de (butylamino-2 éthyl) thio-26 prstinamycine $II_B$ (isomère A 80%, isomère B 20% ), de 0,68 $cm^3$ d'acide trifluoroacétique, de 1,8 g d'acide métachloroperbenzoïque et après purification par chromatographie «flash» [éluant: chloroforme-méthanol (90-10 en volumes)] en recueillant des fractions de 15 $cn^3$ et concentration à sec des fractions 9 à 15 sous pression réduite (2,7 kPa) à 30°C, on obtient 1,7 g de (butylamino-2 éthyl) sulfinyl-26 pristinamycine $II_B$ (isomère $A_2$ 70%, isomère $B_1$ 15%, isomère $B_2$ 15%) sous forme d'une poudre jaune fondant vers 140°C.

Spectre RMN (isomère $A_2$):
0,85 à 1,00 (mt, $-CH_3$ en 31 et 30 + $-CH_3$ de la chaîne)
1,34 (mt, $-C\underline{H_2}CH_3$)
1,48 (mt, $-CH_2C\underline{H_2}CH_2CH_3$)
1,75 (s, $-CH_3$ en 33)
2,50 à 3,30 (mt, $-H_{26}$, <$CH_2$ en 2,
$-S-CH_2-CH_2-N-CH_2-$, $-H_4$)
↓ |
O
3,80 (s, >$CH_2$ en 17)
4,80 (d, $-H_{27}$)

5,50 (d, $-H_{13}$)
6,17 (d, $-H_{11}$)
6,40 (dd, >NH en 8)
8,12 (s, $-H_{20}$)

Après concentration à sec des fractions 18 à 24 sous pression réduite (2,7 kPa) à 30°C, on obtient 0,5 g de (butylamino-2 éthyl) sulfinyl-26 pristinamycine $II_B$ (isomère $A_1$ 85%, isomère $B_1$ 15%) sous forme d'une poudre jaune vers 170°C.

Spectre RMN (isomère $A_1$):
0,85 à 1,00 (mt, $-CH_3$ en 31, 30 et $-CH_3$ de la chaîne)
1,33 (mt, $-C\underline{H}_2CH_3$)
1,478 (mt, $-CH_2C\underline{H}_2C\underline{H}_2CH_3$)
1,71 (s, $-Ch_3$ en 33)
2,50 à 3,25 (mt, $-S-CH_2CH_2N<$ et $-H_4$)
↓
O
3,79 (AB limite, $<CH_2$ en 17)
5,26 (d, $-H_{27}$)
5,44 (d, $-H_{13}$)
6,13 (d, $-H_{11}$)
6,62 (mt, >NH en 8)
8,10 (s, $-H_{20}$)

La (butylamino-2 éthyl) thio-26 pristinamycine $II_B$ (isomère A 80%, isomère B 20%) peut être préparée comme décrit ci-après à l'exemple 17.

*Exemple 17*

En opérant d'une manière analogue à celle décrite à l'exemple 1, mais à partir de 3,15 g de (butylamino-2 éthyl) thio-26 pristinamycine $II_B$ (isomère B), de 0,37 $cm^3$ d'acide trifluoroacétique, de 0,97 g d'acide métachloroperbenzoïque et après purification par chromatographie «flash» [éluant: chloroforme-méthanol (90-10 en volumes)] en recueillant des fractions de 15 $cm^3$ et concentration à sec des fractions 18 à 35 sous pression réduite (2,7 kPa) à 30°C, on obtient 1,18 g de (butylamino-2 éthyl) sulfinyl-26 pristinamycine $II_B$ (isomère $B_1$ 65%, isomère $B_2$ 35%) sous forme d'une poudre jaune fondant vers 140°C.

Spectre RMN:
0,90 à 1,05 (mt, $-CH_3$ en 30 et 31 $-CH_3$ de la chaîne de $B_1$ et $B_2$)
1,40 (mt, $-C\underline{H}_2CH_3$ de $B_1$ et $B_2$)
1,50 (mt, $-CH_2C\underline{H}_2CH_2CH_3$ de $B_1$ et $B_2$)
1,57 (s, $-CH_3$ en 33 de $B_1$ et $B_2$)
2,63 (t, $>NCH_2CH_2CH_2CH_3$ de $B_1$ et $B_2$)
2,65 à 3,30 (mt, $-S-CH_2CH_2N<$,
↓
O
$>CH_2$ en 15, $-H_4$ de $B_1$ et $B_2$)
3,74 et 3,92 (2d, $>Ch_2$ en 17 de $B_1$)
3,73 et 3,94 (2d, $>CH_2$ en 17 de $B_2$)
4,78 (d, $-H_{27}$ de $B_2$)
4,75 à 4,90 (mt, $-H_{13}$ et $-H_{14}$ de $B_1$ et $B_2$)
5,27 (d, $-H_{27}$ de $B_1$)
5,70 (2d, $-H_{11}$ de $B_1$ et $B_2$)
7,69 (dd, >NH en 8 de $B_2$)
7,79 (dd, >NH en 8 de $B_1$)
7,84 (s, $-H_{20}$ de $B_2$)
7,85 (s, $-H_{20}$ de $B_1$)

En opérant d'une manière analogue à celle décrite à l'exemple 3, mais à partir de 25 g de pristinamycine $II_A$ et 6,34 g de butylamino-2 éthanethiol et après purification par chromatographie «flash» [éluant: chloroforme-méthanol (90-10 en volumes)] en recueillant des fractions de 60 $cm^3$ et concentration à sec des fractions 12 à 15 sous pression réduite (2,7 kPa) à 30°C, on obtient 3,15 g de (butylamino-2 éthyl) thio-26 pristinamycine $II_B$ (isomère B) sous forme d'une poudre jaune fondant vers 110°C. Après concentration à sec des fractions 15 à 25 sous pression réduite (2,7 kPa) à 30°C, on obtient 5,89 g de (butylamino-2 éthyl) thio-26 pristinamycine $II_B$ (isomère A 80%, isomère B 20%).

*Exemple 18*

En opérant d'une manière analogue à celle décrite à l'exemple 1, mais à partir de 8,6 g de (décylamino-2 éthyl) thio-26 pristinamycine $II_B$, de 0,9 $cm^3$ d'acide trifluoroacétique, de 2,35 g d'acide métachloroperbenzoïque et après purification par chromatographie «flash» [éluant: chloroforme-méthanol (90-10 en volumes)] en recueillant des fractions de 40 $cm^3$ et concentration à sec des fractions 12 à 15 sous pression réduite (2,7 kPa) à 30°C, on obtient 1,5 g de (décylamino-2 éthyl) sulfinyl-26 pristinamycine $II_B$ (isomère $A_2$ 80%) sous forme d'une poudre jaune fondant vers 128°C.

Spectre RMN:
0,88 (t, $-(CH_2)_9-C\underline{H}_3$)
1,30 [m, $(>CH_2)_8$]
1,50 [m, $(>CH_2)_8$]
1,77 (s, $-CH_3$ en 33)
4,81 (d, $-H_{27}$)
5,51 (d, $-H_{13}$)
6,19 (d, $-H_{11}$)
6,53 (mt, >NH en 8)
8,13 (s, $-H_{20}$)

Après concentration à sec des fractions 15 à 19 sous pression réduite (2,7 kPa) à 30°C, on obtient 2,51 g de (décylamino-2 éthyl) sulfinyl-26 pristinamycine $II_B$ (mélange d'isomères) sous forme d'une poudre jaune fondant vers 124°C.

Spectre RMN (mélange d'isomères de type $A_2$ 50%, $A_1$ 15%, $B_1$ 20% et $B_2$ 15%)
1,54 (s, $-CH_3$ en 33 de $B_1$ et $B_2$)
3,72 et 3,88 (2d, $>CH_2$ en 17 de $B_1$)
3,70 et 3,92 (2d, $>CH_2$ en 17 de $B_2$)
4,75 (d, $-H_{27}$ de $B_2$)
5,25 (d, $-H_{27}$ de $B_1$)
7,67 (dd, >NH en 8 de $B_2$)
7,77 (dd, >NH en 8 de $B_1$)
7,81 (s, $-H_{20}$ de $B_1$ et $B_2$)
(pics caractéristiques des isomères $A_2$ et $A_1$ identiques à ceux cités respectivement ci-avant et ci-après).

On obtient une solution aqueuse à 1% de (décylamino-2 éthyl) sulfinyl-26 pristinamycine $II_B$ à l'état de chlorhydrate avec:

| | | |
|---|---|---|
| (décylamino-2 éthyl) sulfinyl-26 pristinamycine $II_B$ | | 15 mg |
| acide chlorhydrique 0,1N | | 0,20 $cm^3$ |
| eau distillée | qsp | 1,5 $cm^3$ |

Après concentration à sec des fractions 20 à 24

sous pression réduite (2,7 kPa) à 30°C, on obtient 1,12 g de (décylamino-2 éthyl) sulfinyl-26 pristinamycine $II_B$ (isomères $A_1$ 60%, $A_2$ 20%, $B_1$ 20%) sous forme de poudre jaune fondant vers 136°C.

Spectre RMN (isomère $A_1$):

2,50 à 3,20 (mt, $>CH_2$ en 15,
-$H_4$ et -S-$CH_2$-$CH_2$-N-$CH_2$-)
↓
O

3,82 (AB limite, $>CH_2$ en 17)
5,27 (d, -$H_{27}$)
5,46 (d, -$H_{13}$)
6,15 (d, -$H_{11}$)
6,62 (mt, >NH en 8)
8,12 (s, -$H_{20}$)

La (décylamino-2 éthyl) thio-26 pristinamycine $II_B$ peut être préparée de la manière suivante:

En opérant d'une manière analogue à celle décrite à l'exemple 3, mais à partir de 5,25 g de pristinamycine $II_A$ et de 3,26 g de décylamino-2 éthanethiol et après purification par chromatographie «flash» [éluant: chlorue de méthylène-méthanol (95-5 en volumes)] et concentration à sec des fractions 20 à 43 sous pression réduite (2,7 kPa) à 30°C, on obtient 1,2 g de (décylamino-2 éthyl) thio-26 pristinamycine $II_B$ sous forme de poudre jaune fondant vers 80°C.

Spectre RMN (mélange d'isomères 70-30 de A et B):

0,88 (t, -$CH_3$)
1,30 } (mt, -$(CH_2)_8$-)
1,53 }
1,54 (s, -$CH_3$ en 33 de B)
1,72 (s, -$CH_3$ en 33 de A)
2,6 à 3 (mt, -S$CH_2$-$CH_2$-N-$CH_2$-)
|
3,38 (d large, -$H_{26}$ de A)
3,50 (mt, -$H_{26}$ de B)
4,64 (d, J = 3,5, -$H_{27}$ de B)
4,72 (s large, -$H_{27}$ de A)
7,80 (s, -$H_{20}$ de B)
8,12 (s, -$H_{20}$ de A)

*Exemple 19*

En opérant d'une manière analogue à celle décrite à l'exemple 1, mais à partir de 4,4 g de (cyclohexylamino-2 éthyl) sulfinyl-26 pristinamycine $II_B$ (isomères A 80%, B 20%), de 0,5 $cm^3$ d'acide trifluoroacétique, de 1,15 g d'acide métachloroperbenzoïque et après purification par chromatographie «flash» [éluant: chloroforme-méthanol (90-10 en volumes)] en recueillant des fractions de 40 $cm^3$ et concentration à sec des fractions 24 à 29 sous pression réduite (2,7 kPa) à 30°C, on obtient 0,38 g de (cyclohexylamino-2 éthyl) sulfinyl-26 pristinamycine $II_B$ (isomère $A_2$ 90%) sous forme d'une poudre jaune clair fondant vers 166°C.

Spectre RMN:

1,05 à 1,35 [mt, $>CH_2$ du cyclohexyle (en partie)]
1,77 (s, -$CH_3$ en 33)
1,55 à 2,25 [mt, $>CH_2$ en 25,
-$H_{29}$ et $>CH_2$ du cyclohexyle (en partie)]
2,45 à 3,35 (mt, -$H_{26}$ , $>CH_2$ en 15,
-$H_4$ et -S-$CH_2$-$CH_2$N-CH<)
↓ |
O

3,82 (s, $>CH_2$ en 17)
4,82 (d, -$H_{27}$)
5,52 (d, -$H_{13}$)
6,19 (d, -$H_{11}$)
6,38 (dd, >NH en 8)
8,14 (s, -$H_{20}$)

La cyclohexylamino-2 éthyl) thio-26 pristinamycine $II_B$ peut être obtenue de la manière suivante:

En opérant d'une manière analogue à celle décrite à l'exemple 3, mais à partir de 5,25 g de pristinamycine $II_A$ et de 3,6 g de cyclohexylamino-2 éthanethiol et après purification par chromatographie «flash» [éluant: chloroforme-méthanol (93-7 en volumes)] et concentration à sec des fractions 7 à 18 sous pression réduite (2,7 kPa) à 30°C, on obtient 1,7 g de (cyclohexylamino-2 éthyl) thio-26 pristinamycine $II_B$ sous forme d'une poudre beige fondant vers 120°C.

Spectre RMN:

1 à 1,4 [mt, $>CH_2$ du cyclohexyle (en partie)]
1,54 (s, -$CH_3$ en 33 isomère B)
1,73 (s, -$CH_3$ en 33 isomère A)
1,6 à 2 [mt, $>CH_2$ du cyclohexyle (en partie)]
2,80 (mt, $>NCH_2$-)
2,93 (t, -$SCH_2$-)
3,36 (d large, -$H_{26}$ isomère A)
3,50 (mt, -$H_{26}$ isomère B)
4,64 (d, J = 3, -$H_{27}$ isomère B)
4,72 (s large, -$H_{27}$ isomère A)
6,50 (mt, -$HH_8$ isomère A)
7,75 (mt, -$NH_8$ isomère B)
7,80 (s, -$H_{20}$ isomère B)
8,12 (s, -$H_{20}$ isomère A)

Le cyclohexylamino-2 éthanethiol peut être préparé selon la méthode décrite par D.D. REYNOLDS, M.K. MASSAD, D.L. FIELDS et D.L. JOHNSON, J. Org. Chem., 26, 5109 (1961).

*Exemple 20*

En opérant d'une manière analogue à celle décrite à l'exemple 2 mais à partir de 5 g de (N-cyclohexyl N-méthyl-amino-2 éthyl) thio-26 pristinamycine $II_B$ (isomères A 80%, B 20%), de 1,17 g de bicarbonate de sodium, de 1,2 g d'acide métachloroperbenzoïque à 98% après purification par chromatographie «flash» [éluant: dichlorométhane-méthanol (80-20 en volumes)] en recueillant des fractions de 30 $cm^3$ et concentration à sec des fractions 40 à 60 sous pression réduite (2,7 kPa) à 30°C, on obtient 3,5 g d'un solide jaune qui est à nouveau purifié par chromatographie «flash» [éluant: acétate d'éthyle-méthanol (80-20 en volumes)] en recueillant des fractions de 25 $cm^3$. Après concentration à sec des fractions 11 à 18 sous pression réduite (2,7 kPa) à 30°C, on obtient 1,2 g d'un solide jaune qui est agité dans 30 $cm^3$ d'éther éthylique, séparé par filtration puis séché sous pression réduite (90 Pa) à 35°C. On obtient ainsi 1,1 g de (N-cyclohexyl N-méthyl-amino-2 éthyl) sulfinyl-26 pristinamycine $II_B$ (isomère $A_2$) sous forme d'une poudre jaune fondant vers 126°C.

Spectre RMN:

1,10 à 2 (mt, $>CH_2$ du cyclohexyle)
1,76 (s, -$CH_3$ en 33)
2,34 (s, $>N-CH_3$)
2,45 (mt, >N-CH<)

2,7 à 3,15 (mt, $-S-CH_2-CH_2N\langle$ et $-H_4$)
↓
O

2,93 et 3,14 (2dd, $\rangle CH_2$ et 15)
3,25 (ddd, $-H_{26}$)
3,82 (s, $\rangle CH_2$ en 17)
4,82 (d, $-H_{27}$)
5,52 (d, $-H_{13}$)
6,18 (d, $-H_{11}$)
6,43 (dd, $\rangle NH$ en 8)
8,13 (s, $-H_{20}$)

La (N-cyclohexyl N-méthyl-amino-2 éthyl) thio-26 pristinamycine $II_B$ (isomère A 80%, B 20%) peut être obtenue d'une manière analogue à celle décrite à l'exemple 3 pour la préparation de la matière première mais à partir de 10,5 g de pristinamycine $II_A$ et de 4 g de N-cyclohexyl N-méthyl-amino-2 éthanethiol. Après purification par chromatographie «flash» [éluant: acétate d'éthyle-méthanol (80-20 en volumes)] en recueillant des fractions de 30 $cm^3$ et concentration à sec des fractions 42 à 96 sous pression réduite (2,7 kPa) à 30°C, on obtient un solide jaune qui est agité dans 80 $cm^3$ d'oxyde d'isopropyle, séparé par filtration puis séché sous pression réduite (90 Pa) à 35°C. On obtient ainsi 7,9 g de (N-cyclohexyl N-méthyl-amino-2 éthyl) thio-26 pristinamycine $II_B$ (isomères A 80% et B 20%) sous forme d'une poudre jaune fondant vers 116°C.

Spectre RMN (mélange 80/20 de deux isomères A et B):

1,25 et 1,6 à 1,9 (mt, $\rangle CH_2$ du cyclohexyle pour A et B)
1,56 (s, $-CH_3$ en 33 de B)
1,73 (s, $-CH_3$ en 33 de A)
2,25 à 2,5 (m, $\rangle CH-$ du cyclohexyle pour A et B)
2,32 (s, $\rangle N-CH_3$ de B)
2,35 (s, $\rangle N-CH_3$ de A)
2,6 à 2,8 (mt, $-H_4$ de A et B)
2,78 (AB limite, $-SCH_2CH_2N\langle$ de A et B)
2,9 et 3,14 (2dd, $\rangle CH_2$ en 15 de A)
3,41 (d large, $-H_{26}$ de A)
3,73 et 3,91 (2d, $\rangle CH_2$ en 17 de B)
3,83 (s, $\rangle CH_2$ en 17 de A)
4,65 (d, $-H_{27}$ de B)
4,76 (s large, $H_{27}$ de A)
5,49 (d, $-H_{13}$ de A)
6,16 (d, $-H_{11}$ de A)
6,36 (mf, $\rangle NH$ en 8 de A)
7,73 (mf, $\rangle NH$ en 8 de B)
7,82 (s, $-H_{20}$ de B)
8,13 (s, $-H_{20}$ de A)

Le N-cyclohexyl N-méthyl-amino-2 éthanethiol peut être obtenu de la manière suivante:

A 20 g de dichlorhydrate de S-(N-cyclohexyl N-méthyl-amino-2 éthyle) isothiouronium, on ajoute sous atmosphère d'azote, 23 $cm^3$ d'une solution aqueuse de soude 6N. Après 2 heures d'agitation à 100°C, le mélange est refoidi à 25°C puis additionné d'une solution concentrée d'acide chlorhydrique jusqu'à pH 9. La solution est lavée par 3 fois 50 $cm^3$ de dichlorométhane puis les phases organiques sont réunies, séchées sur sulfate de magnésium, filtrées puis concentrées à sec sous pression réduite (2,7 kPa) à 30°C pour donner une huile qui est purifiée par distillation sous pression réduite (130 Pa). On obtient 4,3 g de N-cyclohexyl N-méthyl-amino-2 éthanethiol sous forme d'un liquide incolore [Eb (130 Pa) = 68°C].

Le dichlorhydrate de N-cyclohexyl N-méthyl-amino-2 éthane thiouronium peut être obtenue de la manière suivante:

A 30 g de chlorhydrate de (N-cyclohexyl N-méthyl-amino)-2 chloro-1 éthane dans 300 $cm^3$ d'éthanol, on ajoute 10,7 g de thiourée. La solution obtenue est chauffé à 78°C pendant 18 heures. Après refroidissement, le solide blanc obtenu est filtré puis rincé à l'éthanol. On obtient ainsi 21,5 g de dichlorhydrate de N-cyclohexyl N-méthyl-amino-2 éthane tiouronium sous forme d'un solide blanc fondant à 248°C.

Le chlorhydrate de (N-cyclohexyl N-méthyl-amino)-2 chloro-1 éthane peut être obtenu de la manière suivante:

A 120 $cm^3$ de chlorure de thionyle sont ajoutés goutte à goutte 25 g de N-cyclohexyl N-méthyl-amino-2 éthanol puis le mélange est chauffé à 70°C pendant 24 heures. Après distillation du chlorure de thionyle en excès, l'huile orangée obtenue est agitée dans 200 $cm^3$ d'éther éthylique pour donner un solide blanc qui est séparé par filtration puis lavé à l'éther. On obtient 30 g de chlorhydrate de (N-cyclohexyl N-méthyl-amino)-2 chloro-1 éthane sous forme d'un solide blanc fondant à 154°C.

*Exemple 21*

En opérant d'une manière analogue à celle décrite à l'exemple 1, mais à partir de 4,3 g de [(méthyl-4 pipérazinyl-1) carbonyloxy-2 éthyl] thio-26 pristinamycine $II_B$ (isomère A), de 0,45 $cm^3$ d'acide trifluoroacétique, de 1,2 g d'acide métachloroperbenzoïque et après purification par chromatographie «flash» [éluant: chloroforme-méthanol (90-10 en volumes)] en recueillant des fractions de 30 $cm^3$ et concentration à sec des fractions 42 à 56 sous pression réduite (2,7 kPa) à 30°C, on obtient 1,2 g de [(méthyl-4 pipérazinyl-1) carbonyloxy-2 éthyl] sulfinyl-26 pristinamycine $II_B$ (isomère $A_2$) sous forme d'une poudre jaune clair fondant vers 135°C.

Spectre RMN:

1,78 (s, $-CH_3$ en 33)
2,32 (s, $\rangle N-CH_3$)
2,42 (m, 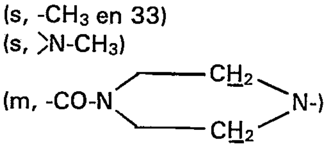
2,95 à 3,28 (2mt, $-S-CH_2-$)
↓
O
3,54 (m, 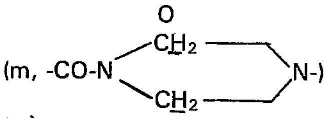
3,82 (s. $\rangle CH_2$ en 17)
4,48 (mt, $-CH_2-O-C-N\langle$)
‖
O
4,82 (d, $-H_{27}$)
5,50 (d, $-H_{13}$)
6,20 (d, $-H_{11}$)
6,39 (dd, $\rangle NH$ en 8)
8,14 (s, $-H_{20}$)

Après concentration à sec des fractions 65 à 95 sous pression réduite (2,7 kPa) à 30°C, on obtient

0,65 g de [(méthyl-4 piperazinyl-1) carbonyloxy-2 éthyl] sulfinyl-26 pristinamycine $II_B$ (isomère $A_1$) sous forme d'une poudre jaune clair fondant vers 140°C.

Spectre RMN:

1,75 (s, $-CH_3$ en 33)

2,34 (s, $>N-CH_3$)

2,44 (m, -CO- 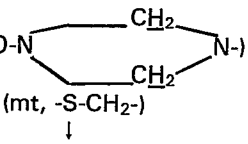

2,90 à 3,15 (mt, $-S-CH_2-$)
↓
O

3,55 (m, -CO- 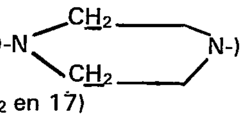

3,83 (s, $>CH_2$ en 17)

4,51 à 4,65 (2ddd, $-CH_2-O-C-N<$)
‖
O

5,28 (d, $-H_{27}$)

6,19 (d, $-H_{11}$)

6,55 (dd, >NH en 8)

8,14 (s, $-H_{20}$)

La [(méthyl-4 piperazinyl-1) carbonyloxy-2 éthyl] thio-26 pristinamycine $II_B$ peut être préparé de la manière suivante:

En opérant d'une manière analogue à celle décrit à l'exemple 3, mais à partir de 5,25 g de pristinamycine $II_A$ et de 3,76 g (méthyl-4 piperazinyl-1) carbonyloxy-2 éthanethiol et après purification par chromatographie «flash» [éluant: chloroforme-méthanol (90-10 en volumes)] et concentration à sec des fractions 10 à 18 sous pression réduite (2,7 kPa) à 30°C, on obtient 2,55 g de [(méthyl-4- piperazinyl-1) carbonyloxy-2 éthyl] thio-26 pristinamycine $II_B$ sous forme d'une poudre beige fondant vers 100°C.

Spectre RMN:

1,54 (s, $-CH_3$ en 33 de l'isomère B)

1,73 (s, $-CH_3$ en 33 de l'isomère A)

2,3 (s, $>N-CH_3$)

2,4 (m, $\begin{matrix}-CH_2\\ \\-CH_2\end{matrix}>N-$)

3,55 (m, $-OOC-N<\begin{matrix}CH_2-\\ \\CH_2-\end{matrix}$)

3,98 (mt, $-CH_2-OCO-$)

4,59 (d, J, = 4, $-H_{27}$ de l'isomère B)

4,69 (s large, $-H_{27}$ de l'isomère A)

7,05 (t, >NH en 8 de l'isomère A)

7,7 (m, >NH en 8 de l'isomère B)

7,80 (s, $-H_{20}$ de l'isomère B)

8,10 (s, $-H_{20}$ de l'isomère A)

Le (méthyl-4 piperazinyl-1) carbonyloxy-2 éthanethiol peut être préparé selon la méthode décrite par D.D. REYNOLDS, D.L. FIELDS et D.L. JOHNSON, J. Org. Chem. 26, 5111 (1961).

*Exemple 22*

En opérant d'une manière analogue à celle décrite à l'exemple 1, mais à partir de 7,8 g [méthyl-1 pyrrolidinyl-2(S)] méthylthio-26 pristinamycine $II_B$ (isomère A), de 0,91 $cm^3$ d'acide trifluoroacétique, de 2,4 g d'acide métachloroperbenzoïque et après purification par chromatographie «flash» [éluant: chloroforme-méthanol (90-10 en volumes)] en recueillant des fractions de 60 $cm^3$ et concentration à sec des fractions 26 à 36 sous pression réduite (2,7 kPa) à 30°C, on obtient 2,3 g de [méthyl-1 pyrrolidinyl-2(S)] méthylsulfinyl-26 pristinamycine $II_B$ (isomère $A_2$) sous forme d'une poudre jaune clair fondant vers 140°C.

Spectre RMN:

1,76 (s, $-CH_3$ en 33)

2,48 (s, $>NCH_3$)

1,70 à 2,60 (mt, $-H_{29}$ et $>CH_2$ en 25 et 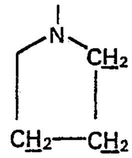

2,75 à 3,25 (mt $-S-CH_2-CH<$)
↓
O

3,82 (s, $>CH_2$ en 17)

4,81 (d, $-H_{27}$)

5,52 (d, $-H_{13}$)

6,20 (d, $-H_{11}$)

6,42 (dd, >NH en 8)

8,14 (s, $-H_{20}$)

Après concentration à sec des fractions 46 à 59 sous pression réduite (2,7 kPa) à 30°C, on obtient 1,1 g de [méthyl-1 pyrrolidinyl-2(S)] méthylsulfinyl-26 pristinamycine $II_B$ (isomère $A_1$) sous forme d'une poudre jaune clair fondant vers 148°C.

Spectre RMN:

1,73 (s, $-CH_3$ en 33)

1,70 à 2,50 (mt, 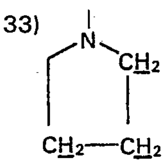 , $-H_{29}$)

2,41 (s, $>N-CH_3$)

2,65 à 3,25 (mt, $>CH_2$ en 15, -H en 4, $-S-CH_2-CH<$
↓
O

3,82 (AB limite, $>CH_2$ en 17)

5,45 (d, $-H_{13}$)

6,17 (d, $-H_{11}$)

8,11 (s, $-H_{20}$)

La (méthyl-1 pyrrolidinyl-2) méthylthio-26 pristinamycine $II_B$ peut être préparée de la manière suivante:

En opérant d'une manière analogue à celle décrite à l'exemple 3, mais à partir de 10,5 g de pristinamycine $II_A$ et 3,14 g de [méthyl-1 pyrrolidinyl-2(S)] méthanethiol et après purification par chromatographie «flash» [éluant: chloroforme-méthanol (90-10 en volumes)] et concentration à sec des fractions 20 à 35 sous pression réduite (2,7 kPa) à 30°C, on obtient 7,8 g de l'isomère A sous forme d'une poudre jaune fondant à environ 120°C.

Spectre RMN:

1,70 (s, $-CH_3$ en 33)

2,38 (s, $>N-CH_3$)

1,70 à 2,50 (mt, $-H_{29}$, $>CH_2$ en 25 et 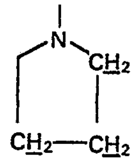 )

2,6 à 3,20 (mt, $-S-CH_2-CH<$)

3,82 (s, $>CH_2$ en 17)

4,73 (d, $-H_{27}$)

5,45 (d, $-H_{13}$)

6,15 (d, $-H_{11}$)

6,41 (dd, >NH en 8)

8,11 (s, $-H_{20}$)

A 25 g de dichlorhydrate de S-[méthyl-1 pyrrolidinyl-2(S) méthyl] isothiouronium brut en solution dans 100 $cm^3$ d'eau distillée, on ajoute 100 $cm^3$ d'une solution aqueuse de soude 4N puis le mélange est agité à 90°C sous atmosphère d'azote pendant 2 heures. Le mélange réactionnel est refroidi à 0°C, additionné de 25 $cm^3$ d'une solution aqueuse d'acide chlorhydrique 12N puis extrait avec 2 fois 200 $cm^3$ de chlorure de méthylène. La phase organique est séchée sur sulfate de sodium, filtrée puis concentrée à sec sous pression réduite (2,7 kPa) à 30°C. On obtient ainsi 5,9 g de [méthyl-1 pyrrolidinyl-2(S)] méthanethiol sous forme d'une huile jaune clair mise en oeuvre dans la suite de la réaction sans purification supplémentaire.

Rf = 0,15; chromatoplaque de gel de silice; éluant: chloroforme-méthanol (90-10 en volumes).

A 11,9 g de chlorhydrate de [méthyl-1 pyrrolidinyl-2(S)] chlorométhane en solution dans 50 $cm^3$ d'éthanol, on ajoute 19,7 g de thiourée puis on agite à reflux pendant 48 heures. Le mélange est concentré à sec sous pression réduite (2,7 kPa) à 40°C. Le résidu est repris avec 100 $cm^3$ d'éthanol chaud puis filtré sur charbon végétal activé. Après concentration à sec du filtrat sous pression réduite (2,7 kPa) à 40°C, on obtient 25 g d'une huile jaune clair composée du dichlorhydrate de S-[méthyl-1 pyrrolidinyl-2(S) méthyl] isothiouronium et de l'excès de thiourée.

Rf = 0,1; chromatoplaque de gel de silice; éluant: chloroforme-méthanol (90-10 envolumes).

Le chlorhydrate de [méthyl-1 pyrrolidinyl-2(S)] chlorométhane peut être préparé selon la méthode décrite par T. HAYASHI et coll., H. Org. Chem. 48, 2195 (1983).

*Exemple 23*

En opérant d'une manière analogue à celle décrite à l'exemple 1, mais à partir de 2,6 g (méthyl-1 piperidinyl-4) thio-26 pristinamycine $II_B$, de 0,3 $cm^3$ d'acide trifluoroacétique, de 0,8 g d'acide métachloroperbenzoïque et après purification par chromatographie «flash» [éluant: chloroforme-méthanol (90-10 en volumes)] en recueillant des fractions de 40 $cm^3$ et concentration à sec des fractions 20 à 35 sous pression réduite (2,7 kPa) à 30°C, on obtient 0,33 g de (méthyl-1 pipéridinyl-4) sulfinyl-26 pristinamycine $II_B$ (isomère $A_2$) sous forme d'une poudre jaune fondant vers 170°C.

Spectre RMN:

1,76 (s, $CH_3$ en 33)

2,2 à 3,00 (mt, 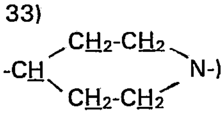

2,32 (s, $>N-CH_3$)

3,82 (s, $>CH_2$ en 17)

4,85 (d, $-H_{27}$)

5,50 (d, $-H_{13}$)

6,19 (d, $-H_{11}$)

6,37 (dd, >NH en 8)

8,15 (s, $-H_{20}$)

La (méthyl-1 piperidinyl-4) thio-26 pristinamycine $II_B$ peut être obtenue de la manière suivante:

En opérant d'une manière analogue à celle décrite à l'exemple 3, mais à partir de 3,15 g de pristinamycine $II_A$ et 1,6 g de méthyl-2 piperidinethiol-4 et addition de 0,6 g de triéthylamine au mélange réactionnel et après purification par chromatographie «flash» [éluant: chlorure de méthylène-méthanol (92-8 en volumes)] et concentration à sec des fractions 4 à 20 sous pression réduite (2,7 kPa) à 30°C, on obtient 0,9 g de (méthyl-1 piperidinyl-4) thio-26 pristinamycine $II_B$ sous forme d'une poudre jaune fondant vers 180°C.

Spectre RMN:

2,10 (m, 4H ; -S- 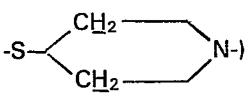

2,25 (s, 3H : -S- 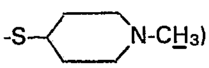

2,8 (m, 4H : -S- 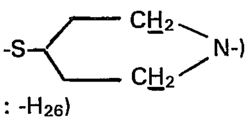

3,55 (m, 1H : $-H_{26}$)

4,62 (m, 1H : $-H_{27}$)

7,70 (m, 1H : $-H_8$)

8,10 (s, 1H : $-H_{20}$)

Le méthyl-2 piperidinethiol-4 peut être préparé par la méthode décrite par H. BARRER et R.E. LYLE, J. Org. Chem., 27, 641 (1962).

*Exemple 24*

A 7,8 g de (diéthylamino-2 éthyl) thio-26 pristinamycine $II_B$ en solution dans 60 $cm^3$ de méthanol, on ajoute à 0°C sous atmosphère d'azote, 0,92 $cm^3$ d'acide trifluoroacétique. Après 15 minutes à 0°C, la température est élevée à 15°C, puis 1,37 g de dioxyde de sélénium est ajouté. Lorsque tout le dioxyde de sélénium est dissous, on ajoute lentement à une température inférieure à 25°C, 7 $cm^3$ d'une solution aqueuse d'eau oxygénée à 30%. Après 1 heure d'agitation à 25°C, le mélange réactionnel est refroidi à 10°C, additionné de 50 $cm^3$ d'une solution aqueuse saturée de bicarbonate de sodium puis extrait avec 4 fois 50 $cm^3$ de chlorure de méthylène. Les phases organiques sont réunies, séchées sur du sulfate de magnésium, filtrées puis concentrées à sec sous pression réduite (2,7 kPa) à 30°C. Le solide jaune obtenu est purifié par chromatographie «flash» [éluant: chloroforme-méthanol 90-10 en volumes)] en recueillant des fractions de 40 $cm^3$. Après concentration à sec sous pression réduite (2,7 kPa) à 30°C des fractions 31 à 38, on obtient un solide jaune qui est purifié par chromatographie «flash» [éluant: acétate d'éthyle-méthanol (80-20 en volumes)] en recueillant des fractions de 40 $cm^3$. Après concentration à sec sous pression réduite des fractions 27 à 33, on obtient un solide

blanc qui est agité dans 50 $cm^3$ d'éther éthylique, séparé par filtration puis séché sous pression réduite (90 Pa) à 30°C. On obtient ainsi 0,5 g de (diéthylamino-2 éthyl) sulfonyl-26 pristinamycine $II_B$ (isomère B) sous forme d'un solide blanc fondant vers 150°C.

Spectre RMN:

0,97 (d, $-CH_3$ en 30 et 31 et $-CH_3$ de l'éthyle)
1,75 (s, $-CH_3$ en 33)

$CH_2$-
2,62 (q, -N< )
$CH_2$-

3,00 à 3,40 (mt, $-SO_2CH_2CH_2N<$)
3,82 (s, $>CH_2$ en 17)
5,34 (d, $-H_{13}$)
5,43 (d, $-H_{13}$)
6,16 (d, $-H_{11}$)
6,54 (dd, >NH en 8)
8,10 (s, $-H_{20}$)

*Exemple 25*

On opère d'une manière analogue à celle décrite à l'exemple 24, mais à partir de 6,86 g de (diisopropylamino-2 éthyl) thio-26 pristinamycine $II_B$ (isomère A), de 0,77 $cm^3$ d'acide trifluoroacétique, de 1,15 g de dioxyde de sélénium, de 6,33 $cm^3$ d'une solution aqueuse d'eau oxygénée à 30%. Après purification par chromatographie «flash» [éluant: acétate d'éthyle-méthanol (80-20 en volumes)] en recueillant des fractions des 40 $cm^3$ en concentration à sec des fractions 28 à 31 sous pression réduite (2,7 kPa) à 30°C, on obtient 0,7 g d'un solide jaune qui est à nouveau purifié par chromatographie «flash» [éluant: acétate d'éthyle-méthanol (85-15 en volumes)] en recueillant des fractions de 30 $cm^3$. Après concentration à sec sous pression réduite des fractions 26 à 33, on obtient un solide jaune qui est agité dans 30 $cm^3$ d'éther éthylique, séparé par filtration puis séché sous pression réduite (90 Pa) à 30°C. On obtient 0,6 g de (diisopropylamino-2 éthyl) sulfonyl-26 pristinamycine $II_B$ (isomère A) sous forme d'un solide jaune clair fondant vers 140°C.

Spectre RMN:

1,06 (d, $-CH_3$ isopropyle)
1,75 (s, $-CH_3$ en 33)
2,79 (mt, $-H_4$)
2,92 et 3,10 (2dd, $>CH_2$ en 15)
2,7 à 3,30 [mt, $-S-CH_2CH_2N(CH<_2]$

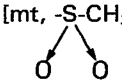

3,52 (d large, $-H_{26}$)
3,82 (s. $>CH_2$ en 17)
5,27 (d fin, $-H_{27}$)
5,47 (d, $-H_{13}$)
6,17 (d, $-H_{11}$)
6,42 (mt, >NH en 8)
8,12 (s, $-H_{20}$)

*Exemple de référence 1*

A une solution de 0,41 $cm^3$ de diméthylamino-3 propylamine dans 15 $cm^3$ de méthanol contenant 2,4 $cm^3$ d'une solution méthanolique 2N d'acide chlorhydrique gazeux maintenue à 55°C, on ajoute 0,5 g de pristinamycine $I_A$ et 20 mg de cyanoborohydrure de sodium. On laisse ensuite renvenir la solution obtenue à une température voisine de 20°C pendant environ 2 heures, puis on la concentre à sec sous pression réduite (2,7 kPa) à 30°C. Le résidu obtenu est trituré avec un mélange de 50 $cm^3$ de chlorure de méthylène et de 50 $cm^3$ d'une solution aqueuse saturée de bicarbonate de sodium; la phase organique est décantée et la phase aqueuse est extraite 2 fois par 20 $cm^3$ au total de chlorure de méthylène. Les phases organiques sont réunies, séchées sur du sulfate de magnésium, filtrées puis concentrées à sec sous pression réduite (2,7 kPa) à 30°C. Le résidu obtenu est purifié par chromatographie «flash» [éluant: chloroforme-méthanol (80-20 volumes)]. Les fractions 15 à 30 sont réunies et concentrées à sec sous pression réduite (2,7 kPa) à 30°C; le résidu obtenu est trituré avec 5 $cm^3$ d'éther éthylique, filtré et séché sous pression réduite (0,027 kPa) à 20°C. On obtient ainsi 60 mg de désoxy-5γ (diméthylamino-3 propyl) amino-5γ pristinamycine $I_A$ sous forme d'une poudre crème fondant vers 160°C.

Le spectre de RMN complet présente les caractéristiques suivantes:

| δ (ppm) | Forme du signal | Attribution |
|---|---|---|
| 8,40 | d | 6 NH |
| 8,25 | d | 1 NH |
| 7,55 | dd | $1'H_6$ |
| 7,05 | m | 6γ + 6δ + 6ε |
| 7 | dd | $1'H_4$ |
| 6,90 | dd | $1'H_5$ |
| 6,70 | d } | 4δ + 4ε |
| 6,40 | d } | |
| 6,50 | d | 2 NH |
| 5,75 | ddd | 1β |
| 5,45 | d | 6α |
| 5,25 | dd | 4α |
| 5 | s (large) | 5α |
| 4,75 | dd | 1α |
| 4,60 | m | 2α |
| 4,45 | d (large) | $5\varepsilon_1$ |
| 4,40 | dd | 3α |
| 3,4 | dd (large) | $3\delta_1$ |
| 3,20 | dd (large) | $3\delta_2$ |
| 3 | s | 4 $CH_3$ |
| 3 | m | 5γ + $4\beta_{1\text{ et }2}$ |
| 2,80 | s | 4 $N(CH_3)_2$ |
| 2,65 | t | $-NCH_2-$ (chaîne) |
| 2,35 | m | $5\varepsilon_1 + 5\beta_1$ |
| 2,25 | t | $-NCH_2-$ (chaîne) |
| 2,20 | s | $-N(CH_3)_2$ (chaîne) |
| 1,60 | m | $-CH_2-$ (chaîne) 2β + 3γ |
| 1,25 | d | 1γ |
| 0,90 | t | 2γ |
| 0,50 | dddd | $5\beta_2$ |

On obtient une solution aqueuse à 10% de désoxy-5γ (diméthylamino-3 propyl) amino-5γ pristinamycine $I_A$ (produit A), à l'état de chlorhydrate, avec:

| | |
|---|---|
| produit A | 0,1 g |
| acide chlorhydrique 2N | 0,52 $cm^3$ |
| eau distillée | qsp 1 $cm^3$ |

En opérant de manière analogue à celle décrite à l'exemple de référence 1, on prépare les synergistines de formule générale (V) suivantes qui peuvent être associées aux produits selon l'invention:

[Les symboles ------, Z et $R_1$ sont définis comme en 1) pour la formule générale (V)].

| Exemple de référence | Y | X | 1) Point de fusion<br>2) Solubilité |
|---|---|---|---|
| 2 | $-N(CH_3)_2$ | $-NH(CH_2)_2N(CH_3)_2$ | 1) Poudre jaune, F env. 180°C<br>2) Solution aqueuse à 10% à l'état de chlorhydrate |
| 3 | $-N(CH_3)_2$ | 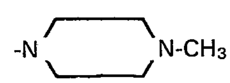 | 1) Poudre blanche, F env. 195°C<br>2) Solution aqueuse à 10% à l'état de chlorhydrate |
| 4 | $-N(CH_3)_2$ | 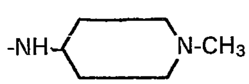 | 1) Poudre beige, F env. 195°C<br>2) Solution aqueuse à 3,7% à l'état de chlorhydrate |
| 5 | $-N(CH_3)_2$ | -NHOH | 1) Poudre blanche, F env. 170°C<br>2) Solution aqueuse à 10% à l'état de chlorhydrate |
| 6 | $-N(CH_3)_2$ | $-NH(CH_2)_3OH$ | 1) Poudre crème, F env. 160°C<br>2) Solution aqueuse à 2% à l'état de chlorhydrate |
| 7 | -H | $-NH(CH_2)_3N(CH_3)_2$ | 1) Poudre beige, F env. 140°C<br>2) Solution aqueuse à 10% à l'état de chlorhydrate |

*Exemple de référence 8*

A une solution de 2 g de pristinamycine $I_A$ dans 25 $cm^3$ de méthanol, on ajoute 2,8 $cm^3$ d'une solution éthanolique 5N de diméthylamine puis 2 $cm^3$ d'une solution méthanolique 5N d'acide chlorhydrique gazeux. A la solution ainsi obtenue, on ajoute 76 mg de cyanoborohydrure de sodium puis on agite pendant 48 heures à une température voisine de 20°C. Le mélange réactionnel est ensuite concentré à sec sous pression réduite (2,7 kPa) à 30°C. Le résidu est trituré avec un mélange de 25 $cm^3$ de chlorure de méthylène et 25 $cm^3$ d'une solution aqueuse saturée de bicarbonate de sodium; la phase organique est décantée et la phase aqueuse est extraite 2 fois par 50 $cm^3$ au total de chlorure de méthylène. Les phases organiques sont réunies, séchées sur du sulfate de magnésium, filtrées puis concentrées à sec sous pression réduite (2,7 kPa) à 30°C. Le résidu est purifié par chromatographie «flash» [éluant: chloroforme-méthanol (92-8 en volumes)]. Les fractions 5 à 12 sont réunies et concentrées à sec sous pression réduite (2,7 kPa) à 30°C. On obtient ainsi 0,7 g de désoxy-5γ diméthylamino-5γ pristinamycine $I_A$ sous forme d'une poudre beige fondant vers 170°C.

Spectre RMN:

0,70 (dt, 1H : $5\beta_2$)

2,10 à 2,60 (m, 4H : $5\delta_1 + 5\beta + 5\gamma$)

2,15 (s, 3H X 0,8 : $-N(CH_3)_2$ 1er isomère)

2,20 (s,, 3H X 0,2 : $-N(CH_3)_2$ 2ème isomère)

On obtient une solution aqueuse à 2% de désoxy-5γ diméthylamino-5γ pristinamycine $I_A$ (produit B), à l'état de chlorhydrate avec:

| | | |
|---|---|---|
| produit B | | 0,05 g |
| acide chlorhydrique 0,1N | | 0,56 $cm^3$ |
| eau destillée | qsp | 2,5 $cm^3$ |

*Exemple de référence 9*

En opérant d'une manière analogue à celle décrite à l'exemple de référence 8, on obtient 0,35 g de désoxy-5γ méthylamino-5γ pristinamycine $I_A$ sous forme de poudre jaune fondant vers 185°C.

On obtient une solution aqueuse à 1% de désoxy-5γ méthylamino-5γ pristinamycine $I_A$, à l'état de chlorhydrate.

*Exemple de référence 10*

En opérant d'une manière analogue à celle décrite à l'exemple de référence 8, on obtient 1,2 g de désoxy-5γ [N-(diméthylamino-2 éthyl) N-méthylamino]-5γ pristinamycine $I_A$ sous forme d'une poudre blanche fondant vers 120°C.

On obtient une solution aqueuse à 10% de désoxy-5γ [N-(diméthylamino-2 éthyl) N-méthylamino]-5γ pristinamycine $I_A$ (produit D), à l'état de chlorhydrate.

*Exemple de référence 11*

On ajoute 5 g de tamis molécualire 3 Å à une solution de 3 g de pristinamycine $I_A$, 3,3 g de diéthylamino-4 méthyl-2 butylamine, 0,11 g de cyanoborohydrure de sodium et 9 $cm^3$ d'une solution méthanolique 5N d'acide chlorhydrique gazeux dans 75 $cm^3$ de méthanol. La suspension obtenue est agitée pendant 4 jours à une température voisine

de 20°C, puis est filtrée; le filtrat est concentré à sec sous pression réduite (2,7 kPa) à 30°C. Le résidu est trituré avec un mélange de 50 cm$^3$ de chlorure de méthylène et de 50 cm$^3$ d'une solution aqueuse saturée de bicarbonate de sodium; la phase organique est décantée et la phase aqueuse est extraite 2 fois par 50 cm$^3$ au total de chlorure de méthylène. Les phases organiques sont réunies, séchées sur du sulfate de magnésium, filtrées puis concentrées à sec sous pression réduite (2,7 kPa) à 30°C. Le résidu est purifié par chromatographie «flash» [éluant: chloroforme-méthanol (90-10 en volumes)]. On obtient ainsi 0,7 g de désoxy-5γ (diéthylamino-4 méthyl-2 butyl) amino-5γ pristinamycine $I_A$ sous forme d'une poudre beige fondant vers 160°C.

Spectre RMN:

1,10 (mt, 9H : $-N(CH_2C\underline{H}_3)_2$ + $\underset{|}{C}H-C\underline{H}_3$)

vers 1,7 (m, 4H : $-C\underline{H}_2-C\underline{H}_2-CH_2-N(C_2H_5)_2$)

2,90 (m, 6H : $-C\underline{H}_2N(C\underline{H}_2CH_3)_2$)

7,70 (mt, 1H × 0,45 : l'$H_6$ 1er isomère)

7,77 (mt, 1H × 0,55 : l'$H_6$ 2ème isomère)

On obtient une solution aqueuse à 10% de désoxy-5γ (diéthylamino-4 méthyl-2 butyl) amino-5γ pristinamycine $I_A$ (produit F), à l'état de chlorhydrate, avec:

| | | |
|---|---|---|
| produit F | | 0,1 g |
| acide chlorhydrique 0,1N | qsp | 1 cm$^3$ |

*Exemple de référence 12*

A une solution de 12,5 g de désoxy-5γ hydroxyimino-5γ pristinamycine $I_A$ dans 300 cm$^3$ de méthanol contenant 10 cm$^3$ d'une solution méthanolique 2N d'acide chlorhydrique gazeux, on ajoute 0,7 g de cyanoborohydrure de sodium. La solution obtenue est agitée 2 jours à une température voisine de 20°C, puis concentrée à sec sous pression réduite (2,7 kPa) à 30°C. Le résidu est trituré dans un mélange de 200 cm$^3$ de chlorure de méthylène et de 100 cm$^3$ d'une solution aqueuse saturée de bicarbonate de sodium; la phase organique est décantée et la phase aqueuse est extraite par 100 cm$^3$ de chlorure de méthylène. Les phases organiques sonr réunies, séchées sur du sulfate de magnésium, filtrées et concentrées à sec sous pression réduite (2,7 kPa) à 30°C. On obtient après purification par chromatographie «flash» [éluant: chloroforme-méthanol (95-5 en volumes)] 6,8 g de désoxy-5γ hydroxyamino-5γ pristinamycine $I_A$ sous forme d'une poudre blanche fondante vers 170°C.

Spectre RMN:

0,4 (m, 1H : 5$\beta_2$)

2,45 (d, 1H : 5$\beta_1$)

3,1 (d : 5γ dans un massif complexe)

7,80 (mt, 1H × 0,75 : 1'$H_6$ 1er isomère)

7,95 (mt, 1H × 0,25 : 1'$H_6$ 2ème isomère)

La désoxy-5γ hydroxyimino-5γ pristinamycine $I_A$ peut être obtenue en agitant pendant 5 heures à une température voisine de 20°C, 15 g de pristinamycine $I_A$ et 7,5 g de chlorhydrate d'hydroxylamine en solution dans 150 cm$^3$ de méthanol contenant 8 cm$^3$ d'une solution méthanolique 2N d'acide chlorhydrique gazeux. Le mélange réactionnel est ensuite concentré à sec sous pression réduite (2,7 kPa) à 30°C. Le résidu est trituré avec un mélange de 100 cm$^3$ de chloroforme et de 100 cm$^3$ d'une solution aqueuse saturée de bicarbonate de sodium; la phase organique est décantée et la phase aqueuse est extraite 2 fois par 200 cm$^3$ au total de chloroforme. Les phases organiques sont réunies, séchées sur du sulfate de magnésium, filtrées et concentrées à sec sous pression réduite (2,7 kPa) à 30°C. On obtient ainsi 14 g de désoxy-5γ hydroxyimino-5γ pristinamycine $I_A$ sous forme d'une poudre beige fondant à 210°C.

Spectre RMN:

0,35 (dd, 1H : 5$\beta_2$)

3,25 (m, 2H : 4$\varepsilon_2$ + 5$\beta_1$)

5,05 (d, 1H : 5α)

5,5 (m, 2H dont 5$\varepsilon_1$)

7,80 (dd, 1H × 0,40 : 1'H 1er isomère)

7,90 (dd, 1H × 0,60 : 1'H 2ème isomère)

*Exemple de référence 13*

En opérant d'une manière analogue à celle décrite à l'exemple de référence 11, on obtient 0,8 g de [N-(carboxyméthyl) méthylamino]-5γ désoxy-5γ pristinamycine $I_A$ sous forme de poudre crème fondant vers 140°C.

On obtient une solution aqueuse à 2% de [N-(carboxyméthyl) méthylamino]-5γ désoxy-5γ pristinamycine $I_A$ (produit K) avec:

| | | |
|---|---|---|
| produit K | | 0,2 g |
| eau distillée | qsp | 10 cm$^3$ |

*Exemple de référence 14*

A une solution de 3,2 g de désoxy-5γ (diméthylamino-2 éthyl) amino-5γ pristinamycine $I_A$ dans 50 cm$^3$ de chloroforme contenant 0,6 cm$^3$ de triéthylamine, on ajoute 0,3 cm$^3$ de chlorure d'acétyle. Le mélange réactionnel est agité pendant 30 minutes à une température voisine de 20°C, puis concentré à sec sous pression réduite (2,7 kPa) à 30°C. Le résidu est purifié par chromatographie «flash» [éluant: chloroforme-méthanol (90-10 en volumes)]; par concentration à sec des fractions 10 à 21 sous pression réduite (2,7 kPa) à 30°C, on obtient 1,8 g de désoxy-5γ [N-diméthylamino-2 éthyl) acétamido]-5γ pristinamycine $I_A$ sous forme d'une poudre blanche fondant vers 170°C.

Spectre RMN:

0,9 (m, 4H : 2γ + 5$\beta_2$)

2,05 à 2,15 (m, 3H : 5$\delta_1$ + 5$\delta_2$ + 5γ)

2,15 (s, 3H : $-COCH_3$)

2,45 (s, 6H : $-N(CH_3)_2$)

2,35 à 2,60 (m, 5H : $>N-C\underline{H}_2-C\underline{H}_2-N<$ + 5$\beta_1$)

7,8 (mt, 1H × 0,75 : 1'$H_6$ 1er isomère)

8,25 (mt, 1H × 0,25 : 1'$H_6$ 2ème isomère)

On obtient une solution aqueuse à 10% de désoxy-5γ [N-(diméthylamino-2 éthyl) acétamido]-5γ pristinamycine $I_A$ (produit L), à l'état de chlorhydrate, avec:

| | | |
|---|---|---|
| produit L | | 0,1 g |
| acide chlorhydrique 0,2N | | 0,51 cm$^3$ |
| eau distillée | qsp | 1 cm$^3$ |

La désoxy-5γ (diméthylamino-2 éthyl) amino-5γ pristinamycine $I_A$ peut être préparée comme décrit à l'exemple de référence 2.

*Exemple de référence 15*

En opérant d'une manière analogue à celle décrite à l'exemple de référence 14, on obtient 1,6 g de désoxy-5γ [N-(diméthylamino-3 propyl) acétamido]-5γ pristinamycine $I_A$ sous forme d'une poudre ocre fondant à 210°C.

On obtient une solution aqueuse à 10% de désoxy-5γ [N-(diméthylamino-3 propyl) acétamido]-5γ pristinamycine $I_A$ (produit M), à l'état de chlorhydrate.

*Exemple de référence 16*

A une solution de 3,6 g de méthylène-5δ pristinamycine $I_A$ dans un mélange de 25 $cm^3$ de méthanol et de 5 $cm^3$ de chloroforme, on ajoute 1,95 g de diméthylamino-3 propanethiol, puis on agite la solution obtenue pendant 20 heures à une température voisine de 20°C. Le mélange réactionnel est ensuite versé dans 250 $cm^3$ d'eau distillée; l'émulsion obtenue est extraite 3 fois par 250 $cm^3$ au total de chlorure de méthylène. Les phases organiques sont réunies, séchées sur du sulfate de magnésium, filtrées plus concentrées à sec sous pression réduite (2,7 kPa) à 30°C. Le résidu obtenu est purifié par chromatographie «flash» [éluant: chloroforme-méthanol (95-5 en volumes)]; les fractions 10 à 38 sont concentrées à sec sous pression réduite (2,7 kPa) à 30°C. Le résidu obtenu est trituré dans 30 $cm^3$ d'éther éthylique; les cristaux obtenus sont séparés par filtration, puis séchées sous pression réduite (27 Pa) à 20°C. On obtient ainsi 2,4 g de (diméthylamino-3 propyl) thiométhyl-5δ pristinamycine $I_A$ sous forme de cristaux blancs fondant à 234°C.

Spectre RMN:

| δ (ppm) | Forme | Attribution |
|---|---|---|
| 11.65 | s (large) | OH |
| 8,70 | d | 6 NH |
| 8,40 | d | 1 NH |
| 7,80 | dd | $1'H_6$ |
| 7,45 | m | $1'H_4 + 1'H_5$ |
| 7,27 | m } | 6 γ + 6δ + 6ε |
| 7,17 | m } | |
| 7,05 | d } système AB | 4δ + 4ε |
| 6,60 | d } | |
| 6,47 | d | 2 >NH |
| 5,87 | ddd | 1β |
| 5,83 | d | 6α |
| 5,24 | m | 5α + 4α |
| 5,03 | ddd | $5ε_1$ |
| 4,85 | dd | 1α |
| 4,80 | m | 2α |
| 4,53 | dd | 3α |
| 3,53 | m | $3δ_1$ |
| 3,35 | dd } système ABX | $-C\underline{H}_2-S-SCH_2-$ |
| 3,15 | dd } | |
| 3,25 | s | 4 >$NCH_3$ |
| 3,25 | m | $3δ_2$ |
| 2,90 | s | 4 $-N(CH_3)_2$ |
| 2,90 | m | 4β |
| 2,55 | t | $-C\underline{H}_2N\langle^{CH_3}_{CH_3}$ |
| 2,50 | dd | $5ε_2$ |
| 2,40 | t | $-Ch_2SC\underline{H}_2-$ |
| 2,40 à 2,20 | m | $5δ + 5β_1$ |
| 2,25 | s | $-CH_2N(C\underline{H}_3)_2$ |
| 2 | m | $3β_1$ |
| 1,75 | m | $-SCH_2C\underline{H}_2CH_2-$ |
| 1,8 à 1,45 | m | $2β_1 + 2β_2 + 3γ_1$ |
| 1,30 | d | 1γ |
| 1,25 à 1,05 | m | $3γ_2 + 3β_2$ |
| 0,9 | t | 2γ |
| 0,60 | dd | $5β_2$ |

Ob obtient une solution aqueuse à 10% de (diméthylamino-3 propyl) thiométhyl-5δ pristinamycine $I_A$ (produit AA), avec:

| | |
|---|---|
| produit AA | 30 mg |
| acide chlorhydrique 0,1N | qsp 0,3 $cm^3$ |

La méthylène-5δ pristinamycine $I_A$ peut être préparée de la manière suivante:

A une solution de 12 g de diméthylaminométhylène-5δ pristinamycine $I_A$ dans 230 $cm^3$ de tétrahydrofuranne contenant 1,2 $cm^3$ d'acide trifluoroacétique, on ajoute 0,43 g de cyanoborohydrure de sodium. La solution obtenue est agitée pendant 4 heures à une température voisine de 20°C, puis est concentrée à sec sous pression réduite (2,7 kPa) à 30°C. Le résidu obtenu est purifié par chromatographie «flash» [éluant: chloroforme-méthanol (95-5 en volumes)]; les fractions 4 à 15 sont concentrées à sec sous pression réduite (2,7 kPa) à 30°C. On obtient ainsi 5,5 g de méthylène-5δ pristinamycine $I_A$ sous forme de cristaux blancs fondant à 245°C.

Spectre RMN:

0,55 (dd, 1H × $5β_2$)

2,40 (d, 1H : $5β_1$)

3,55 (dd, 1H : $5ε_2$)

5,25 (m, 2H : $5α + 5ε_1$)

5,30 et 6,10 (2s, 2H : $=C\langle^{\underline{H}}_{\underline{H}}$ )

7,85 (dd, 1H : $1'H_6$)

La diméthylaminoéthylène-5δ pristinamycine $I_A$ peut être préparée de la manière suivante:

A une solution de 46 g de pristinamycine $I_A$ dans 460 $cm^3$ de dichloro-1,2 éthane, on ajoute 230 $cm^3$ de tert-butoxy bis(diméthylamino) méthane; la solution obtenue est agitée pendant 18 heures à une température voisine de 20°C. Le mélange réactionnel est dilué par 1 litre de chlorure de méthylène puis lavé 3 fois par 3 litres au total d'une solution aqueuse à 0,4% de chlorure d'ammonium. La phase organique est séchée sur du sulfate de magnésium, filtrée puis concentrée à sec sous pression réduite (2,7 kPa) à 30°C. Le résidu obtenu est trituré avec 600 $cm^3$ d'eau distillée; le mélange est filtré et le produit solide est séché sous pression réduite (2,7 kPa) à 20°C. On obtient 41 g de diméthylaminométhylène-5δ pristinamycine $I_A$ brute sous forme d'une poudre beige. Ce produit est d'une qualité suffisante pour être utilisé tel que dans les phases ultérieures. Il peut toutefois être purifié de la manière suivante:

23,5 g de diméthylaminométhylène-5δ pristinamycine $I_A$ brute sont purifiés par chromatographie «flash» [éluant: chloroforme-méthanol (98-2 en volumes)]. Les fractions 16 à 25 sont réunies et concentrées à sec sous pression réduite (2,7 kPa) à 30°C. On obtient ainsi 12 g de diméthylaminométhylène-5δ pristinamycine $I_A$ sous forme d'une poudre beige fondant vers 195°C.

Spectre RMN:
0,9 (t, 3H : 2γ)
1,0 (dd, 1H : 5$\beta_2$)
2,50 (d, 1H, 5$\beta_1$)
3,10 (s, 6H : $-N(CH_3)_2$)
3,79 (d, 1H : 5$\varepsilon_2$)
5,50 (d, 1H : 5$\varepsilon_1$)
7,40 (s, 1H : $=C\underline{H}N(CH_3)_2$)
7,75 (dd, 1H : 1'$H_6$)

*Exemple de référence 17*

En opérant d'une manière analogue à celle décrite à l'exemple de référence 16, mais à partir de 0,9 g de méthylène-5δ virginiamycine S et 0,52 g de diméthylamino-3 propanethiol et après purification par chromatographie «flash» [éluant: chloroforme-méthanol (90-10 en volumes)] et concentration à sec des fractions 13 à 25 sous pression réduite (2,7 kPa) à 30°C, on obtient 0,3 g de (diméthylamino-3 propyl) thiométhyl-5δ virginiamycine S sous forme d'une poudre blanche fondant vers 142°C.

Spectre RMN:
0,45 (dd, 1H : 5$\beta_2$)
1,90 (m, 2H : $-SCH_2C\underline{H}_2CH_2N<$)
2,40 (s, 6H : $-CH_2-N<(C\underline{H}_3)(C\underline{H}_3)$)
2,60 (m, 4H : $-S-C\underline{H}_2-CH_2-C\underline{H}_2-N<$)
3,45 (d, 1H : 5$\varepsilon_2$)
4,85 (m, 3H dont 5$\varepsilon_1$)
5,25 (dd, 1H : 5α)
7,78 (dd, 1H : 1'$H_6$)

On obtient une solution aqueuse à 10% de (diméthylamino-3 propyl) thiométhyl-5δ virginiamycine S (produit AB), à l'état de chlorhydrate, avec:

| | |
|---|---|
| produit AB | 0,1 g |
| acide chlorhydrique | qsp 1 $cm^3$ |

La méthylène-5δ virginiamycine S peut être préparé d'une manière analogue à celle décrite à l'exemple de référence 16 pour la méthylène-5δ pristinamycine $I_A$, mais à partir de 2 g de diméthylaminométhylène-5δ virginiamycine S et 74 mg de cyanoborohydrure de sodium. Après purification par chromatographie «flash» [éluant: chloroforme-méthanol (98-2 en volumes)] et concentration à sec des fractions 2 à 5 sous pression réduite (2,7 kPa) à 30°C, on obtient 1 g de méthylène-5δ virginiamycine S sous forme d'une poudre beige fondant vers 190°C.

Spectre RMN:
0,35 (dd, 1H : 5$\beta_2$)
2,45 (dd, 1H : 5$\beta_1$)
3,55 (dd, 1H : 5$\varepsilon_2$)
5,25 (dd, 1H : 5$\varepsilon_1$)
5,25 (m, 1H : 5α)
5,30 et 6,15 (2s, 2H : $=C<(\underline{H})(\underline{H})$)
7,75 (dd, 1 : 1'$H_6$)

La diméthylaminoéthylène-5δ virginiamycine S peut être obtenue en opérant d'une manière analogue à celle décrite à l'exemple de référence 16 pour la diméthylaminométhylène-5δ pristinamycine $I_A$, mais à partir de 2 g de virginiamycine S et 10 $cm^3$ de bis-diméthylamino tert-butoxyméthane et après purification par chromatographie «flash» [éluant: chloroforme-méthanol (98-2 en volumes)] et concentration à sec des fractions 9 à 12 sous pression réduite (2,7 kPa) à 30°C, on obtient 0,8 g de diméthylaminométhylène-5δ virginiamycine S sous forme d'une poudre beige fondant vers 175°C.

Spectre RMN:
0,9 (m, 4H : 2γ + 5$\beta_2$)
3,05 (s, 6H : $=CH-N(C\underline{H}_3)_2$)
3,65 (d, 1H : 5$\varepsilon_2$)
4,85 (d, 1H : 5$\varepsilon_1$)
5,15 (dd, 1H : 5α)
7,10 à 7,40 (m : aromatiques + $=CH-N<$)
7,70 (dd, 1H : 1'$H_6$)

*Exemple de référence 18*

En opérant d'une manière analogue à celle décrite à l'exemple de référence 16, mais à partir de 6 g de méthylène-5δ pristinamycine $I_A$ et 4 $cm^3$ de (méthyl-4 pipérazinyl-1)-2 éthanethiol et après purification par chromatographie «flash» [éluant: chloroforme-méthanol (97-3 en volumes)] et concentration à sec des fractions 8 à 20 sous pression réduite (2,7 kPa) à 30°C, on obtient 2,6 g de [(méthyl-4 pipérazinyl-1)-2 éthyl] thiométhyl-5δ pristinamycine $I_A$ sous forme de cristaux blancs fondant à 216°C.

Spectre RMN:
0,60 (dd, 1H : 5$\beta_2$)
2,27 (s, 3H : $>N-CH_3$)
2,40 à 2,80 (m, 11H : $-C\underline{H}_2-N<(C\underline{H}_2-C\underline{H}_2)(C\underline{H}_2-C\underline{H}_2)>N-$ + 5$\beta_1$)
5,05 (dd, 1H : 5$\varepsilon_1$)
5,27 (m, 2H : 5α + 4α)
7,85 (mt, 1H X 0,8 : 1'$H_6$ 1er isomère)
7,95 (mt, 1H X 0,2 : 1'$H_6$ 2ème isomère)

On obtient une solution aqueuse à 5% de [(méthyl-4 pipérazinyl-1)-2 éthyl] thiométhyl-5δ pristinamycine $I_A$ (produit AC), à l'état de chlorhydrate, avec:

| | |
|---|---|
| produit AC | 0,1 g |
| acide chlorhydrique 0,1N | 0,96 $cm^3$ |
| eau distillée | qsp 2 $cm^3$ |

*Exemple de référence 19*

En opérant d'une manière analogue à celle décrite à l'exemple de référence 16, mais à partir de 2 g de méthylène-5δ pristinamycine $I_A$ et 3 $cm^3$ de (méthyl-4 pipérazinyl-1)-3 propanethiol et après purification par chromatographie «flash» [éluant: chloroforme-méthanol (90-10 en volumes)] et concentration à sec des fractions 10 à 15 sous pression réduite (2,7 kPa) à 30°C, on obtient 1,9 g de (méthyl-4 pipérazinyl-1)-3 propyl) thiométhyl-5δ

pristinamycine $I_A$ sous forme d'une poudre blanche fondant vers 156°C.

Spectre RMN:

0,65 (dd, 1H : $5\beta_2$)

2,30 (s, 3H : >N-$CH_3$)

2,50 (m, 13H : -$C\underline{H}_2$N< ($C\underline{H}_2CH_2$)($C\underline{H}_2CH_2$) >N- + -S$C\underline{H}_2$- + $5\beta_1$)

5,27 (m, 2H : $5\alpha + 4\alpha$)

7,85 (dd, 1H X 0,8 : $1'H_6$ 1er isomère)

7,95 (dd, 1H X 0,2 : $1'H_6$ 2ème isomère)

On obtient une solution aqueuse à 10% de [(méthyl-4 pipérazinyl-1)-3 propyl] thiométhyl-5δ pristinamycine $I_A$ (produit AD), à l'état de chlorhydrate, avec:

| | |
|---|---|
| produit AD | 0,1 g |
| acide chlorhydrate 0,5N | 0,38 $cm^3$ |
| eau distillé | qsp 1 $cm^3$ |

*Exemple de référence 20*

En opérant d'une manière analogue à celle décrite à l'exemple de référence 16, mais à partir de 4 g de méthylène-5δ pristinamycine $I_A$ et 4 $cm^3$ de bis-diméthylamino-1,3 propanethiol-2 et après purification par chromatographie «flash» [éluant: chloroforme-méthanol (95-5 en volumes)] et concentration à sec des fractions 20 à 60 sous pression réduite (2,7 kPa) à 30°C, on obtient 0,59 g de [bis(diméthylamino)-1,3 propyl) thiométhyl-5δ pristinamycine $I_A$ sous forme d'une poudre blanche fondant vers 170°C.

Spectre RMN:

0,63 (dd, 1H : $5\beta_2$)

2,40 (s, 6H : -$N(CH_3)_2$)

2,50 (m, 10H : -CH< ($C\underline{H}_2$N<)($C\underline{H}_2$N<) + -$N(CH_3)_2$)

4,97 (s, 1H : $5\varepsilon_1$)

5,30 (m, 2H : $5\alpha + 4\alpha$)

7,85 (mt, 1H X 0,85 : $1'H_6$ 1er isomère)

7,95 (mt, 1H X 0,15 : $1'H_6$ 2ème isomère)

On obtient une solution aqueuse à 7,5% de bis[(diméthylamino)-1,3 propyl] thiométhyl-5δ pristinamycine $I_A$ (produit AE), à l'état de chlorhydrate, avec:

| | |
|---|---|
| produit AE | 0,03 g |
| acide chlorhydrate 0,1N | 0,3 $cm^3$ |
| eau distillé | qsp 0,4 $cm^3$ |

*Exemple de référence 21*

En opérant d'une manière analogue à celle décrite à l'exemple de référence 16, mais à partir de 3 g de méthylène-5δ pristinamycine $I_A$ et 0,97 g de méthyl-2 mercapto-4 pipéridine et après purification par chromatographie «flash» [éluant: chloroforme-méthanol (95-5 en volumes)] et concentration à sec des fractions 10 à 16 sous pression réduite (2,7 kPa) à 30°C, on obtient 1,1 g de (méthyl-1 pipéridyl-4) thiométhyl-5δ pristinamycine $I_A$ sous forme d'une poudre blanche fondant à 260°C.

Spectre RMN:

0,6 (dd, 1H : $5\beta_2$)

2 (m, 4H : -S- 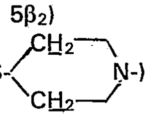 N-)

2,20 (s, 3H : -S- 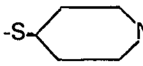 N-$C\underline{H}_3$)

2,35 (m, 1H : $5\beta_1$)

2,90 (m, 4H : -S 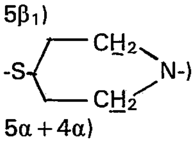 N-)

5,30 (m, 2H : $5\alpha + 4\alpha$)

7,85 (dd, 1H : $1'H_6$)

On obtient une solution aqueuse à 5% de (méthyl-1 pipéridyl-4) thiométhyl-5δ pristinamycine $I_A$ (produit AF), à l'état de chlorhydrate, avec:

| | |
|---|---|
| produit AF | 0,03 g |
| acide chlorhydrate 0,1N | 0,3 $cm^3$ |
| eau distillé | qsp 0,6 $cm^3$ |

*Exemple de référence 22*

En opérant comme à l'exemple de référence 16 mais à partir de 2 g de méthylène-5δ pristinamycine $I_A$ et de 0,66 g de diéthylamino-2 éthanethiol, on obtient après purification par chromatographie «flash» [éluant: chloroforme-méthanol (95-5 en volumes)] et concentration à sec des fractions 9 à 18 sous pression réduite (2,7 kPa) à 30°C, 0,8 g de (diéthylamino-2 éthyl) thiométhyl-5δ pristinamycine $I_A$ sous forme d'une poudre blanche fondant à 230°C.

Spectre RMN:

0,65 (dd, 1H : $5\beta_2$)

2,38 (d, 1H : $5\beta_1$)

2,3 à 2,8 (m, 8H : -S $C\underline{H}_2C\underline{H}_2$ N< ($C\underline{H}_2$-)($C\underline{H}_2$-) )

3,15 (dd, 1H : -$C\underline{H}_2$S-)

3,35 (dd, 1H : -$C\underline{H}_2$S-)

5,01 (dd, 1H : $5\varepsilon_1$)

7,851 (dd, 1H X 0,9 : $1'H_6$ 1er isomère)

7,90 (dd, 1H X 0,1 : $1'H_6$ 2ème isomère)

On obtient une solution aqueuse à 5% de (diéthylamino-2 éthyl) thiométhyl-5δ pristinamycine $I_A$ (produit $AF_1$), à l'état de chlorhydrate, avec:

| | |
|---|---|
| produit $AF_1$ | 30 g |
| acide chlorhydrate 0,1N | 0,29 $cm^3$ |
| eau distillé | qsp 0,6 $cm^3$ |

*Exemple de référence 23*

A une solution de 5,5 g de diméthylaminométhylène-5δ pristinamycine $I_A$ dans 60 $cm^3$ d'acide acétique on ajoute goutte à goutte 5,3 g de diméthylamino-2 éthylamine de manière à ne pas dépasser 25°C. La solution obtenue est agitée pendant 20 heures à une température voisine de 20°C, puis est versée lentement dans une solution aqueuse saturée de bicarbonate de sodium; le mélange obtenu est extrait 2 fois par 750 $cm^3$ au total de chlorure de méthylène. Les phases organiques sont réunies, séchées sur du sulfate de magnésium, filtrées et concentrées à sec sous pression réduite (2,7 kPa) à 30°C. Le résidu est purifié par chromatographie «flash» [éluant: chloroforme-méthanol (90-10 en volumes)]; les fractions 10 à 12 sont concentrées à sec sous pression réduite (2,7 kPa) à 30°C. On obtient ainsi 3 g de (diméthylamino-2 éthyl) aminoéthylène-5δ pristinamycine $I_A$ sous forme d'une poudre beige fondant vers 180°C.

Spectre RMN:
0,90 (mt, 4H : $2\gamma+5\beta_2$)
2,25 (mt, 6H : $-N(CH_3)_2$)
2,50 (mt, 3H : $-CH_2N\langle$ $+5\beta_1$)
3,25 (mt, 2H : $\rangle N-C\underline{H}_2-$)
3,50 (mt, 2H : $5\varepsilon_2+3\delta_1$)
4,90 (mt, 1H : $5\varepsilon_1$)
entre 7,15 et 7,4 (m, 1H : $=C\langle^{NH-}_{\underline{H}}$ )
9,90 (mt, 1H (échangeable $D_2O$) : $-N\underline{H}-$)

On obtient une solution aqueuse à 1% de (diméthylamino-2 èthyl) aminoéthylène-5δ pristinamycine $I_A$ (produit AG), avec:

| | |
|---|---|
| produit AG | 0,1 g |
| eau distillé | qsp 10 $cm^3$ |

*Exemple de référence 24*

En opérant d'une manière analogue à celle décrite à l'exemple de référence 23, mais à partir de 13,8 g de diméthylaminométhylène-5δ pristinamycine $I_A$ et 3,4 g d'amino-4 méthyl-2 pipéridine et après purification par chromatographie «flash» [éluant: chloroforme-méthanol (92,5-7,5 en volumes)] et concentration à sec des fractions 15 à 20 sous pression réduite (2,7 kPa) à 30°C, on obtient 4,0 g de (méthyl-1 pipéridyl-4) aminoéthylène-5δ pristinamycine $I_A$ sous forme d'une poudre jaunbe fondant à 208°C.

Spectre RMN:
0,40 (m, 4H : $2\gamma+2\beta_2$)
2,0 (m, 4H : 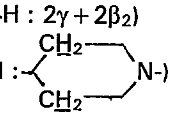
2,35 (s, 3H : $\rangle N-CH_3$)
2,45 (d, 1H : $5\beta_1$)
2,90 (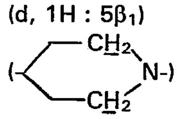
3,20 (sous un massif, 1H : -C$\underline{H}$ 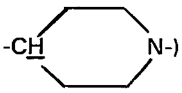
3,50 (d, 1H : $5\varepsilon_2$)
4,85 (sous un massif, 1H : $5\varepsilon_1$)
6,65 (d, 1H : $=C\underline{H}NH-$)
9,70 (dd, 1H X 0,15 : $=CH-N\underline{H}-$ 1er isomère)
10,03 (dd, 1H X 0,85 : $=CH-N\underline{H}-$ 2ème isomère)

On obtient une solution aqueuse à 10% de [(méthyl-1 pipéridyl-4) aminoéthylène-5δ pristinamycine $I_A$ (produit AT), à l'état de chlorhydrate, avec:

| | |
|---|---|
| produit AT | 0,03 g |
| acide chlorhydrate 0,1N | 0,3 $cm^3$ |
| eau distillé | qsp 0,3 $cm^3$ |

L'amino-4 méthyl-2 pipéridine peut être préparée par la méthode décrite par E.F. ELSLAGER, L.M. WERBEL, A. CURRY, N. HEADEN, J. JOHNSON, J. Med. Chem. 17, 99 (1974).

En opérant comme à l'exemple de référence 23, on prépare les synergistiques de formule générale (V) suivantes, qui peuvent être associées aux produits selon l'invention.

[Les symboles ------, X et Z sont définis comme en 2b) pour la formule générale (V) et, sauf mention spéciale, Y représente un radical diméthylamino].

| Exemple de référence | $R_4$ | 1) Point de fusion 2) Solubilité |
|---|---|---|
| 25 | $-NH-(CH_2)_2N(C_2H_5)_2$ | 1) Poudre jaune F env. 150°C 2) Solution aqueuse à 5% à l'état de chlorhydrate |
| 26 | $-NH-(CH_2)_2NH\ CH_3$ | 1) Poudre jaune F = 174°C 2) Solution aqueuse à 1% à l'état de chlorhydrate |
| 27 | $-NH-(CH_2)_3N(CH_3)_2$ | 1) Poudre jaune F env. 155°C 2) Solution aqueuse à 6,6% à l'état de chlorhydrate |
| 28 | $-NH-CH(CH_3)-CH_2N(CH_3)_2$ | 1) Poudre jaune F env. 160°C 2) Solution aqueuse à 5% à l'état de chlorhydrate |
| 29 | $-NHCH_2CH(CH_3)-N(CH_3)_2$ | 1) Poudre orange F env. 175°C 2) Solution aqueuse à 10% à l'état de chlorhydrate |

| Exemple de référence | $R_4$ | 1) Point de fusion<br>2) Solubilité |
|---|---|---|
| 30 | $-NH-CH-(CH_2)_3N(C_2H_5)_2$<br>\|<br>$CH_3$ | 1) Poudre beige<br>F env. 160°C<br>2) Solution aqueuse à 1%<br>à l'état de chlorhydrate |
| 31 | $-NH-(CH_2)_2-$ 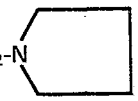 | 1) Poudre jaune<br>F = 183°C<br>2) Solution aqueuse à 1%<br>à l'état de chlorhydrate |
| 32 | $-NH(CH_2)_3-$ 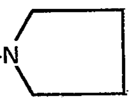 | 1) Poudre jaune<br>F = 170°C<br>2) Solution aqueuse à 1% |
| 33 | $-NH(CH_2)_2-$ 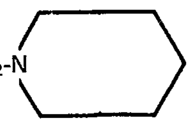 | 1) Poudre jaune<br>F = 162°C<br>2) Solution aqueuse à 1%<br>à l'état de chlorhydrate |
| 34 | $-NH(CH_2)_2-$ 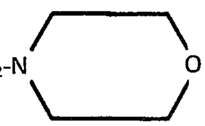 | 1) Poudre beige<br>F env. 172°C<br>2) Solution aqueuse à 1%<br>à l'état de chlorhydrate |
| 35 | $-NH-CH_2-$ 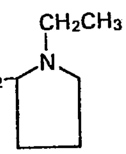 | 1) Poudre beige<br>F env. 160°C<br>2) Solution aqueuse à 1%<br>à l'état de chlorhydrate |
| 36 | -NH- 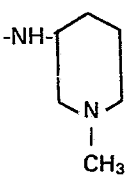 | 1) Poudre beige<br>F = 177°C<br>2) Solution aqueuse à 5%<br>à l'état de chlorhydrate |

| Exemple de référence | Y | $R_4$ | 1) Point de fusion<br>2) Solubilité |
|---|---|---|---|
| 37 | H | -NH- 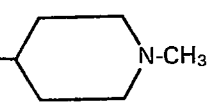 | 1) poudre beige<br>F env. 195°C<br>2) Solution aqueuse à 5%<br>à l'état de chlorhydrate |
| 38 | $-N(CH_3)_2$ | $-NH(CH_2)_2-$ 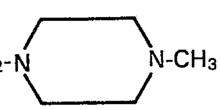 | 1) Poudre jaune<br>F = 150°C<br>2) Solution aqueuse à 10%<br>à l'état de chlorhydrate |
| 39 | $-N(CH_3)_2$ | $-NH-(CH_2)_2-$ 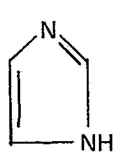 | 1) Poudre jaune<br>F = 138°C<br>2) Solution aqueuse à 10%<br>à l'état de chlorhydrate |

*Exemple de référence*

A une solution de 1,84 g de diméthylaminométhylène-5δ pristinamycine $I_A$ dans 40 $cm^3$ d'acide acétique, on ajoute 2,1 g de diméthylamino-2 éthanethiol. La solution obtenue est agitée pendant 20 heures à une température voisine de 20°C, puis est versée lentement dans une solution aqueuse saturée de biocarbonate de sodium; le mélange obtenu est extrait 3 fois par 400 $cm^3$ au totale de chlorure de méthylène. Les phases organiques sont réunies, séchées sur du sulfate de magnesium, filtrées et concentrées à sec sous pression réduite (2,7 kPa) à 30°C. Le résidu obtenu est purifié par chromatographie «flash» [éluant: chloroforme-méthanol (96-4 en volumes)]; les fractions 5 et 6 sont réunies et concentées à sec sous pression réduite (2,7 kPa) à 30°C. On obtient ainsi 0,8 g de (diméthylamino-2 éthyl) thiométhylène-5δ pristinamycine $I_A$ sous forme d'une poudre jaune fondant vers 150°C.

Spectre RMN:

0,68 (dd, 1H : $5\beta_2$)

2,32 (s, 6H × 0,85 : $-CH_2N(C\underline{H}_3)_2$ 1er isomère)

2,35 (s, 6H × 0,15 : $-CH_2N(C\underline{H}_3)_2$ 2ème isomère)

2,45 (d, 1H : $5\beta_1$)

2,65 (mt, 2H : $-SC\underline{H}_2-$)

3,05 (t, 2H : $-C\underline{H}_2N<$)

3,43 (dd, 1H : $5\varepsilon_2$)

5,15 (dans un massif : $5\varepsilon_1$)

7,60 (s large, 1H : $=C\underline{H}S-$)

7,83 (mt, 1H : $1'H_6$ deux isomères)

On obtient une solution aqueuse à 1% de (diméthylamino-2 éthyl) thiométhylène-5δ pristinamycine $I_A$ (produit AX), à l'état de chlorhydrate, avec:

| | | |
|---|---|---|
| produit AX | | 0,1 g |
| acide chlorhydrique 0,1N | | 1 $cm^3$ |
| eau distillée | qsp | 10 $cm^3$ |

En opérant comme à l'exemple de référence 40 on prépare les synergistines de formule générale (V) suivantes, qui peuvent être associées aux produits selon l'invention .

[Les symboles -------, X et Z sont définis comme en 2) pour la formule générale (V) et, sauf mention spéciale, Y représente un radical diméthylamino].

| Exemple de référence | Y | $R_4$ | 1) Point de fusion 2) Solubilité |
|---|---|---|---|
| 41 | $-N(CH_3)_2$ | $-S-(CH_2)_2N(C_2H_5)_2$ | 1) Poudre beige F env. 192°C 2) Solution aqueuse à 1% à l'état de chlorhydrate |
| 42 | $-N(CH_3)_2$ | $-S-(CH_2)_3N(CH_3)_2$ | 1) Poudre beige F env. 170°C 2) Solution aqueuse à 1% à l'état de chlorhydrate |
| 43 | -H | $-S-(CH_2)_3N(CH_3)_2$ | 1) Poudre beige F env. 140° 2) Solution aqueuse à 10% à l'état de chlorhydrate |
| 44 | $-N(CH_3)_2$ | 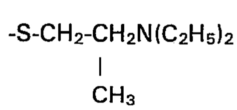 | 1) Poudre beige F env. 234°C 2) Solution aqueuse à 10% à l'état de chlorhydrate |
| 45 | $-N(CH_3)_2$ | 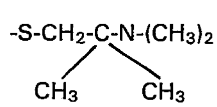 | 1) Poudre beige F env. 200°C 2) Solution aqueuse à 1% à l'état de chlorhydrate |
| 46 | $-N(CH_3)_2$ | 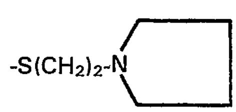 | 1) Poudre beige F env. 180°C 2) Solution aqueuse à 1% à l'état de chlorhydrate |

| Exemple de référence | $R_4$ | 1) Point de fusion<br>2) Solubilité |
|---|---|---|
| 47 | 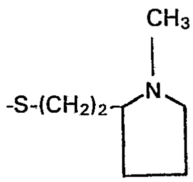 | 1) Poudre beige<br>F env. 215°C<br>2) Solution aqueuse à 0,6%<br>à l'état de chlorhydrate |
| 48 | 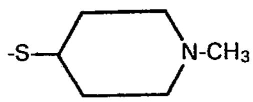 | 1) Poudre jaune<br>F env. 170°C<br>2) Solution aqueuse à 1%<br>à l'état de chlorhydrate |
| 49 | 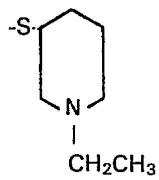 | 1) Poudre beige<br>F env. 175°C<br>2) Solution aqueuse à 1%<br>à l'état de chlorohydrate |
| 50 | $-S-(CH_2)_2N-(CH_2)_2N(CH_3)_2$<br>\|<br>$CH_3$ | 1) Poudre jaune<br>F env. 160°C<br>2) Solution aqueuse à 1% |
| 51 | $-S-CH[CH_2N(CH_3)_2]_2$ | 1) Poudre beige<br>F env. 190°C<br>2) Solution aqueuse à 1%<br>à l'état de chlorhydrate |
| 52 | 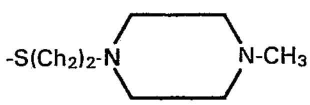 | 1) Poudre beige<br>F env. 170°C<br>2) Solution aqueuse à 1%<br>à l'état de chlorhydrate |
| 53 | 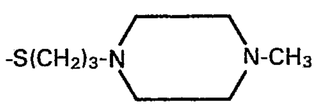 | 1) Poudre beige<br>F env. = 190°C<br>2) Solution aqueuse à 10%<br>à l'état de chlorhydrate |
| 54 | $-S-CH_2-CH-CH_2-N(CH_s3)_3$<br>\| ⊕<br>$CH_3$ | 1) Poudre ocre<br>F env. 150°C<br>2) Solution aqueuse à 1%<br>à l'état de chlorohydrate |
| 55 | $-S(CH_2)_2SO_3H$ | 1) Poudre jaune<br>F > 280°C<br>2) Solution aqueuse à 5% |

*Exemple de référence 56*

A une solution de 0,87 g de (mercapto-2 propyl)-1 méthyl-4 pipérazine dans 50 $cm^3$ d'éthanol additionnée de 0,34 g d'éthylate de sodium, on ajoute une solution de 5,2 g de (méthyl-4 phényl) sulfonyloxyméthylène-5δ pristinamycine $I_A$ dans 50 $cm^3$ de chlorure de méthylène. Le mélange réactionnel est agité pendant 16 heures à une température voisine de 20°C puis dilué avec 500 $cm^3$ de chlorure de méthylène et 100 $cm^3$ d'eau distillée. Après agitation, la phase aqueuse est extraite 2 fois par 50 $cm^3$ de chlorure de méthylène au total. Les phases organiques sont réunies, séchées sur du sulfate de magnésium, filtrées puis concentrées à sec sous pression réduite (2,7 kPa) à 30°C. Le résidu est purifié par chromatographie «flash» [éluant: chloroforme-méthanol (97,5-2,5 en volumes)]. Les fractions 33 à 80 sont réunies et concentrées à sec sous pression réduite (2,7 kPa) à 30°C. On obtient ainsi 1,25 g de [(méthyl-4 pipérazinyl-1)-3 propyl-2] thiométhylène-5δ pristinamycine $I_A$ sous forme d'une poudre beige fondant vers 195°C.

Spectre RMN:

0,70 (dd, 1H : $5\beta_2$)

1,25 (d, 3H : -CH-C$\underline{H}_3$)

2,30 (s, 3H : >N-C$\underline{H}_3$)

2,50 (m, 10H : -C$\underline{H}_2$-
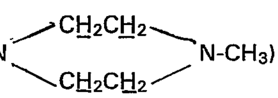

3,40 (dd, 1H : $5\varepsilon_2$)

7,85 (dd large, 1H : $1'H_6$)

On obtient une solution aqueuse à 10% de [(méthyl-4 pipérazinyl-1)-3 propyl-2] thiométhylène-5₃ pristinamycine $I_A$ (produit AAN) sous forme de chlorhydrate, avec:

| | |
|---|---|
| produit AAN | 0,03 g |
| acide chlorhydrique 0,1N | 0,3 $cm^3$ |

La (mercapto-2 propyl)- méthyl-4 pipérazine est préparée en chauffant à 100°C pendant 16 heures un mélange de 19 $cm^3$ de sulfure de propylène et de 29 $cm^3$ de N-méthylpipérazine. On obtient ainsi 32 g d'une huile incolore distillant à 105°C sous 1,3 kPa.

La (méthyl-4 phényl) sulfonyloxyméthylène-5δ pristinamycine $I_A$ peut être obtenue de la manière suivante:

A une solution de 2,7 g d'hydroxyméthylène-5δ pristinamycine $I_A$ dans 30 $cm^3$ de chlorure de méthylène, on ajoute à une température voisine de -30°C, 0,42 $cm^3$ de triéthylamine puis 0,57 g de chlorure de l'acide p-toluènesulfonique. Le mélange réactionnel est agité ensuite pendant 2 heures à une température voisine de 20°C, puis concentré à sec sous pression réduite (2,7 kPa) à 30°C; le résidu obtenu est purifié par chromatographie «flash» [éluant: chlorure de méthylène-méthanol (96-4 en volumes)]. Après concentration à sec des fractions 4 à 6 sous pression réduite (2,7 kPa) à 30°C, on obtient 2,2 g de (méthyl-4 phényl) sulfonyloxyméthylène-5δ pristinamycine $I_A$ sous forme d'une poudre blanche fondant vers 265°C.

Spectre RMN:

0,50 (dd, 1H : $5\beta_2$)

2,35 (s, 3H : -$SO_2$
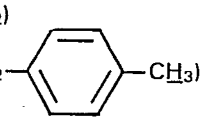

3,30 (dd, 1H : $5\varepsilon_2$)

5,25 (d, 1H : 5α)

5,30 (dd, 1H : $5\varepsilon_1$)

7,35 à 7,90 (système AB + m, 8H : 4δ + 4ε +
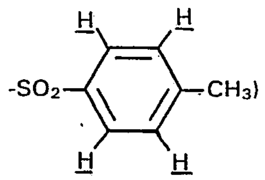

7,85 (dd, 1H : $1'H_6$)

L'hydroxyméthylène-5δ pristinamycine $I_A$ peut être préparée de la manière suivante:

A 420 $cm^3$ d'une solution aqueuse 0,1N d'acide chlorhydrique, on ajoute sous agitation 10,6 g de diméthylaminométhylène-5δ pristinamycine $I_A$. La solution obtenue est ensuite agitée pendant 3 heures à une température voisine de 20°C. On ajoute alors goutte à goutte 30 $cm^3$ d'une solution aqueuse saturée de bicarbonate de sodium de manière à obtenir un pH voisin de 4. Le produit qui précipite est séparé par filtration puis lavé 3 fois par 30 $cm^3$ au total d'eau distillée. Après séchage sous pression réduite (2,7 kPa) à une température voisine de 20°C, on obtient 9,5 g d'hydroxyméthylène-5δ pristinamycine $I_A$ sous forme d'une poudre beige. Ce produit est d'une qualité suffisante pour être utilisé tel quel dans les phases ultérieures. Il peut toutefois être purifié de la manière suivante:

9,5 g d'hydroxyméthylène-5δ pristinamycine $I_A$ brute sont dissous dans 50 $cm^3$ d'acétate d'éthyle; la solution obtenue est versée sur 100 g de gel de silice contenus dans une colonne de 2,8 cm de diamètre. On élue d'abord avec 400 $cm^3$ d'acétate d'éthyle et élimine l'éluat correspondant; on élue ensuite avec 1600 $cm^3$ d'acétate d'éthyle et concentre l'éluat correspondant à sec sous pression réduite (2,7 kPa) à 30°C. On obtient ainsi 6,3 g d'hydroxyméthylène-5δ pristinamycine $I_A$ sious forme de cristaux blancs fondant à 220°C.

Spectre RMN:

0,69 (dd, 1H : $5\delta_2$)

2,43 (d, 1H : $5\beta_1$)

3,40 (d, 1H : $5\varepsilon_2$)

4,0 à 4,2 (m, 3H : 40 + $5\varepsilon_1$ + 5α)

8,15 (s, 1H : =C$\underline{H}$-OH)

11,63 (s large, 1H : =CH-O$\underline{H}$)

*Exemple de référence 57*

En opérant d'une manière analogue à celle décrite à l'exemple de référence 56, on obtient 1 g de (diméthylamino-3 propyl-2) thiométhylène-5δ pristinamycine $I_A$ sous forme d'une poudre jaune fondant à 172°C.

On obtient une solution aqueuse à 5% de (diméthylamino-3 propyl-2) thiométhylène-5δ pristinamycine $I_A$, à l'état de chlorhydrate.

*Exemple de référence 58*

En opérant d'une manière analogue à celle décrite à l'exemple de référence 56, on obtient 1,32 g de (diéthylamino-5 pentyl-2 thiométhylène-5δ pristinamycine $I_A$ sous forme d'une poudre beige fondant vers 185°C.

On obtient une solution aqueuse à 10% de (diéthylamino-5 pentyl-2) thiométhylène-5δ pristinamycine $I_A$, sous forme de chlorhydrate.

*Exemple de référence 59*

Une solution de 7,6 g de [(méthyl-4 phényl) sulfonyloxyméthylène]-5δ pristinamycine $I_A$ dans 60 $cm^3$ de tétrahydrofuranne est refroidie à une température voisine de −10°C. On y ajoute lentement en maintenant cette température une solution de 0,65 g de diméthylamino-2 éthanol dans 60 $cm^3$ de tétrahydrofuranne additionnée de 0,35 g d'une dispersion à 50% d'hydrure de sodium dans l'huile minérale. A la fin de l'addition on laisse remonter lentement la température au voisinage de 20°C. Le mélange réactionnel est agité pendant 24 heures à cette température puis dilué avec 500 $cm^3$ de chlorure de méthylène et lavé 2 fois avec 50 $cm^3$ d'une solution saturée de chlorure d'ammonium. La phase organique est séchée sur du sulfate de magnésium, filtrée puis concentrée à sec sous pression réduite (2,7 kPa) à 40°C. Le résidu obtenu est purifié par chromatographie «flash» [éluant: chloroforme-méthanol (95-5 en volume)]. Les fractions 12 à 17 sont réunies et concentrées à sec sous pression réduite (2,7 kPa) à 25°C. On obtient ainsi 1,5 g de (diméthylamino-2 éthoxyméthylène)-5δ pristinamycine $I_A$ sous forme d'une poudre beige fondant vers 160°C.

Spectre RMN:

0,65 (dd, 1H : $5\beta_2$)
2,3 (s, 6H : $-N(C\underline{H}_3)_2$)
2,65 (m, 2H : $-C\underline{H}_2N<$)
3,42 (dd, 1H : $5\varepsilon_2$)
4,15 (t, 2H : $-OC\underline{H}_2-$)
5,15 (d, 1H : $5\varepsilon_1$)
7,45 (sous les aromatiques, 1H : $>C=C\underline{H}O-$)
7,80 (dd, 1H : $1'H_6$)

On obtient une solution aqueuse à 1% de (diméthylamino-2 éthoxyméthylène)-5δ pristinamycine $I_A$ (produit AAQ), sous forme de chlorhydrate, avec:

| | | |
|---|---|---|
| produit AAQ | | 0,03 g |
| acide chlorhydrique 0,1N | | 0,3 $cm^3$ |
| eau distillée | qsp | 3 $cm^3$ |

La présente invention concerne également les médicaments constitués par un produit de formule générale (I), sous forme libre ou de préférence sous forme de sel d'addition avec un acide pharmaceutiquement acceptable sous forme d'une association avec des synergistines connues ou de préférence avec des synergistines de formule générale (V), l'association pouvant en outre contenir tout autre produit pharmaceutiquement compatible, inerte ou physiologiquement actif. Les médicaments selon l'invention peuvent être utilisés par voie parentérale, orale, rectale ou topique.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tesl que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration rectale sont les suppositoires ou les capsules rectales, qui contiennent outre le principe actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention associés à des synergistines connues ou de préférence à des synergistines de formule générale (V), sont particulièrement utiles dans le traitement des infections d'origine microbienne. Les doses dépendent de l'effet recherché et de la durée du traitement; pour un adulte, elles sont généralement comprises entre 500 et 2000 mg par jour par voie parentérale particulièrement par voie intra-veineuse en perfusion lente, la dose de synergistine de formule générale (V) étant elle-même comprise entre 500 et 2000 mg par jour.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre des compositions selon l'invention.

*Exemple*

On prépare une solution injectable pour perfusion contenant 1 g/l de mélange actif ayant la composition suivante:

| | | |
|---|---|---|
| — (diéthylamino-2 éthyl) sulfinyl-26 pristinamycine $II_B$ | | 0,6 g |
| — [(méthyl-4 pipérazinyl-1)-2 éthyl] thiométhyl-5δ pristinamycine $I_A$ | | 0,4 g |
| — solution aqueuse d'acide chlorhydrique 0,1N | | 12,7 $cm^3$ |
| — eau distillée | qsp | 1000 $cm^3$ |

**Revendications pour les Etats contractants:**
BE, CH, DE, FR, GB, IT, LI, LU, NL, SE

1. Nouveau dérivé de la pristinamycine $II_B$, caractérisé en ce qu'il répond à la formule générale:

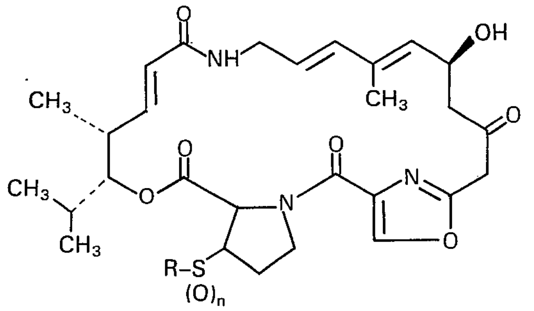

dans la quelle le symbole R représente

– soit un radical hétérocyclyle azoté de 4 à 7 chaînons, contenant éventuellement 1 ou plusieurs autres hétéroatomes choisis parmi l'azote, l'oxygène ou le soufre à l'état de sulfoxyde ou de sulfone, et éventuellement substitué par un radical alcoyle,

– soit une chaîne alcoyle contenant 2 à 4 atomes de carbone substituée par 1 ou 2 radicaux choisis parmi phényle, cycloaloylamino ou N-alcoyl N-cycloalcoyl-amino contenant 3 à 6 chaînons, alcoylamino, dialcoylamino ou dialcoylcarbamoyloxy (les parties alcoyle de ces 2 derniers radicaux pouvant éventuellement former avec l'atome d'azote auquel elles sont rattachées un hétérocycle saturé ou insaturé de 4 à 7 chaînons contenant éventuellement un autre hétéroatome choisi parmi l'azote, l'oxygène ou le soufre à l'état de sulfoxyde ou de sulfone, et éventuellement substitué par un radical alcoyle), un substituée par un ou plusieurs hétérocycles azotés de 4 à 7 chaînons contenant éventuellement 1 ou 2 autres hétéroatomes choisis parmi l'azote, l'oxygène ou le soufre à l'état de sulfoxyde ou de sulfone, et éventuellement substitués par un radical alcoyle, lesdits hétérocycles étant rattachés à la chaîne qui les porte par l'intermédiaire d'un atome de carbone, étant entendu que l'un au moins des substituants portés par la chaîne alcoyle ci-dessus est un substituant azoté capable de former des sels,

– soit un radical [méthyl-1-pyrrolidinyl-2(S)] méthyle,

le symbole n est égal à 1 ou 2 et les radicaux alcoyle cités ci-dessus sont droits ou ramifiés et contiennent 1 à 10 atomes de carbone, sous ses formes isomères ou leurs mélanges, ainsi que ses sels d'addition avec les acides.

2. Un dérivé de la pristinamycine $II_B$ selon la revendication 1, caractérisé en ce que le symbole R représente:

– soit un radical hétérocyclyle azoté à 5 ou 6 chaînons éventuellement substitué par un radical alcoyle,

– soit une chaine alcoyle contenant 2 à 4 atomes de carbone substituée par 1 ou 2 radicaux choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces deux derniers radicaux pouvant éventuellement former avec l'atome d'azote auquel elles sont rattachées, un hétérocycle saturé ou insaturé à 5 ou 6 chaînons contenant éventuellement un autre hétéroatome chosi parmi l'azote, l'oxygène ou le soufre à l'état de sulfoxyde ou de sulfone, et éventuellement substitué par un radical alcoyle), ou substitué par un hétérocycle azoté à 5 ou 6 chaînons contenant éventuellement un autre hétéroatome choisi parmi l'azote, l'oxygène ou le soufre à l'état de sulfoxyde ou de sulfone et éventuellement substitué par un radical alcoyle, ledit hétérocycle étant rattaché à la chaîne alcoyle qui le porte par l'intermédiaire d'un atome de carbone, étant entendu que l'un au moins des substituants portés par la chaîne alcoyle ci-dessus est un substituant azoté capable de former des sels, le symbole n est égal à 1 ou 2 et les radicaux alcoyle cites ci-dessus sont droits ou ramifiés et contiennent 1 à 10 atomes de carbone, sous ses formes isomères ou leurs mélanges ainsi que ses sels d'addition avec les acides.

3. Un dérivé de la pristinamycine $II_B$ selon la revendication 1, caractérisé en ce que le symbole R représente une chaîne alcoyle contenant 2 à 4 atomes de carbone substituée par 1 ou 2 radicaux choisis parmi phényle, cycloalcoylamino ou N-alcoyl N-cycloalcoyl-amino contenant 5 ou 6 chaînons , alcoylamino contenant 1 à 4 atomes de carbone, dialcoylamino (dont les parties alcoyle contiennent 1 à 3 atomes de carbone ou forment avec l'atome d'azote auquel elles sont rattachées un hétérocycle saturé à 5 ou 6 chaînons), ou représente un hétérocycle azoté à 5 ou 6 chaînons éventuellement substitué par un radical alcoyle contenant 1 à 4 atomes de carbone, étant entendu que l'un au moins des substituants portés par la chaîne alcoyle ci-dessus est un substituant azoté capable de former des sels et que l'un au moins des radicaux portés par cette chaîne est placé en position −1 ou en position −2, sous ses formes isomères et leurs mélanges, ainsi que ses sels d'addition avec les acides.

4. La (diéthylamino-2 méthyl-1 éthyl) sulfinyl-26 pristinamycine $II_B$, ses formes isomères et leurs mélanges ainsi que ses sels d'addition avec les acides.

5. La [diméthylamino-2 butyl(2R)] sulfinyl-26 pristinamycine $II_B$, ses formes isomères et leurs mélanges, ainsi que ses sels d'addition avec les acides.

6. La (diéthylamino-2 propyl) sulfonyl-26 pristinamycine $II_B$, ses formes isomères et leurs mélanges, ainsi que ses sels d'addition avec les acides.

7. La (diisopropylamino-2 éthyl) sulfonyl-26 pristinamycine $II_B$, ses formes isomères et leurs mélanges, ainsi que ses sels d'addition avec les acides.

8. Procédé de préparation d'un nouveau dérivé de la pristinamycine $II_B$ selon la revendication 1, caractérisé en ce que l'on oxyde un dérivé de pristinamycine $II_B$ (son sel ou un dérivé protégé) de formule générale:

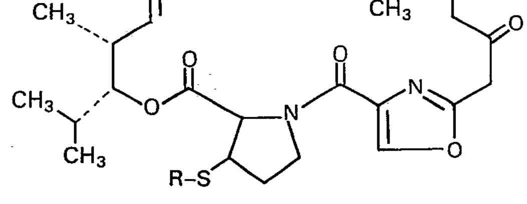

dans laquelle R est défini comme dans la revendication 1, étant entendu que dans les cas où R contient un hétérocycle soufré, l'atome de soufre peut se trouver à l'état de sulfure, de sulfoxyde ou de sulfone, puis sépare éventuellement le produit obtenu en ses isomères, élimine le ces échéant le radical protecteur et transforme éventuellement le produit obtenu en un sel d'addition avec un acide.

9. Procédé selon la revendication 2 caractérisé en ce que, lorsque l'on veut obtenir un produit selon la revendication 1 pour lequel n = 1, on utilise un agent d'oxydation choisi parmi les acides percarboxyliques ou persulfoniques ou les peracides minéraux.

10. Procédé selon la revendication 2 caractérisé en ce que, lorsque l'on veur obtenir un produit selon la revendication 1 pour lequel n = 2, on utilise un agent d'oxydation choisi parmi le dioxyde de sélénium en présence d'eau oxygénée ou un peracide.

11. Procédé de préparation d'un nouveau dérivé de la pristinamycine $II_B$ selon la revendication 1 pour lequel n = 2, caractérisé en ce que l'on oxyde un dérivé de la pristinamycine $II_B$ selon la revendication 1 pour lequel n = 1, puis sépare éventuellement le produit obtenu en ses isomères et transforme éventuellement le produit obtenu en un sel d'addition avec un acide.

12. Composition pharmaceutique caractérisée en ce qu'elle contient au moins un dérivé selon la revendication 1 associé à une synergistine connue ou une synergistine soluble de formule générale:

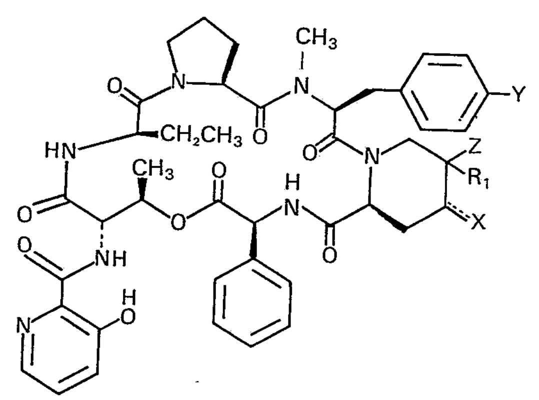

dans laquelle Y représente un atome d'hydrogène ou un radicale diméthylamino et

1) ou bien ------- représente une simple liaison, Z et $R_1$ représentent un atome d'hydrogène et X représente un radical de formule générale:

$$-N\begin{matrix}R_2\\R_3\end{matrix}$$

dans laquelle:

– soit $R_2$ représente un atome d'hydrogène et $R_3$ représente un radical hydroxy ou alcoyle éventuellement substitué par un radical carboxy, alcoyloxycarbonyle, hydroxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou bien $R_3$ représente un radical cycloalcoyle contenant 3 à 7 atomes de carbone ou un hétérocycle saturé à 4 à 7 chaînons choisi parmi les cycles azétidine, pyrrolidine, pipéridine et azépine, ces hétérocycles pouvant être éventuellement substitués sur l'atome d'azote par un radical alcoyle,

– soit $R_2$ représente un radical formyle ou alcoxylcarbonyle et $R_3$ représente un radical alcoyle substitué par un radical carboxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou bien $R_3$ représente un hétérocycle à 4 à 7 chaînons choisi parmi les cycles azétidine, pyrrolidine, pipéridine et azépine, ces hétérocycles pouvant être substitués sur l'atome d'azote par un radical alcoyle,

– soit $R_2$ et $R_3$, identiques ou différents, représentent un radical alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipridinyle, pipérazinyle, N-alcoylpipérazinyle ou azépinyle,

– soit $R_2$ et $R_3$ forment ensemble avec l'atome auquel ils sont rattachés un hétérocycle à 4 à 7 chaînons choisi parmi les cycles azétidine, pyrrolidine, pipéridine, morpholine et pipérazine éventuellement substitué par un radical alcoyle,

2) ou bien ------- représente une double liaison, X représente un atome d'oxygène et Z représente un radical de formule générale:

$$-CH\begin{matrix}R_4\\R_5\end{matrix}$$

défini de la manière suivante:

a) soit $R_1$ et $R_5$ représentent chacun un atome d'hydrogène et $R_4$ représente un radical pyrrolidinyl-3 thio ou pipéridyl-3 ou 4 thio (ces radicaux étant éventuellement substitués par un radical alcoyle) ou bien $R_4$ représente un radical alcoylthio substitué par un ou deux radicaux hydroxysulfonyle, alcoylamino, dialcoylamino (éventuellement substitué par un radical mercapto ou dialcoylamino) ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2, ou 3 (ces deux derniers cycles étant éventuellement substitués sur l'atome d'azote par un radical alcoyle),

b) soit $R_1$ et $R_5$ forment ensemble une liaison de valence et $R_4$ représente un radical pyrrolidinyl-3 amino, pipéridyl-3 ou 4 amino, pyrrolidynyl-3 oxy, pipéridinyl-3 ou 4 oxy, pyrrolidinyl-3 thio, pipéridyl-3 ou 4 thio (ces radicaux étant éventuellement substitués sur l'atome d'azote du cycle par un radical alcoyle), ou bien $R_4$ représente un radical alcoyl-

amino, alcoyloxy ou alcoylthio substitués par un ou deux radicaux hydroxysulfonyle, alcoylamino, diacoylamino (éventuellement substitué par un radical dialcoylamino), trialcoylammonio ou imidazolyle-4 ou 5 par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle), étant entendu que les radicaux alcoyle et portions alcoyle se rapportant aux symboles définis ci-dessus contiennent 1 à 5 atomes de carbone et sont en chaîne droite ou ramifiée, le cas échéant sous forme d'un de ses isomères ou de leurs mélanges, et éventuellement sous forme de sel d'addition avec un acide, de sel métallique ou de sel d'addition avec une base organique azotée, ladite composition pouvant également contenir d'autres adjuvants pharmaceutiquement acceptables.

**Revendications pour l'Etat contractant:** AT

Un procédé de préparation d'un nouveau dérivé de la pristinamycine $II_B$, caractérisé en ce qu'il répond à la formule générale:

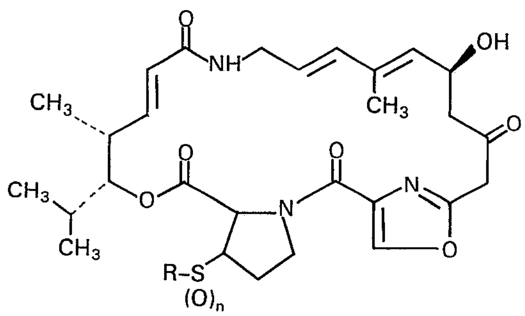

(I)

dans laquelle le symbole R représente

– soit un radical hétérocyclyle azoté de 4 à 7 chaînons, contenant éventuellement 1 ou plusieurs autres hétéroatomes choisis parmi l'azote, l'oxygène ou le soufre à l'état de sulfoxyde ou de sulfone, et éventuellement substitué par un radical alcoyle,

– soit une chaîne alcoyle contenant 2 à 4 atomes de carbone substituée par 1 ou 2 radicaux choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces 2 derniers radicaux pouvant éventuellement former avec l'atome d'azote auquel elles sont rattachées un hétérocycle saturé ou insaturé de 4 à 7 chaînons contenant éventuellement un autre hétéroatome choisi parmi l'azote, l'oxygène ou le soufre à l'état de sulfoxyde ou de sulfone, et éventuellement substitué par un radical alcoyle), ou substituée par un ou plusieurs hétérocycles azotés de 4 à 7 chaînons contenant éventuellement 1 ou 2 autres hétéroatomes choisis parmi l'azote, l'oxygène ou le soufre à l'état de sulfoxyde ou de sulfone, et éventuellement substitués par un radical alcoyle, lesdits hétérocycles étant rattachés à la chaîne qui les porte par l'intermédiaire d'un atome de carbone, étant entendu que l'un au moins des substituants portés par la chaîne alcoyle ci-dessus est un substituant azoté capable de former des sels,

– soit un radical [méthyl-1-pyrrolidinyl-2(S)] méthyle,

le symbole n est égal à 1 ou 2 et les radicaux alcoyle cités ci-dessus sont droits ou ramifiés et contiennent 1 à 10 atomes de carbone, sous ses formes isomères ou leurs mélanges, ainsi que ses sels d'addition avec les acides, caractérisé en ce que l'on oxyde un dérivé de pristinamycine $II_B$ (son sel ou un dérivé protégé) de formule générale:

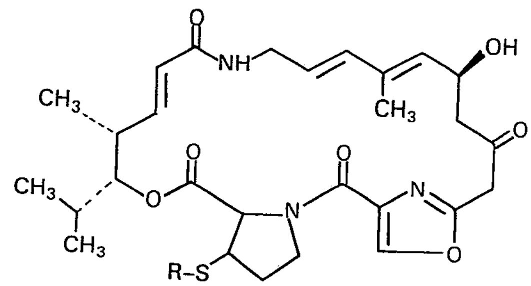

(II)

dans laquelle R est défini comme précédemment, étant entendu que dans le cas où R contient un hétérocycle soufré l'atome de soufre peut se trouver à l'état de sulfure, de sulfoxyde ou de sulfone, ou bien pour obtenir un dérivé de la pristinamycine $II_B$ pour lequel n = 2, caractérisé en ce que l'on oxyde un dérivé de la pristinamycine $II_B$ pour lequel n = 1, puis sépare eventuellement le produit obtenu en ses isomères, le cas échéant élimine le radical protecteur et transforme éventuellement le produit obtenu en un sel d'addition avec un acide.

**Patentansprüche für die Vertragsstaaten:** BE, CH, DE, FR, GB, IT, LI, LU, NL, SE

1. Neues Derivat des Pristinamycins $II_B$, dadurch gekennzeichnet, dass es der allgemeinen Formel:

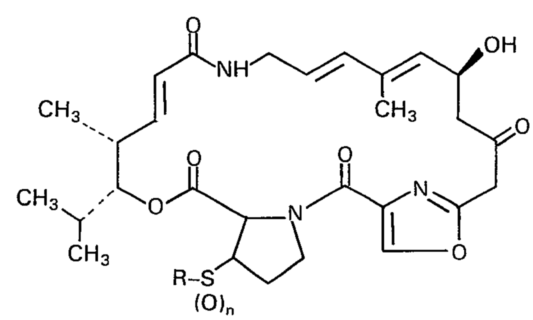

entspricht, worin das Symbol R bedeutet

–entweder einen stickstoffhaltigen Heterocyclylrest mit 4 bis 7 Kettengliedern, enthaltend gegebenenfalls ein oder mehrere andere Heteroatome, ausgewählt unter Stickstoff, Sauerstoff oder Schwefel im Zustand des Sulfoxids oder Sulfons und gegebenenfalls substituiert durch einen Alkylrest,

– oder eine Alkylkette mit 2 bis 4 Kohlenstoffatomen, substituiert durch ein oder zwei Reste, ausgewählt unter Phenyl, Cycloalkylamino oder N-Alkyl-N-cycloalkylamino, enthaltend 3 bis 6 Kettenglieder, Alkylamino, Dialkylamino oder Dialkylcarbamoyloxy

(die Alkylteile dieser beiden letzteren Reste können gegebenenfalls mit dem Stickstoffatom, an das sie gebunden sind, einen gesättigten oder ungesättigten Heterocyclus mit 4 bis 7 Kettengliedern bilden, enthaltend gegebenenfalls ein weiteres Heteroatom, ausgewählt unter Stickstoff, Sauerstoff oder Schwefel im Zustand des Sulfoxids oder Sulfons, und gegebenenfalls substituiert durch einen Alkylrest), oder substituiert durch ein oder mehrere stickstoffhaltige Heterocyclen mit 4 bis 7 Kettengliedern, enthaltend gegebenenfalls 1 oder 2 weitere Heteroatome, ausgewählt unter Stickstoff, Sauerstoff oder Schwefel im Zustand des Sulfoxids oder Sulfons, und gegebenenfalls substituiert durch einen Alkylrest, wobei die genannten Heterocyclen an die Kette, welche sie trägt, über ein Kohlenstoffatom gebunden sind, wobei wenigstens einer der Substituenten, die von der obigen Alkylkette getragen sind, ein stickstoffhaltiger Substituent ist, der Salze zu bilden vermag,

- oder einen [1-Methylpyrrolidinyl-2(S)]-methylrest, das Symbol n gleich 1 oder 2 ist und die oben erwähnten Alkylreste gerade oder verzweigt sind und 1 bis 10 Kohlenstoffatome enthalten, unter seinen isomeren Formen oder deren Gemische sowie seine Additionssalze mit Säuren.

2. Derivat des Pristinamycins $II_B$ gemäss Anspruch 1, dadurch gekennzeichnet, dass das Symbol R bedeutet:

- entweder einen stickstoffhaltigen Heterocyclylrest mit 5 oder 6 Kettengliedern, gegebenenfalls substituiert durch einen Alkylrest,

- oder eine Alkylkette mit 2 bis 4 Kohlenstoffatomen, substituiert durch 1 oder 2 Reste, ausgewählt unter Phenyl, Cycloalkylamino oder N-Alkyl-N-cycloalkylamino, enthaltend 3 bis 6 Kettenglieder, Alkylamino, Dialkylamino oder Dialkylcarbamoyloxy (wobei die Alkylteile dieser zwei letzteren Reste gegebenenfalls mit dem Stickstoffatom, an das sie gebunden sind, einen gesättigten oder ungesättigten Heterecyclus mit 5 oder 6 Kettengliedern bilden können, enthaltend gegebenenfalls ein weiteres Heteroatom, ausgewählt unter Stickstoff, Sauerstoff oder Schwefel im Zustand des Sulfoxids oder Sulfons, und gegebenenfalls substituiert durch einen Alkylrest), oder substituiert durch einen stickstoffhaltigen Heterocyclus mit 5 oder 6 Kettengliedern, enthaltend gegenebenfalls ein weiteres Heteroatom, ausgewählt unter Stickstoff, Sauerstofff oder Schwefel im Zustand des Sulfoxids oder Sulfons, und gegebenenfalls substituiert durch einen Alkylrest, wobei dieser Heterocyclus mit der Alkylkette, die ihn trägt, über ein Kohlenstoffatom gebunden ist, und dass wenigstens einer der Substituenten, die durch die obige Alkylkette getragen sind, ein stickstoffhaltiger Substituent ist, der Salze zu bilden vermag, das Symbol n gleich 1 oder 2 ist und die oben erwähnten Alkylreste gerade oder verzweigt sind und 1 bis 10 Kohlenstoffatome enthalten, unter seinen isomeren Formen oder deren Gemischen sowie seine Additionssalze mit Säuren.

3. Derivat des Pristinamycins $II_B$ gemäss Anmspruch 1, dadurch gekennzeichnet, dass das Symbol R eine Alkylkette mit 2 bis 4 Kohlenstoffatomen bedeutet, substituiert durch 1 oder 2 Reste, ausgewählt unter Phenyl, Cycloalkylamino oder N-Alkyl-N-cycloalkylamino enthaltend 5 oder 6 Kettenglieder, Alkylamino enthaltend 1 bis 4 Kohlenstoffatome, Dialkylamino (dessen Alkylteile 1 bis 3 Kohlenstoffatome enthalten oder mit dem Stickstoffatom, an das sie gebunden sind, einen gesättigten Heterocyclus mit 5 oder 6 Kettengliedern bilden), oder einen stickstoffhaltigen Heterocyclus mit 5 oder 6 Kettengliedern bedeutet, gegebenenfalls substituiert durch einen Alkylrest mit 1 bis 4 Kohlenstoffatomen, und dass wenigstens einer der Substituenten, die von der vorstehenden Alkylkette getragen sind, ein stickstoffhaltiger Substituent ist, der Salze zu bilden vermag, und dass wenigstens einer der Reste, die von dieser Kette getragen sind, in 1- oder 2-Stellung steht, unter seinen isomeren Formen und deren Gemischen sowie die Additionssalze mit Säuren.

4. 26-(2-Diethylamino-1-methylethyl)-sulfinyl-pristinamycin-$II_B$, seine isomeren Formen und deren Gemische sowie seine Additionssalze mit Säuren.

5. 26-[2-Dimethylamino-(2R)butyl]-sulfinyl-pristinamycin-$II_B$, seine isomeren Formen und deren Gemische sowie seine Additionssalze mit Säuren.

6. 26-(2-Diethylaminopropyl)-sulfonyl-pristinamycin-$II_B$, seine isomeren Formen und deren Gemische sowie seine Additionssalze mit Säuren.

7. 26-(2-Diisopropylaminoethyl)-sulfonyl-pristinamycin-$II_B$. seine isomere Formen und deren Gemische sowie seine Additioinssalze mit Säuren.

8. Verfahren zur Herstellung eines neuen Derivats des Pristinamycins $II_B$ gemäss Anspruch 1, dadurch gekennzeichnet, dass man ein Derivat des Pristinamycins $II_B$ sein Salz oder ein geschütztes Derivat) der allgemeinen Formel:

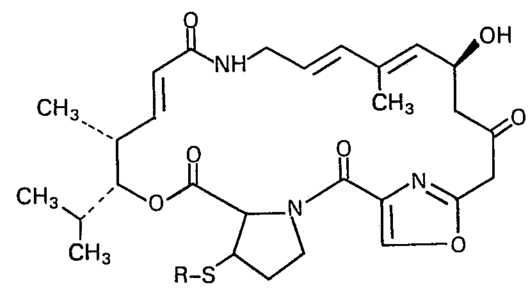

worin R wie in Anspruch 1 definiert ist, wobei, im Falle dass R einen schwefelhaltigen Heterocyclus enthält, das Schwefelatom sich im Zustand des Sulfids, Sulfoxids oder Sulfons befinden kann, oxidiert, dann das erhaltene P«rodukt gegebenenfalls in seine Isomeren auftrennt, die Schutzgruppe gegebenenfalls entfernt und das erhaltene Produkt gegebenenfalls in ein Additionssalz mit einer Säure umwandelt.

9. Verfahren gemäss Anspruch 2, dadurch gekennzeichnet, dass man, falls ein Produkt gemäss Anspruch 1 mit n = 1 erhalten werden soll, ein Oxidationsmittel, ausgewählt unter den Percarbonsäuren oder Persulfonsäuren oder den mineralischen Persäuren, verwendet.

10. Verfahren gemäss Anspruch 2, dadurch gekennzeichnet, dass man, falls ein Produkt gemäss

Anspruch 1 mit n = 2 erhalten werden soll, ein Oxidationsmittel, ausgewählt unter Selendioxid in Anwesenheit von Wasserstoffperoxid, oder eine Persäure verwendet.

11. Verfahren zur Herstellung eines neuen Derivats des Pristinamycins $II_B$ gemäss Anspruch 1, wobei n = 2, dadurch gekennzeichnet, dass man ein Derivat des Pristinamycins $II_B$ gemäss Anspruch 1, wobei n = 1, oxidiert, dann das erhaltene Produkt gegebebenfalls in seine Isomeren aufftrennt und das erhaltene Produkt gegenenenfalls in ein Additionssalz mit einer Säure umwandelt.

12. Pharmazeutische Zusammensetzung, dadurch gekennzeichnet, dass sie wenigstens ein Derivat gemäss Anspruch 1 enthält, assoziiert mit einem bekannten Synergistin oder einem löslichen Synergistin der allhemeinen Formel:

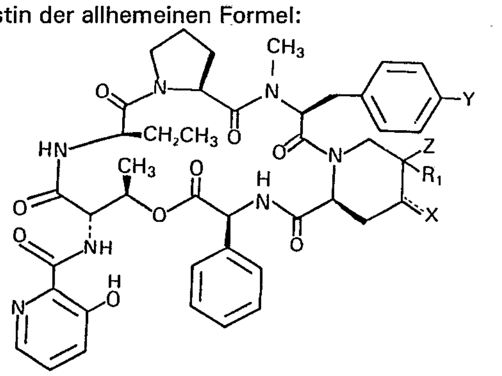

worin Y ein Wasserstoffatom oder einen Dimethylaminorest bedeutet und

1) entweder ------- eine einfache Bindung bedeutet, Z und $R_1$ ein Wasserstoffatom bedeuten und X einen Rest der allgemeinen Formel:

$R_2$
-N<
$R_3$

bedeutet, worin:

– entweder $R_2$ ein Wasserstoffatom bedeutet und $R_3$ einen Hydroxy- oder Alkylrest bedeutet, gebenenfalls substituiert durch einen Rest Carboxy, Alkoxycarbonyl, Hydroxy, Alkylamino oder Dialkylamino, dessen Alkylreste mit dem Stickstoffatom, an das sie gebunden sind, einen Heterocyclus mit 4 bis 7 Kettengliedern bilden können, ausgewählt unter Azetidinyl, Pyrrolidinyl, Piperidinyl, Piperazinyl , N-Alkylpiperazinyl oder Azepinyl, oder $R_3$ bedeutet einen Cycloalkylrest mit 3 bis 7 Kohlenstoffatomen oder einen gesättigten Heterocyclus mit 4 bis 7 Kettengliedern, ausgewählt unter den Cyclen Azetidin, Pyrrolidin, Piperidin und Azepin, wobei diese Heterocyclen gegebenenfalls am Stickstoffatom durch einen Alkylrest substituiert sein können,

– oder $R_2$ bedeutet einen Formyl- oder Alkylcarbonylrest und $R_3$ bedeutet einen Alkylrest substituiert durch einen Carboxy-, Alkylamino- oder Dialkylaminorest, dessen Alkylreste mit dem Stickstoffatom, an das sie gebunden sind, einen Heterocyclus mit 4 bis 7 Kettengliedern bilden können, ausgewählt unter Azetidinyl, Pyrrolidinyl, Piperidinyl, Piperazinyl, N-Alkylpiperazinyl oder Azepinyl, oder aber $R_3$ bedeutet einen Heterocyclus mit 4 bis 7 Kettengliedern, ausgewählt unter den Cyclen Azetidin, Pyrrolidin, Piperidin und Azepin, wobei diese Heterocyclen am Stickstoffatom durch einen Alkylrest substituiert sein können,

– oder $R_2$ und $R_3$, die identisch oder verschieden sind, bedeuten einen Alkyrest, gegebenenfalls substituiert durch einen Carboxy-, Alkyloxycarbonyl-, Hydroxy-, Alkylamino- oder Dialkylaminorest, dessen Alkylreste gegebenenfalls mit dem Stickstoffatom, an das sie gebunden sind, einen Heterocyclus mit 4 bis 7 Kettengliedern bilden, ausgewählt unter Azetidinyl, Pyrrolidinyl, Piperidinyl, Piperazinyl, N-Alkylpiperazinyl oder Azepinyl,

– oder $R_2$ und $R_3$ bilden zusammen mit dem Stickstoffatom, an das sie gebunden sind, einen Heterocyclus mit 4 bis 7 Kettengliedern, ausgewählt unter den Cyclen Azetidin, Pyrrolidin, Piperidin, Morpholin und Piperazin, gegebenenfalls substituiert durch einen Alkylrest.

2) oder aber ------- bedeutet eine Doppelbindung, X bedeutet ein Sauerstoffatom und Z bedeutet einen Rest der allgemeinen Formel:

$R_4$
-CH<
$R_5$

der folgendermassen definiert ist:

a) entweder $R_1$ und $R_5$ bedeuten jeweils ein Wasserstoffatom und $R_4$ bedeutet einen Pyrrolidinyl-3-thio- oder Piperidyl-3- oder -4-thiorest (wobei diese Restre gegebenenfalls durch einen Alkylrest substituiert sind), oder aber $R_4$ bedeutet einen Alkylthiorest, substituiert durch einen oder zwei Hydroxysulfonyl-, Alkylamino-, Dialkylamino- (gegebenenfalls substituiert durch einen Mercapto- oder Dialkylaminorest) -reste oder durch ein oder zwei Cyclen, ausgewählt unter Piperazino (gegebenenfalls substituiert durch einen Alkyl- oder Mercaptoalkylrest), Morpholino, Thiomorpholino, Piperidino, Pyrrolidinyl-1, Piperidyl-2, 3- oder -4 und Pyrrolidinyl-2 oder -3 (wobei diese beiden letzteren Cyclen gegebenenfalls am Stickstoffatom durch einen Alkylrest substituiert sind),

b) oder $R_1$ und $R_5$ bilden zusammen eine Valenzbindung und $R_4$ bedeutet einen Rest Pyrrolidinyl-3-amino, Piperidyl-3- oder -4-amino, Pyrrolidinyl-3-oxy, Piperidyl-3- oder -4-oxy, Pyrrolidinyl-3 thio, Piperidyl-3- oder -4-thio (wobei diese Reste gegebebenfalls am Stickstoffatom des Rings durch einen Alkylrest substituiert sind), oder aber $R_4$ bedeutet einen Alkylamino-, Alkyloxy- oder Alkylthiorest, substituiert durch ein oder zwei Reste Hydroxysulfonyl, Alkylamino, Dialkylamino (gegebenenfalls substituiert durch einen Dialkylaminorest), Trialkylammonio oder Imidazolyl-4- oder -5 oder durch ein oder zwei Cyclen, ausgewählt unter Piperazino (gegebenenfalls substituiert durch einen Alkyl- oder Mercaptoalkylrest), Morpholino, Thiomorpholino, Piperidino, Pyrrolidinyl-1, Piperidyl-2, -3 oder -4 und Pyrrolidinyl-2 oder -3 (wobei diese beiden letzteren Ringe gegebenenfalls am Stickstoffatom durch einen Alkylrest substituiert sind), und dass die Alkylreste und Alkylteile, die sich auf die oben definierten Symbole beziehen, 1 bis 5 Kohlenstoffatome enthalten und in gerader oder verzweigter Kette sind, gegebenenfalls in Form eines ihrer Isomeren oder

deren Gemische und gegebenenfalls in Form des Additionssalzes mit einer Säure, des Metallsalzes oder des Additionssalzes mit einer organischen Stickstoffbase, wobei diese Zusammensetzung auch andere pharmazeutisch annehmbare Hilfsmittel enthalten kann.

**Patentanspruch Für den Vertragsstaat:** AT

Ein Verfahren zur Herstellung eines neuen Derivats von Pristinamycin $II_B$, dadurch gekennzeichnet, dass es der allgemeinen Formel:

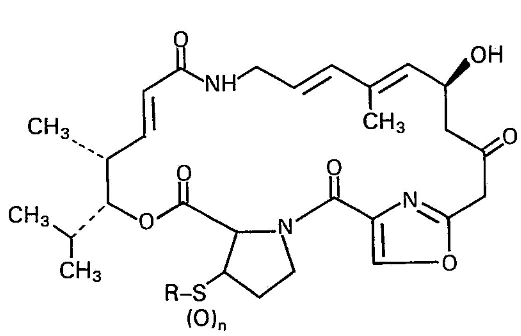

(I)

entspricht, in welcher das Symbol R darstellt

– entweder einen stickstoffhaltigen Heterocyclylrest mit 4 bis 7 Gliedern, der gebenenfalls 1 oder mehrere andere Heteroatome, ausgewählt aus Stickstoff, Sauerstoff oder Schwefel, enthält, im Zustand des Sulfoxids oder Sulfons und gegebenenfalls substituiert durch einen Alkylrest,

– oder eine Alkylkette mit 2 bis 4 Kohlenstoffatomen, substituiert durch 1 oder 2 Reste, ausgewählt aus Phenyl, Cycloalkylamino oder N-Alkyl-N-cycloalkylamino mit 3 bis 6 Gliedern, Alkylamino, Dialkylamino oder Dialkylcarbamoyloxy (die Alkylteile dieser letzteren beiden Reste können gegebenenfalls zusammen mit dem Stickstoffatom, an das sie gebunden sind, einen gesättigten oder ungesättigten Heterocyclus mit 4 bis 7 Gliedern, der gegebenenfalls ein weiteres Heteroatom, ausgewählt aus Stickstoff, Sauerstoff oder Schwefel, enthalten kann, im Zustand des Sulfoxids oder Sulfons und gegebenenfalls substituiert durch einen Alkylrest, bilden) oder substituiert durch einen oder mehrere stickstoffhaltige Heterocyclen mit 4 bis 7 Gliedern, die gegebenenfalls 1 oder 2 weiter Heteroatome, ausgewählt aus Stickstoff, Sauerstoff oder Schwefel, enthalten, im Zustand des Sulfoxids oder Sulfons und gegebenenfalls substituiert durch einen Alkylrest, welche Heterocyclen an die sie tragende Kette über ein Kohlenstoffatom gebunden sind, wobei selbstverständlich zumindest einer der an der obigen Alkylkette vorhandenen Substituenten ein Stickstoffsubstituent ist, der Salze zu bilden vermag,

– oder einen [1-Methyl-2(S)-pyrrolidinyl]-methyl-rest,

– das Symbol n gleich 1 oder 2 ist und die oben genannten Alkylreste geradkettig oder verzweigt sind und 1 bis 10 Kohlenstoffatome enthalten, in ihren isomeren Formen und deren Mischungen, sowie ihren Säureadditionssalzen, dadurch gekennzeichnet, dass man ein Derivat von Pristinamycin $II_B$ (sein Salz oder ein geschütztes Derivat) der allgemeinen Formel

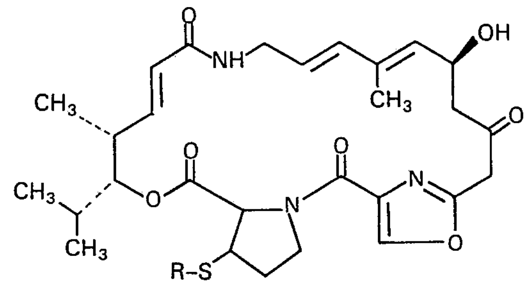

(II)

in welcher R die obige Bedeutung hat (selbstverständlich kann sich, falls R einen schwefelhaltigen Heterocyclus enthält, das Schwefelatom im Zustand des Schwefels, des Sulfoxids oder des Sulfons befinden) oxidiert

oder zur Herstellung eines Derivats von Pristinamycin $II_B$, worin n = 2, dadurch gekennzeichnet, dass man ein Derivat von Pristinamycin $II_B$, worin n = 1, oxydiert,

dann gegebenenfalls das erhaltene Produkt in seine Isomeren trennt, zutreffendenfalls die Schutzgruppe entfernt und gegebenenfalls das erhaltene Produkt in ein Säureadditionssalz überführt.

**Claims for the Contracting States:**
BE, CH, DE, FR, GB, IT, LI, LU, NL, SE

1. A new derivative of pristinamycin $II_B$, which corresponds to the general formula:

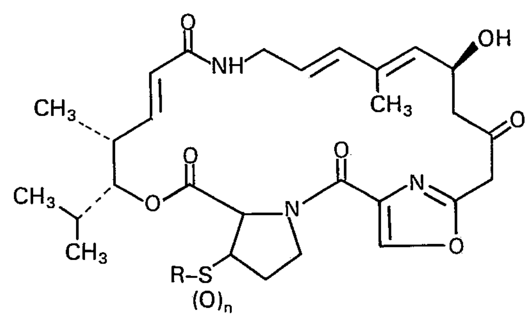

in which the symbol R denotes

– either a nitrogenous 4 to 7-membered heterocyclic ring radical optionally containing 1 or more other hetero atoms chosen from nitrogen, oxygen or sulphur in the form of sulphoxide or sulphone, and optionally substituted by an alkyl radical,

– or an alkyl chain containing 2 to 4 carbon atoms and substituted by 1 or 2 radicals chosen from phenyl, cycloalkylamino or N-alkyl-N-cycloalkylamino containing 3 to 6 chain members, alkylamino, dialkylamino or dialkylcarbamoyloxy radicals (the alkyl moieties of these two latter radicals being optionally capable of forming, with the nitrogen atom to which they are attached, a saturated or unsaturated 4 to 7-membered heterocyclic ring optionally containing another hetero atom chosen from nitrogen, oxygen or sulphur in the form of sulphoxide or sulphone, and optionally substituted by an alkyl radical), or substituted by one or more nitrogenous 4 to

7-membered heterocyclic rings optionally containing 1 or 2 other hetero atoms chosen from nitrogen, oxygen or sulphur in the form of sulphoxide or sulphone, and optionally substituted by an alkyl radical, the said heterocyclic rings being attached to the chain which carries them via the intermediacy of a carbon atom, it being understood that at least one of the substituents carried by the above alkyl chain is a nitrogen-containing substituent capable of forming salts,

- or a [(S)-1-methyl-2-pyrrolidinyl]methyl radical, the symbol n is equal to 1 or 2 and the abovementioned alkyl radicals are linear or branched and contain 1 to 10 carbon atoms, in its isomeric forms or their mixtures, as well its salts of addition with acids.

2. A derivative of pristinamycin $II_B$ according to claim 1 wherein the symbol R denotes:

- either a nitrogenous 5 or 6-membered heterocyclic ring radical optionally substituted by an alkyl radical,

- or an alkyl chain containing 2 to 4 carbon atoms substituted by 1 or 2 radicals chosen from phenyl, cycloalkylamino or N-alkyl-N-cycloalkylamino containing 3 to 6 chain members, alkylamino, dialkylamino or dialkylcarbamoyloxy radicals (the alkyl moieties of these two latter radicals being optionally capable of forming, with the nitrogen atom to which they are attached, a saturated or unsaturated 5 or 6-membered heterocyclic ring optionally containing another hetero atom chosen from nitrogen, oxygen or sulphur in the form of sulphoxide or sulphone, and optionally substituted by an alkyl radical), or substituted by a nitrogenous 5 or 6-membered heterocyclic ring optionally containing another hetero atom chosen from nitrogen, oxygen or sulphur in the form of sulphoxide or sulphone and optionally substituted by an alkyl radical, the said heterocyclic ring being attached to the alkyl chain which carries it via the intermediacy of a carbon atom, it being understood that at least one of the substituents carried by the above alkyl chain is a nitrogen-containing substituent capable of forming salts, the symbol n is equal to 1 or 2 and the abovementioned alkyl radicals are linear or branched and contain 1 to 10 carbon atoms, in its isomeric forms or their mixtures, as well as its salts of addition with acids.

3. A derivative of pristinamycin $II_B$ according to claim 1, wherein the symbol R denotes an alkyl chain containing 2 to 4 carbon atoms, substituted by 1 or 2 radicals chosen from phenyl, cycloalkylamino or N-alkyl-N-cycloalkylamino containing 5 or 6 chain members, alkylamino containing 1 to 4 carbon atoms or dialkylamino (in which the alkyl moieties contain 1 to 3 carbon atoms or form, with the nitrogen atom to which they are attached, a saturated 5 or 6-membered heterocyclic ring) or denotes a nitrogenous 5 or 6-membered heterocyclic ring optionally substituted by an alkyl radical containing 1 to a carbon atoms, it being understood that at least one of the substituents carried by the above alkyl chain is a nitrogen-containing substituent capable of forming salts and that at least one of the radicals carried by this chain is placed in a 1- position or in a 2- position, in its isomeric forms and their mixtures, as well as its salts of addition with acids.

4. 26-(2-Diethylamino-1-methylethyl)sulphinylpristinamycin $II_B$, its isomeric forms and their mixtures, as well as its salts of addition with acids.

5. 26-[(2R)2-Dimethylaminobutyl]sulhinylpristinamycin $II_B$, its isomeric forms and their mixtures, as well as salts of addition with acids.

6. 26-(2-Diethylaminopropyl)sulphonylpristinamycin $II_B$, its isomeric forms and their mixtures, as well as its salts of addition with acids.

7. 26-(2-Diisopropylaminoethyl)sulphonylpristinamycin $II_B$, its isomeric forms and their mixtures, as well as its salts of addition with acids.

8. A process for the preparation of a new derivative of pristinamycin $II_B$ according to claim 1, wherein a derivative of pristinamycin $II_B$ (its salt or a protected derivative) of general formula:

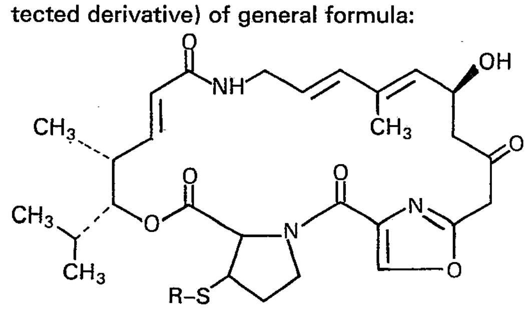

in which R is defined as in claim 1, it being understood that in the cases where R contains a sulphur-containing heterocyclic ring, the sulphur atom can be in the form of sulphide, sulphoxide or sulphone, is oxidized, the product obtained is then separated, if appropriate, into its isomers, the protective radical is removed where applicable and the product obtained is optionally converted into a salt of addition with an acid.

9. A process according to claim 2, wherein the intention is to obtain a product according to claim 1 for which n = 1, an oxidizing agent chosen from percarboxylic or persulphonic acids or inorganic peracids is used.

10. A process according to claim 2, wherein, when the intention is to obtain a product according to claim 1 for which n = 2, an oxidizing agent chosen from selenium dioxide in the presence of hydrogen peroxide or a peracid is uesd.

11. A process for the preparation of a new derivative of pristinamycin $II_B$ according to claim 1 for which n = 2, wherein a derivative of pristinamycin $II_B$ according to claim 1 for which n = 1 is oxidized, and then the product obtained is separated, if appropriate, into its isomers and the products obtained is optionally converted into a salt of addition with an acid.

12. A pharmaceutical composition which contains at least one derivative according to claim 1 in combination with a known synergistin or a soluble synergistin of general formula:

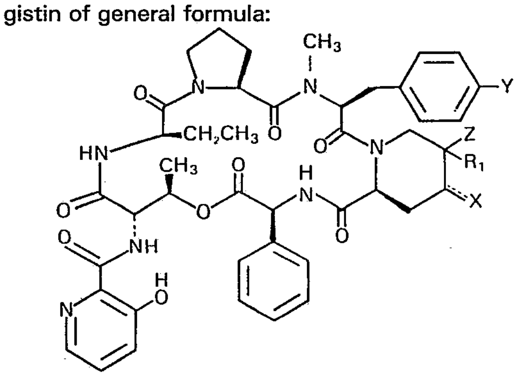

in which Y denotes a hydrogen atom or a dimethylamino radical and

1) either ------ denotes a single bond, Z and $R_1$ denote a hydrogen atom and X denotes a radical of general formula:

$$-N\langle^{R_2}_{R_3}$$

in which:

– either $R_2$ denotes a hydrogen atom and $R_3$ denotes a hydroxy or alkyl radical optionally substituted by a carboxy, alkyloxycarbonyl, hydroxy, alkylamino or dialkylamino radical whose alkyl radicals can form, with the nitrogen atom to which they are attached, a 4 to 7-member heterocyclic ring chosen from azetidinyl, pyrrolidinyl, piperidinyl, piperazinyl, N-alkylpiperazinyl or azepinyl, or $R_3$ denotes a cycloalkyl radical containing 3 to 3 carbon atoms or a saturated 4 to 7-membered heterocyclic ring chosen from the azetidine, pyrrolidine, piperidine and azepine rings, these heterocyclic rings being optionally capable of being substituted by an alkyl radical on the nitrogen atom,

– or $R_2$ denotes a formyl or alkylcarbonyl radical and $R_3$ denotes an alkyl radical substituted by a carboxy, alkylamino or dialkylamino radical whose alkyl radicals can form, with the nitrogen atom to which they are attached, a 4 to 7-membered heterocyclic ring chosen from azetidinyl, pyrrolidinyl, piperidinyl, piperazinyl, N-alkylpiperazinyl or azepinyl ring, or $R_3$ denotes a 4 to 7-membered heterocyclic ring chosen from the azetidine, pyrrolidine, piperidine and azepine rings, these heterocyclic rings being capable of being substituted by an alkyl radical on the nitrogen atom,

– or $R_2$ and $R_3$, which are identical or different, denote a alkyl radical optionally substituted by a carboxy, alkyloxycarbonyl, hydroxy, alkylamino or dialkylamino radical whose alkyl radicals optionally form, with the nitrogen atom to which they are attached, a 4 to 7-membered heterocyclic ring chosen from azetidinyl. pyrrolidinyl, piperidinyl, piperazinyl, N-alkylpiperazinyl or azepinyl rings

– or $R_2$ and $R_3$ form, together with the nitrogen atom to which they are attached, a 4 to 7-membered heterocyclic ring chosen from the azetidine, pyrrolidine, piperidine, morpholine and piperazine rings, optionally substituted by an alkyl radical,

2) or ------ denotes a double bond, X denotes an oxygen atom and Z denotes a radical of general formula:

$$-CH\langle^{R_4}_{R_5}$$

defined as follows:

a) either $R_1$ and $R_5$ each denote a hydrogen atom and $R_4$ denotes a 3-pyrrolidinylthio or 3- or 4-piperidylthio radical (these radicals being optionally substituted by an alkyl radical) or $R_4$ denotes an alkylthio radical substituted by one or two hydroxysulphonyl, alkylamino or dialkylamino (optionally substituted by a mercapto or dialkylamino radical) radicals or by one or two rings chosen from piperazino (optionally substituted by an alkyl or mercaptoalkyl radical), morpholino, thiomorpholino, piperidino, 1-pyrrolidinyl, 2, 3 or 4-piperidyl and 2- or 3-pyrrolidinyl (these last two rings being optionally substituted by an alkyl radical on the nitrogen atom),

b) or $R_1$ and $R_5$ together form a valency bond and $R_4$ denotes a 3-pyrrolidinylamino, 3- or 4--piperidylamino, 3-pyrrolidinyloxy, 3- or 4-piperidyloxy, 3-pyrrolidinylthio, 3- or 4-piperidinylthio radical (these radicals being optionally substituted by an alkyl radical on the nitrogen atom in the ring), or $R_4$ denotes an alkylamino, alkyloxy or alkylthio radical substituted by one or two hydroxysulphonyl, alkylamino, dialkylamino (optionally substituted by a dialkylamino radical), trialkylammonio or 4- or 5-imidazolyl radicals, or by one or two rings chosen from piperazino (optionally substituted by an alkyl or mercaptoalkyl radical), morpholino, thiomorpholino, piperidino, 1-pyrrolidinyl, 2, 3 or 4-piperidyl and 2- or 3-pyrrolidinyl (these two latter rings being optionally substituted by an alkyl radical on the nitrogen atom), it being understood that ther alkyl radicals and alkyl moieties referred to in the symbols defined above contain 1 to 5 carbon atoms and form a linear or branched chain, if appropriate in the form of one of its isomers or their mixtures, and optionally in the form of a salt of addition with an acid, of a metal salt or of a salt of addition with a nitrogen-containing organic base, the said composition being also capable of containing other pharmaceutically acceptable adjuvants.

**Claims for the Contracting State:** AT

1. A process for the preparation of a new derivative of pristinamycin $II_B$, which corresponds to the general formula:

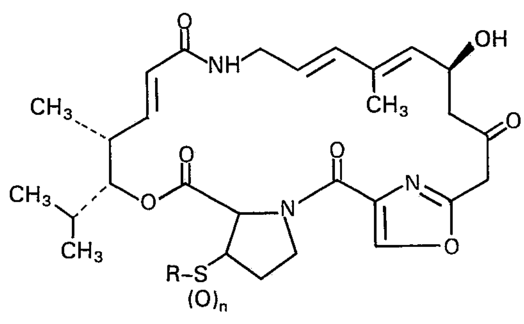

(I)

in which the symbol R denotes

– either a nitrogenous 4 to 7-membered heterocyclic ring radical optionally containing 1 or more other hetero atoms chosen from nitrogen, oxygen or sulphur in the form of sulphoxide or sulphone, and optionally substituted by an alkyl radical,

– or an alkyl chain containing 2 to 4 carbon atoms and substituted by 1 or 2 radicals chosen from phenyl, cycloalkylamino or N-alkyl-N-cycloalkylamino containing 3 to 6 chain members, alkylamino, dialkylamino or dialkylcarbamoyloxy radicals (the alkyl moieties of these 2 latter radicals being optionally capable of forming, with the nitrogen atom to which they are attached, a saturated or unsaturated 4 to 7-membered heterocyclic ring optionally containing another hetero atom chosen from nitrogen, oxygen or sulphur in the form of sulphoxide or sulphone, and optionally substituted by an alkyl radical), or substituted by one or more nitrogenous 4 to 7-membered heterocyclic rings optionally containing 1 or 2 other hetero atoms chosen from nitrogen, oxygen or sulphur in the form of sulphoxide or sulphone, and optionally substituted by an alkyl radical, the said heterocyclic rings being attached to the chain which carries them via the intermediacy of a carbon atom, it being understood that at least one of the substituents carried by the above alkyl chain is a nitrogen-containing substituent capable of forming salts,

– or a [(S)-1-methyl-2-pyrrolidinyl]methyl radical, the symbol n is equal to 1 or 2 and the abovementioned alkyl radicals are linear or branched and contain 1 to 10 carbon atoms, in its isomeric forms or their mixtures, as well its salts of addition with acids, wherein a derivative of pristinamycin $II_B$ (its salt or a protected derivative) of general formula:

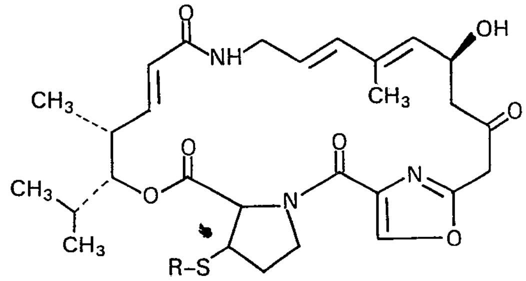

(II)

in which R is defined as above, it being understood that in the case where R contains a sulphur-containing heterocyclic ring, the sulphur atom can be in the form of sulphide, sulphoxide or sulphone, is oxidized or wherein, in order to obtain a derivative of pristinamycin $II_B$ for which n = 2, a derivative of pristinamycin $II_B$ for which n = 1 is oxidized, the product obtained is then separated, if appropriate, into its isomers, the protective radical is removed where applicable and the product obtained is optionally converted into a salt of addition with an acid.